# EUROPÄISCHE PATENTSCHRIFT

(11) **EP 0 281 943 B1**
(45) Veröffentlichungstag und Bekanntmachung des Hinweises auf die Patenterteilung: **12.01.1994**
(21) Anmeldenummer: 88103270.0
(22) Anmeldetag: 03.03.1988
(51) Int. Cl.: C07D 213/79, C07D 213/81, C07D 401/06, C07D 409/06, C07D 405/06, C07D 401/04, C07D 409/04, C07D 405/04, C07D 401/12, C07D 409/12, C07D 405/12

(54) **Substituierte Pyridin-2,4-dicarbonsäure-Derivate, Verfahren zu ihrer Herstellung, Verwendung derselben sowie Arzneimittel auf Basis dieser Verbindungen**
Substituted pyridine-2,4-carboxylic acid derivatives, process for their preparation, their use as well as medicaments based on these compounds
Dérivés d'acide pyridine-2,4-dicarboxyliques substitués, leur procédé de préparation, leur utilisation, ainsi que médicaments basés sur ces composés

(30) Priorität: 07.03.1987 DE 3707429
(43) Veröffentlichungstag der Anmeldung: 14.09.1988
(73) Patentinhaber: HOECHST AKTIENGESELLSCHAFT, 65926 Frankfurt am Main (DE)
(72) Erfinder: Baader, Ekkehard, Dr., D-6240 Königstein/Taunus (DE); Bickel, Martin, Dr., D-6380 Bad Homburg (DE); Brocks, Dietrich, Dr., D-6200 Wiesbaden (DE); Günzler, Volkmar, Dr., D-3550 Marburg-Cappel (DE); Henke, Stephan, Dr., D-6232 Bad Soden am Taunus (DE); Hanauske-Abel, Hartmut, Dr., D-6501 Dexheim (DE)

(56) Entgegenhaltungen:
- EP-A- 0 176 741
- GAZZETTA CHIMICA ITALIANA, Band 99, Nr. 5, 1969 ; L. MINALE et al.:"Struttura e biogenesi delle foemelanine. - Nota VIII. Sulla struttura della gallo-feomelanina-1." ; Seiten 431-449
- BULLETIN OF THE CHEMICAL SOCIETY OF JAPAN, Band 58, Nr. 4, 1985, T. SUGIYAMA et al.:"Change of orientation in photoreaction by oxygen. Effect of oxygen on Photomethoxylation of dimethyl esters of 2,4- and 3,4-Pyridinedicarboxylic acid", Seiten 1331-1332
- HETEROCYCLES, Band 26, Nr. 3, 1987, Seiten 731-744, G. HEINISCH et al.:"Homolytic alkoxycarbonylation reactions in two-phase systems 3 power1. Introduction of a single carboxylic acid ester function into cyano- or alkoxycarbonyl substituted n-heteroaromatics power 2"
- BEILSTEIN, Handbuch der organischen Chemie, 4. Auflage, * 3/4, Band 22, Teil 2, 1979 ; Springer Verlag, Berlin, DE, Seiten 1655, 1778
- FEBS LETTERS, Band 214, Nr. 2, April 1987, Seiten 236-248 ; H.M. HANAUSKE - ABEL et al.: "Pyrroloquinoline quinone and molecules mimicking its functional domains."

## Beschreibung

Verbindungen, die die Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird Protein-gebundenes Prolin der Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Es ist bekannt, daß die Inhibierung der Prolinhydroxylase durch bekannte Inhibitoren wie a,a'-Dipyridyl zu einer Hemmung der C1q-Biosynthese von Makrophagen führt (W. Müller et al., FEBS Lett. 90 (1978), 218; Immunbiology 155 (1978) 47). Dadurch kommt es zu einem Ausfall des klassischen Weges der Komplementaktivierung. Inhibitoren der Prolinhydroxylase wirken daher auch als Immunsuppressiva, z.B. bei Immunkomplexkrankheiten.

Es ist bekannt, daß Prolinhydroxylase durch Pyridin-2,4-und -2,5-dicarbonsäure effektiv gehemmt wird (K. Mayamaa et al., Eur. J. Biochem. 138 (1984) 239-245). Diese Verbindungen sind in der Zellkultur allerdings nur in sehr hohen Konzentrationen als Hemmstoffe wirksam (V. Günzler et al. Collagen and Rel. Research 3, 71, 1983).

In der DE-A 34 32 094 werden Pyridin-2,4- und -2,5-dicarbonsäurediester mit 1-6 C-Atomen im Esteralkylteil als Arzneimittel zur Inhibierung der Prolin- und Lysinhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Es wurde bereits vorgeschlagen (P 37 03 959.8, P 37 03 962.8 und P 37 03 963.6) gemischte Ester/Amide, höher alkylierte Diester und Diamide der Pyridin-2,4- und -2,5-dicarbonsäure einzusetzen, um die Kollagenbiosynthese im Tiermodell wirksam zu hemmen.

Es wurde nun überraschenderweise gefunden, daß auch gemischte Ester/Amide, höher alkylierte Diester und Diamide der Pyridin-2,4-dicarbonsäure, die in 5-Stellung des Pyridinrings einen weiteren Substituenten tragen, ausgezeichnete Hemmstoffe der Kollagenbiosynthese im Tiermodell sind.

Die bisher vorgeschlagenen gemischten Ester/Amide bzw. höher alkylierten Diester und die Diamide hydrolysieren mehr oder weniger schnell zu der Pyridin-2,4 bzw. 2,5-dicarbonsäure, die nach bisherigen Kenntnissen den eigentlichen Wirkstoff darstellt (s. loc. cit. K. Mayama et al.). Es ist daher umso überraschender, daß auch die substituierten Pyridin-2,4-dicarbonsäure-Derivate ausgezeichnete Hemmstoffe der Kollagenbiosynthese im Tiermodell sind, da diese Verbindungen nicht zu den reinen Dicarbonsäuren hydrolysieren, sondern zu Dicarbonsäuren, die in 5-Stellung substituiert sind.

Die Erfindung betrifft somit:
Substituierte Pyridin-2,4-dicarbonsäure-Derivate der Formel I 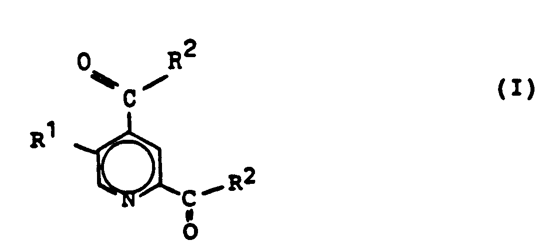 in der bedeuten:
R¹ Halogen, Carboxyl oder C1-C4-Alkoxycarbonyl oder
R¹ Alkyl, Alkenyl oder Alkinyl mit bis zu 9 C-Atomen, wobei die genannten Reste gegebenenfalls durch eine Carbonylgruppe unterbrochen sind und wobei die genannten Reste gegebenenfalls mono- oder disubstituiert sind mit
   Halogen, Hydroxy, Nitro, Cyano, Amino, C1-C₄-Alkoxy, Carbonyl, C1-C₄-Alkoxycarbonyl, C1-C₄-
   Alkylcarbonyloxy, C₁-C₄-Alkyl- oder C₁-C₄-Dialkylamino,
   oder gegebenenfalls substituiert sind mit
   Phenyl, Naphthyl, Thienyl, Furyl, Pyrrolyl oder Pyridyl, wobei diese genannten Aryl- oder Heteroaryl-Reste gegebenenfalls ihrerseits mit
   Halogen, Carboxyl, Amino, C₁-C₄-Alkyl- oder C₁-C₄-Dialkylamino oder Hydroxy monosubstituiert sind, oder
R¹ Phenyl, Naphthyl, Thienyl, Furyl, Pyrrolyl oder Pyridyl, wobei diese genannten Aryl- oder Heteroaryl-Reste gegebenenfalls ihrerseits mit
   Carboxyl, Amino, Hydroxy, C₁-C₄-Alkyl- oder C₁-C₄-Dialkylamino, monosubstituiert sind, oder
R¹ ein Substituent der Formel -OR³, oder -N(R³)₂ ist, wobei R³ Wasserstoff ist oder C₁-C₉-Alkyl, C₁-Cg-Alkenyl, C₁-C₉-Alkinyl oder C₁-C₉-Alkylcarbonyl, wobei diese Reste gegebenenfalls mono-oder disubstituiert sind mit
   Halogen, Hydroxy, Nitro, Cyano, Amino, C1-C₄-Alkoxy, Carboxyl, C1-C₄-Alkoxycarbonyl, C1-C₄-Alkylcarbonyloxy, C₁-C₄-Alkyl- oder C₁-C₄-Dialkylamino, oder gegebenenfalls substituiert sind mit Phenyl, Naphthyl, Thienyl, Furyl, Pyrrolyl oder Pyridyl, wobei diese genannten Aryl- oder Heteroaryl-Reste gegebenenfalls ihrerseits mit Halogen, Carboxyl, Amino, C₁-C₄-Alkyl- oder C₁-C₄-Dialkylamino oder Hydroxy monosubstituiert sind, und wobei die beiden Substituenten R³ in -N(R³)₂ auch unabhängig voneinander verschieden sein können
   und
R² ein Substituent der Formel -OR⁴ oder R⁴-N-R⁵ , wobei R⁴ Wasserstoff oder C₁-C₁₂-Alkyl, welches gegebenenfalls 1- oder 2-fach substituiert ist mit
   Halogen, Hydroxy, Cyano, Carboxyl, C₁-C₄-Alkoxy, C1-C4-Alkoxycarbonyl, C₁-C₄-Alkylcarbony- loxy, C₁-C₄-Alkyl- oder C₁-C₄-Dialkylamino oder gegebenenfalls substituiert ist mit Phenyl, welches seinerseits gegebenenfalls mit Halogen, C₁-C₄-Alkyl oder C₁-C₄-Alkoxy, 1-, 2-, oder 3-fach substituiert ist, wobei bei mehrfach Substitutionen die Substituenten auch voneinander unabhängig verschieden sein können, oder
R⁴ Cyclohexyl, welches gegebenenfalls benzoanelliert ist, oder
R⁴ Phenyl, Naphthyl, Thienyl, Furyl, Pyrrolyl oder Pyridyl, wobei gegebenenfalls die Phenyl-, Naphthyl- und Pyridylreste 1-, 2- oder 3-fach und die Thienyl-Furyl- und Pyrrolylreste 1-fach substituiert sind mit Halogen, C₁-C₄-Alkyl oder C₁-C₄-Alkoxy, wobei bei Mehrfachsubstitutionen die Substituenten auch unabhängig voneinander verschieden sein können und
R⁵ Wasserstoff oder C₁-C₃-Alkyl, wobei gegebenenfalls R⁵ im Falle der C₁-C₃-Alkyle zusammen mit R⁴, welches in diesem Falle C₃-C₅-Alkyl bedeutet, einen heterocyclischen, gesättigten 6-Ring bildet, wobei der heterocyclische 6-Ring auch ein zweites Stickstoffatom beinhalten kann und seinerseits mit Phenyl oder Phenyl-C₁-C₃-alkyl substituiert sein kann,
   und wobei die beiden über die Carbonylgruppe in 2- und 4-Stellung an das Pyridingerüst gebundenen Reste R² auch unabhängig voneinander verschieden sein können und wobei alle genannten Alkylreste mit mehr als 2 C-Atomen auch verzweigt sein können,
   sowie die physiologisch verträglichen Salze, ausgenommen die Pyridin-2,4,5-tricarbonsäure und deren Trimethylester, die 5-Ethyl-Pyridin-2,4-dicarbonsäure, die Verbindungen, in denen R¹ ein Aminomethylrest ist, 5-Methyl-pyridin-2,4-dicarbonsäuredimethylester, 5-Methoxy-pyridin-2,4-dicarbonsäuredimethylester, Pyridin-2,4-dicarbonsäuredimethylester-5-carbonsäureethylester, Pyridin-2,5-dicarbonsäuredimethylester-4-carbonsäuremethylester.

Substituierte Pyridin-2,4-dicarbonsäure-Derivate der Formel 1 nach Anspruch 1, in der bedeuten:
R¹ Halogen, Carboxyl oder
R¹ C₁-C₄-Alkyl, oder C₂-C₄-Alkenyl oder -Alkinyl, wobei die genannten Reste gegebenenfalls durch eine Carbonylgruppe unterbrochen sind und wobei die genannten Reste ihrerseits gegebenenfalls mono-substituiert sind mit Halogen, Hydroxy, Nitro, Cyano, Amino oder Carboxyl oder
R¹ Phenyl, Thienyl, Furyl oder Pyrrolyl, wobei die genannten Aryl- oder Heteroarylreste gegebenenfalls ihrerseits mit Carboxyl monosubstituiert sind oder
R¹ ein Substituent der Formel -OR³ oder -N(R³)₂ ist, wobei R³ Wasserstoff, C₁-C₃-Alkylcarbonyl oder C₁-C₃-Alkyl ist, wobei diese Reste ihrerseits gegebenenfalls mit Carboxyl substituiert sind oder
R³ Phenyl bedeutet, welches seinerseits gegebenenfalls mit Halogen para-substituiert ist und wobei die beiden Substituenten R³ in -N(R³)₂ auch unabhängig voneinander verschieden sein können und
R² ein Substituent der Formel -OR⁴ oder R⁴⁻N-R^{s}, wobei
   R⁴ Wasserstoff oder C₁-C₁₂-Alkyl, welches gegebenenfalls 1- oder 2-fach substituiert ist mit Halogen, Hydroxy, Cyano, Carboxyl, C1-C4-Alkoxy, C1-C4-Alkoxycarbonyl, C1-C4-Alkylcarbony- loxy, C₁-C₄-Alkyl- oder C1-C4-Dialkylamino oder gegebenenfalls substituiert ist mit Phenyl, welches seinerseits gegebenenfalls mit Halogen, C₁-C₄-Alkyl oder C₁-C₄-Alkoxy, 1-, 2-, oder 3-fach substituiert ist, wobei bei mehrfach Substitutionen die Substituenten auch voneinander unabhängig verschieden sein können, oder
R⁴ Cyclohexyl, welches gegebenenfalls benzoanelliert ist, oder
R⁴ Phenyl, Naphthyl, Thienyl, Furyl, Pyrrolyl oder Pyridyl, wobei gegebenenfalls die Phenyl-, Naphthyl-und Pyridylreste 1-, 2- oder 3-fach und die Thienyl-Furyl- und Pyrrolylreste 1-fach substituiert sind mit Halogen, C₁-C₄-Alkyl oder C₁-C₄-Alkoxy, wobei bei Mehrfachsubstitutionen die Substituenten auch unabhängig voneinander verschieden sein können und
R⁵ Wasserstoff oder C₁-C₃-Alkyl, wobei gegebenenfalls R⁵ im Falle der C₁-C₃-Alkyle zusammen mit R⁴, welches in diesem Falle C₃-C₅-Alkyl bedeutet, einen heterocyclischen, gesättigten 6-Ring bildet, wobei der heterocyclische 6-Ring auch ein zweites Stickstoffatom beinhalten kann und seinerseits mit Phenyl oder Phenyl-C₁-C₃-alkyl substitutiert sein kann,
   und wobei die beiden über die Carbonylgruppe in 2- und 4-Stellung an das Pyridingerüst gebundenen Reste R² auch unabhänig voneinander verschieden sein können und wobei alle genannten Alkylreste mit mehr als 2 C-Atomen auch verzweigt sein können,
   sowie die physiologisch verträglichen Salze, ausgenommen die Pyridin-2,4,5-tricarbonsäure und deren Trimethylester, die 5-Ethyl-Pyridin-2,4-dicarbonsäure, die Verbindungen, in denen R¹ ein Aminomethylrest ist, 5-Methyl-pyridin-274,4-dicarbonsäuredimethylester, 5-Methoxy-pyridin-2,4-dicarbonsäuredimethylester, Pyridin-2,4-dicarbonsäuredimethylester-5-carbonsäureethylester, Pyridin-2,5-dicarbonsäuredimethylester-4-carbonsäuremethylester.

Substituierte Pyridin-2,4-dicarbonsäure-Derivate der Formel I' 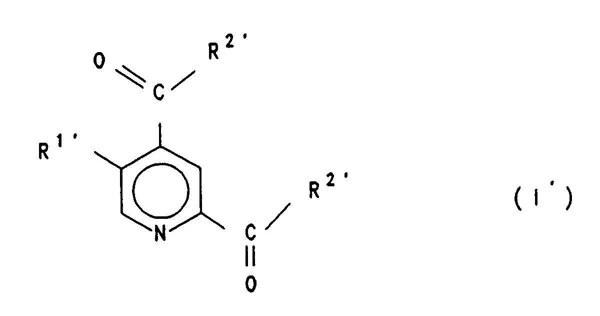 in der die Substituenten R^{1'} und R^{2'} die gleiche Bedeutung wie R¹ und R² in Formel I gemäß Anspruch 1 haben, jedoch einschließlich Pyridin-2,4,5-tricarbonsäure und deren Trimethylester, 5-Ethyl-Pyridin-2,4-dicarbonsäure, die Verbindungen, in denen R^{1'} ein Aminomethylrest ist, 5-Methyl-pyridin-2,4-dicarbonsäure- dimethylester, 5-Methoxy-pyridin-2,4-dicarbonsäuredimethylester, Pyridin-2,4-dicarbonsäuredimethylester-5-carbonsäureethylester, Pyridin-2,5-dicarbonsäuredmethylester-4-carbonsäuremethylester, zur Anwendung als Arzneimittel.

Substituierte Pyridins-2,4-dicarbonsäure-Derivate der Formel I' nach Anspruch 3, in der R^{1'} und R^{2'} die gleiche Bedeutung haben wie R¹ und R² in Formel I gemäß Anspruch 2, zur Anwendung als Arzneimittel.

Unter Halogen werden Fluor, Chlor, Brom und Jod, insbesondere Chlor, Brom und Jod verstanden.

Bei Mehrfachsubstituenten können die Substituenten auch voneinander unabhängig verschieden sein.

Weiterhin betrifft die Erfindung ein Verfahren zur Herstellung von Verbindungen der Formel I, das dadurch gekennzeichnet, ist, daß man entweder Pyridin-2,4-dimethyl-5-halogen zunächst in Pyridin-2,4,5-trimethyl überführt, welches zur Pyridin-2,4,5-tricarbonsäure oxidiert wird, welche in die Trimethoxycarbonyl-Verbindung überführt wird und anschließend zum Pyridin-2,4,5-tricarbonsäuretrikaliumsalz umgesetzt wird, oder daß man Pyridin-2,4-dimethyl-5-halogen zu einer Verbindung der Formel (1,1) oxidiert 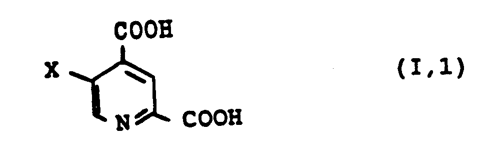 wobei X Halogen bedeutet und anschließend gegebenenfalls
a) die Verbindung der Formel (1,1) in eine Verbindung der Formel (1,2), 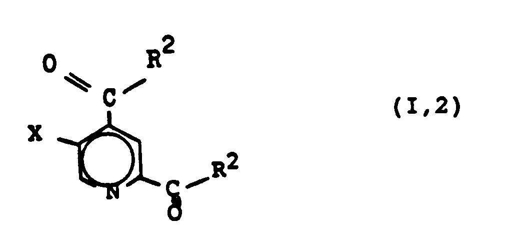 in der X Halogen ist und R² die zu Formel I gemäß Anspruch 1 angegebenen Bedeutungen hat, überführt
   und gegebenenfalls anschließend die Verbindung der Formel (1,2) mit einer Verbindung der Formel II oder 11' 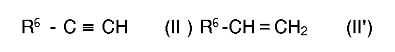 worin R⁶ C₁ -C₇-Alkyl bedeutet, welches gegebenenfalls mono- oder disubstituiert ist mit
      Halogen, Hydroxy, Nitro, Cyano, Amino, C₁-C₄-Alkoxy, Carbonyl, C₁-C₄-Alkoxycarbonyl, C₁-C₄-Alkyl-oder C₁-C₄-Dialkylamino, oder gegebenenfalls substituiert ist mit
      Phenyl, Naphthyl, Thienyl, Furyl, Pyrrolyl oder Pyridyl, wobei diese genannten Aryl- oder Heteroaryl- Reste gegebenenfalls ihrerseits mit
      Halogen, Carboxyl, Amino, C1-C4-Alkyl- oder C1-C4-Dialkylamino oder Hydroxy monosubstituiert sind,
   und in der gegebenenfalls vorhandene freie Carboxylgruppen gegebenenfalls geschützt sind,
   umsetzt und gegebenenfalls die verbliebene C-C-Dreifach- oder C-C-Doppelbindung in dem nunmehr an das Pyridingerüst gebundenen Fragment, das sich von der Verbindung der Formel II oder II' ableitet, hydriert oder gegebenenfalls die Verbindung der Formel (1,2) umsetzt mit einer Verbindung der Formel H₂N-R³, wobei R³ die in Anspruch 1 zu Formel I angegebenen Bedeutungen hat und worin gegebenenfalls vorhandene freie Carboxylgruppen gegebenenfalls geschützt sind oder gegebenenfalls die Verbindung der Formel (1,2) in an sich bekannter Weise in eine Verbindung der Formel (1,3) 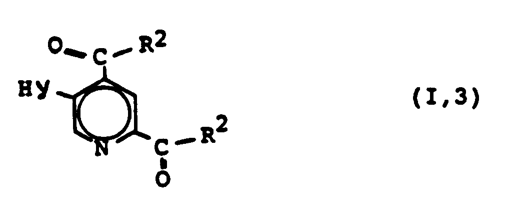 worin Y für O oder NH steht und R² die in Anspruch 1 zu Formel I angegebenen Bedeutungen hat, überführt
   und dann gegebenenfalls mit einer Verbindung der Formel III oder III' 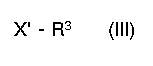 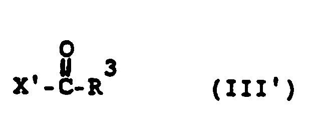 worin X' Chlor, Brom oder Jod bedeutet und R³ die in Anspruch 1 zu Formel I angegebenen Bedeutungen hat und worin gegebenenfalls vorhandene freie Carboxylgruppen gegebenenfalls geschützt sind, umsetzt
   oder gegebenenfalls die Verbindung der Formel (1,2) mit einer Verbindung der Formel IV, 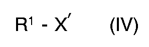 worin X' Chlor, Brom oder Jod und R¹ die in Anspruch 1 zu Formel I angegebenen Bedeutungen hat, wobei die Bedeutungen -OR³, und -N(R³)₂, in denen R³ die in Anspruch 1 zu Formel I angegebenen Bedeutungen hat, ausgenommen sind und worin gegebenenfalls vorhandene freie Carboxylgruppen gegebenenfalls geschützt sind, umsetzt
   oder gegebenenfalls die Verbindung der Formel (1,2) mit einer Verbindung der Formel V 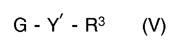 worin
      Y' für O oder NR³ steht,
      G für ein Alkalimetall
      R³ die in Anspruch 1 zu Formel I angegebenen Bedeutungen hat und worin gegebenenfalls vorhandene freie Carboxylgruppen gegebenenfalls geschützt sind, umsetzt oder daß man gegebenenfalls
b) die Verbindung der Formel (1,1) mit einer Verbindung der Formel II oder II' in der gegebenenfalls vorhandene freie Carboxylgruppen gegebenenfalls geschützt sind, umsetzt und gegebenenfalls anschließend die vorhandenen Carbonsäuren in 2- und 4-Stellung des Pyridingerüstes verestert oder in die Diamide oder Ester/Amide überführt und gegebenenfalls die verbliebene C-C-Dreifachbindung oder C-C-Doppelbindung in dem nunmehr an das Pyridingerüst gebundenen Fragment, das sich von der Verbindung der Formel II oder 11' ableitet, hydriert oder gegebenenfalls die Verbindung der Formel (1,1) umsetzt mit einer Verbindung der Formel H₂ N-R³, wobei R³ die in Anspruch 1 zu Formel I angegebenen Bedeutungen hat und worin gegebenenfalls vorhandene freie Carboxylgruppen gegebenenfalls geschützt sind oder
   gegebenenfalls die Verbindung der Formel (1,1) in eine Verbindung der Formel (1,4), 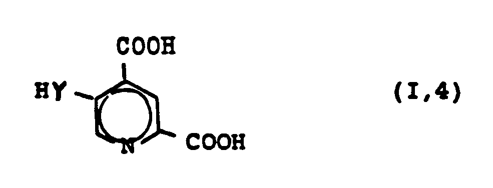 worin Y für O oder NH steht,
   überführt und gegebenenfalls anschließend die vorhandenen Carbonsäuren in 2- und 4-Stellung des Pyridingerüstes verestert oder in die Diamide oder Ester/Amide überführt
   und gegebenenfalls anschließend mit einer Verbindung der Formel III oder III' in der gegebenenfalls vorhandene freie Carboxylgruppen gegebenenfalls geschützt sind, umsetzt oder
   gegebenenfalls die Verbindung der Formel (1,1) mit einer Verbindung der Formel IV, in der gegebenenfalls vorhandene freie Carboxylgruppen gegebenenfalls geschützt sind und wobei die Verbindungen der Formel R³0-J und (R³)₂ N-J, in denen R³ die in Anspruch 1 zu Formel I angegebenen Bedeutungen hat, ausgeschlossen sind, umsetzt oder
   gegebenenfalls die Verbindung der Formel (1,1) mit einer Verbindung der Formel V, in der gegebenenfalls vorhandene freie Carboxylgruppen gegebenenfalls geschützt sind, umsetzt und gegebenenfalls anschließend die in den nach b) erhaltenen Produkten gegebenenfalls vorhandenen Carbonsäuren in 2-und 4-Stellung des Pyridingerüstes verestert oder in die Diamide oder Ester/Amide überführt oder daß man gegebenenfalls
c) zunächst die in der Verbindung der Formel (1,1) vorhandenen Carboxylgruppen in 2- und 4-Stellung des Pyridingerüstes mit einer Schutzgruppe schützt, wobei man eine Verbindung der Formel (1,10) erhält, 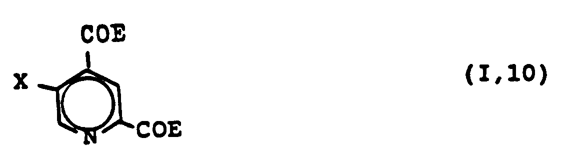 worin E eine Schutzgruppe bedeutet
   und gegebenenfalls anschließend
   die Verbindung der Formel (1,10) mit einer Verbindung der Formel II oder 11' in der gegebenenfalls vorhandene freie Carboxylgruppen gegebenenfalls geschützt sind, umsetzt und gegebenenfalls anschließend die Schutzgruppen E der Carboxylgruppen in 2- und 4-Stellung des Pyridingerüsts entweder selektiv oder gemeinsam abspaltet und gegebenenfalls die erhaltenen freien Carbonsäuren verestert oder in die Diamide oder Ester/Amide überführt und gegebenenfalls die verbliebene C-C-Dreifachbindung oder C-C-Doppelbindung in dem nunmehr an das Pyridingerüst gebundenen Fragment, das sich von der Verbindung der Formel II oder 11' ableitet, hydriert
   oder gegebenenfalls die Verbindung der Formel (1,10) umsetzt mit einer Verbindung der Formel H₂N-R³, wobei R³ die in Anspruch 1 zu Formel I angegebenen Bedeutungen hat und worin gegebenenfalls vorhandene freie Carboxylgruppen gegebenenfalls geschützt sind oder
   gegebenenfalls die Verbindung der Formel (1,10) in eine Verbindung der Formel (1,11), 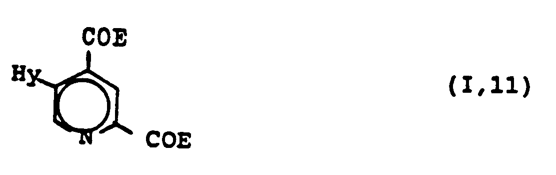 worin Y für O oder NH steht,
   überführt und gegebenenfalls anschließend die Schutzgruppen E der Carboxylgruppen in 2- und 4-Stellung des Pyridingerüsts entweder selektiv oder gemeinsam abspaltet, und gegebenenfalls die erhaltenen freien Carbonsäuren verestert oder in die Diamide oder Ester/Amide überführt
   und gegebenenfalls anschließend mit einer Verbindung der Formel 111 oder III' in der gegebenenfalls vorhandene freie Carboxylgruppen gegebenenfalls geschützt sind, umsetzt oder
   gegebenenfalls die Verbindung der Formel (1,10) mit einer Verbindung der Formel IV, in der gegebenenfalls vorhandene freie Carboxylgruppen gegebenenfalls geschützt sind und wobei die Verbindungen der Formel R³0-J und (R³)₂ N-J, in denen R³ die in Anspruch 1 zu Formel I angegebenen Bedeutungen hat, ausgeschlossen sind, umsetzt oder
   gegebenenfalls die Verbindung der Formel (1,10) mit einer Verbindung der Formel V, in der gegebenenfalls vorhandene freie Carboxylgruppen gegebenenfalls geschützt sind, umsetzt und gegebenenfalls anschließend in den nach c) erhaltenen Produkten die gegebenenfalls vorhandenen Schutzgruppen E der Carboxylgruppen in 2- und 4-Stellung des Pyridingerüsts entweder selektiv oder gemeinsam abspaltet und
   gegebenenfalls die erhaltenen freien Carbonsäuren verestert oder in die Diamide oder Ester/Amide überführt
   und anschließend gegebenenfalls die in den Produkten vorhandenen Schutzgruppen hydrolytisch oder hydrogenolytisch abspaltet und gegebenenfalls die nach a), b) oder c) erhaltenen Verbindungen in ihre physiologisch verträglichen Salze überführt.

Die Herstellung der erfindungsgemäßen Verbindungen ist im nachfolgenden Syntheseschema dargestellt.

### SYNTHESESCHEMA

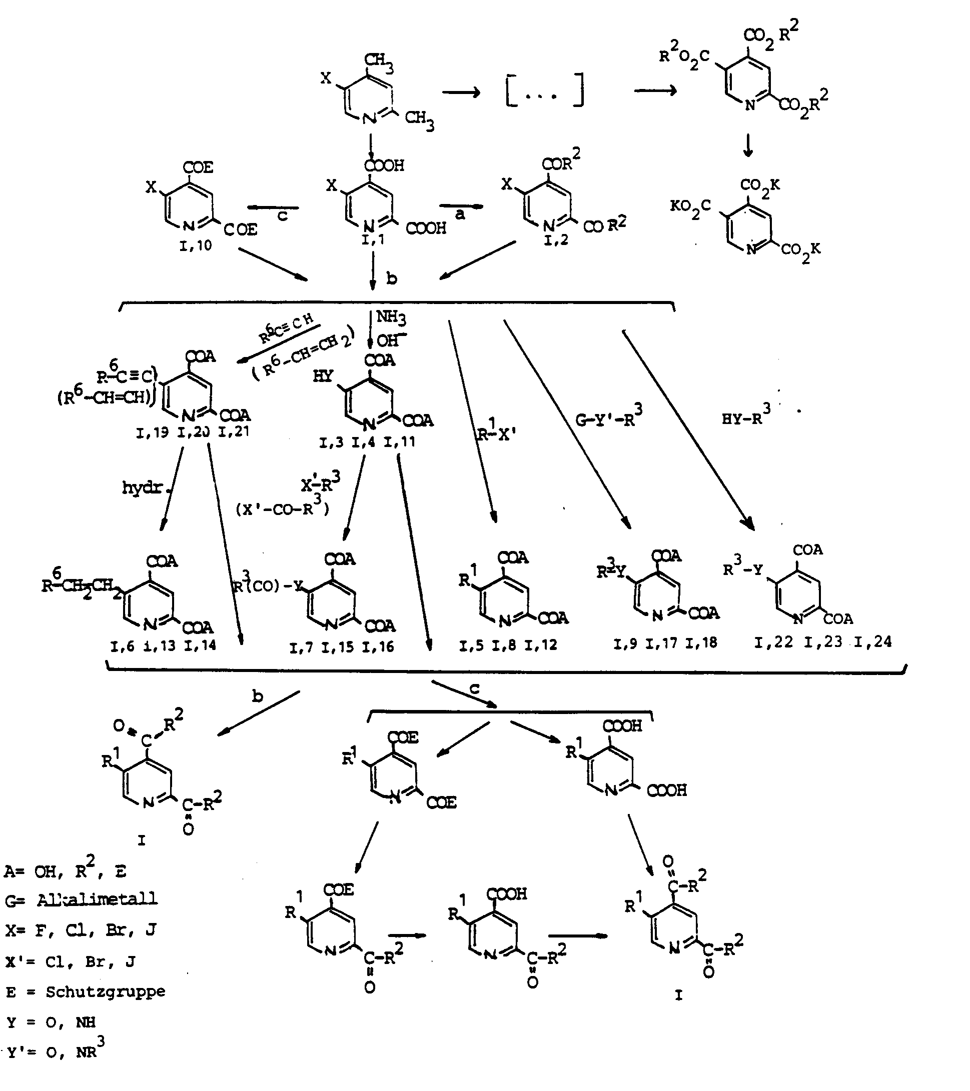

Die Verbindungen der Formel (1,1) erhält man beispielsweise durch Halogenierung von Pyridin-2,4-dimethyl. Die Reaktion kann in konzentrierter Schwefelsäure oder in Oleum, bevorzugt mit einem SOa-Gehalt von 25-65 % bei Temperaturen von 40-80° C durchgeführt werden. Als Halogenierungsagens kann Chlor, Brom oder Jod verwendet werden, welches bevorzugt pro Mol Pyridin-2,4-dimethyl mit einem halben Mol eingesetzt wird. Die Reaktionszeit beträgt bevorzugt 1-6 Stunden. Anschließend erfolgt die Oxidation des Pyridin-5-halogen-2,4-dimethyl zur Verbindung der Formel (1,1) in gängigen Oxidationsmitteln wie Salpetersäure, Chromsäure, Bichromat oder Kaliumpermanganat. Die Lösungsmittel sind z.B. Eisessig, Schwefelsäure oder Wasser wobei bei Verwendung von Wasser der pH-Wert bevorzugt bei 7-9 liegt.

Aus Pyridin-5-halogen-2,4-dimethyl kann nach Methylierung in 5-Stellung durch Oxidation die Pyridin-2,4,5-tricarbonsäure hergestellt werden, die sich beispielsweise durch Umsetzung mit KOH im Methanol in das entsprechende Pyridin-2,4,5-tricarbonsäure-trikaliumsalz überführen läßt.

Zur Herstellung weiterer erfindungsgemäßer Substanzen können nun drei unterschiedliche Verfahrensvarianten angewandt werden:

### Verfahrensvariante a)

Die Verbindungen gemäß Formel (1,1) werden zu den Dicarbonsäure-Derivaten der Formel (1,2) umgesetzt. Die Umsetzung geschieht in analoger Weise wie dies bereits in den deutschen Patentanmeldungen P 37 03 959.8, P 37 03 962.8 und P 37 03 963.6 für Pyridin-2,4- und -2,5-dicarbonsäuren vorgeschlagen wurde. Auf diese Patentanmeldungen wird an dieser Stelle ausdrücklich Bezug genommen.

### Verfahrensvariante b)

Die Verbindungen der Formel (1,1) werden ohne vorherige Umsetzung weiter eingesetzt.

### Verfahrensvariante c)

Die in den Verbindungen der Formel (1,1) vorhandenen beiden Carboxylgruppen in 2- und 4-Position des Pyridinrings werden mit einer gängigen Carboxylschutzgruppe geschützt (Verbindung (1,10)).

Als temporäre Carboxylschutzgruppen sind Esterschutzgruppen geeignet, wie sie auch in der Peptidsynthese Anwendung finden (vgl. z.B. Kontakte Merck 3/79, Seiten 15 und 19ff).

Häufig verwendet werden die Methyl-, Benzyl- oder tert.-Butylester, ferner ONbzl, OMbzl, OPic. Die Abspaltung erfolgt je nach Schutzgruppe durch saure oder alkalische Hydrolyse oder durch Hydrierung in Gegenwart eines Übergangsmetallkatalysators (Houben-Weyl, Methoden der Organischen Chemie, Bd. E5, Seiten 496-504, 4. Aufl., 1985, Georg Thieme Verlag, Stuttgart).

Die weiteren Umsetzungen der Verbindungen (1,1) (1,2) oder (1,10) beruhen auf einer Substitution des Halogenatoms in 5-Position.

So kann beispielsweis die Verbindung gemäß Formel (1,1) (1,2) oder (1,10) mit einer Verbindung der Formel 11 oder 11' umgesetzt werden. Vorzugsweise findet die Reaktion in Gegenwart eines Katalysators wie ((C₆H₅)₃P)₂PdCl₂ bei gleichzeitiger Anwesenheit einer Base wie Triethylamin und gleichzeitiger Kupferkatalyse statt. Die Reaktion kann ohne Lösungsmittel oder in Lösungsmitteln wie chlorierten Kohlenwasserstoffen wie Methylenchlorid, Chloroform, Tri- oder Tetrachlorethylen oder Benzol oder Toluol und bei Temperaturen von Raumtemperatur bis zum Siedepunkt des Lösungsmittels und während einer Reaktionszeit von 30 min bis 16 Stunden erfolgen (s. J.Med.Chem. 1987, 30, 185-193).

Bei der Reaktion zwischen dem Alk-(1)-in-Derivat oder Alk(1)-en-Derivat und dem Pyridin-5-Halogen-2,4-dicarbonsäureester entsteht eine Verbindung der Formel (1,19), (1,20) oder (1,21), die eine C-C-Dreifachbindung bzw. C-C-Doppelbindung enthält, welche gegebenenfalls anschließend mit gängigen Hydrierungsmitteln wie H₂/Pd selektiv und/oder vollständig hydriert werden kann. Dabei verwendet man die üblichen Lösungsmittel wie Alkohole, insbesondere Methanol, Ethanol oder Isopropanol.

Ebenso können die Verbindungen der Formel (1,1), (1,2) und (1,10) mit einem Amin H₂N-R³ umgesetzt werden, welches sich unter Halogenwasserstoffabspaltung in 5-Position addiert (Verbindungen (1,22), (1,23), (1,24)). Die Umsetzung erfolgt bevorzugt in Gegenwart von inerten Lösungsmitteln wie Toluol in der Siedehitze, vorzugsweise bei 110° -130° C. Die Amine der Formel H2N-R3 lassen sich - sofern sie nicht käuflich sind - auf einfache Weise nach literaturbekannten Verfahren herstellen.

Zur Herstellung der in 5-Stellung mit -OR³, oder -N(R³)₂ substituierten Pyridin-2,4-dicarbonsäure-Derivate überführt man zunächst die Verbindung (1,1), (1,2) oder (1,10) in die entsprechenden Alkohole oder Amine ((I,3), (1,4) (1,11): Y = O, NH). Dies kann beispielsweise durch Umsetzung der Verbindung (1,1), (1,2) oder (1,10) mit Natron- oder Kalilauge, die vorzugsweise 1-15 N ist ((I,3), (1,4), (1,11): Y = O) oder mit einer Ammioniaklösung, deren Dichte vorzugsweise zwischen 0,7 und 0,89 liegt, im Autoklaven, vorzugsweise bei Temperaturen von 100-160 °C und bei Reaktionszeiten von 1-4 Stunden, erfolgen ((I,3), (1,4), (1,10): Y = NH). Ein Katalysator wie z.B. ein Kupfersalz, vorzugsweise Kupfersulfat kann für beide Reaktionen eingesetzt werden.

Die entstandenen Alkohole oder Amine werden dann gegebenenfalls im nachfolgenden Reaktionsschritt mit Verbindungen der Formel III oder III' umgesetzt. Werden Verbindungen gemäß Formel III oder III' eingesetzt, die freie Carboxylgruppen enthalten, so ist es zweckmäßig, diese vor der Umsetzung mit einer geeigneten Schutzgruppe zu schützen, welche nach beendeter Reaktion gegebenenfalls wieder abgespalten werden kann (s. loc. cit. Kontakte Merck, Houben-Weyl, Bd. E5).

Die beiden Reaktanten, der Alkohol oder das Amin gemäß Formel (1,3), (1,4) oder (1,11) (Y = 0, NH) und das Halogenid gemäß Formel 111 oder III' werden in äquimolaren Mengen oder in bis zu einem etwa 5- fachen Überschuß an III oder III' vermengt und bei Temperaturen zwischen Raumtemperatur und 100° C bevorzugt zwischen 30 und 60 °C bis zur Beendigung der Reaktion umgesetzt. Die Beendigung der Reaktion läßt sich mittels Dünnschichtchromatographie (DC-Kontrolle) bestimmen. Eine Variante dieses Verfahrens besteht darin, daß man in einem geeigneten Lösungsmittel wie Diäthylether oder Dimethoxyäthan oder Tetrahydrofuran, chlorierten Kohlenwasserstoffen, wie Methylenchlorid, Chloroform, Tri- oder Tetrachloräthylen, Benzol, Toluol oder auch polaren Lösungsmitteln, wie Dimethylformamid oder Aceton oder Dimethylsulfoxid arbeitet. Auch hier kann ein Überschuß an Halogenid gemäß Formel III oder III', der bis zur etwa 5-fachen Menge betragen kann, angewandt werden. Die Reaktionstemperaturen liegen dabei zwischen Raumtemperatur und dem Siedepunkt des Lösungsmittels, wobei Temperaturen im Bereich von Raumtemperatur bis 130°C besonders bevorzugt sind.

Gegebenenfalls kann die Umsetzung auch in Gegenwart von Basen erfolgen. Als zusätzliche Basen kommen anorganische Säurefänger wie Carbonate oder Hydrogencarbonate, z.B. Natrium- oder Kaliumcarbonat oder Natrium- oder Kaliumhydrogencarbonat oder organische Säurefänger wie tertiäre Amine, wie Triäthylamin, Tributylamin, Äthyldiisopropylamin oder heterocyclische Amine wie N-Alkylmorpholin, Pyridin, Chinolin oder Dialkylaniline aber auch Alkalimetallhydride wie Natriumhydrid in Betracht.

Eine weitere Möglichkeit erfindungsgemäße Verbindungen der Formel (1,5) (1,8) oder (1,12) herzustellen, besteht darin, daß eine Verbindung der Formel (1,1), (1,2) oder (1,10) mit einem substituierten oder unsubstituierten, gesättigten oder einfach ungesättigten Alkylhalogenid, bevorzugt Jodid oder Bromid (Formel IV), umgesetzt wird. Bevorzugt erfolgt die Umsetzung in Gegenwart einer starken Base wie ButylLithium.

Eine Möglichkeit Verbindungen der Formel (1,9), (1,17) und (1,18) herzustellen, besteht darin, die Verbindungen der Formel (1,1), (1,2) oder (1,10) mit einem Alkalisalz eines Alkohols (gemäß Formel V) umzusetzen. Dabei wird bevorzugt Methanol, Ethanol oder Isopropanol verwendet, das Alkalimetall kann vorzugsweise Natrium oder Kalium sein. Die Umsetzung gelingt bei Temperaturen zwischen Raumtemperatur und dem Siedepunkt des Lösungsmittels, die Reaktionszeiten können zwischen 10 h und 100 h liegen, bevorzugt bei 60 Stunden.

Gemäß Verfahrensvariante b) werden zunächst - wie oben beschrieben - die 5-Halogen-pyridin-2,4-dicarbonsäuren in die Verbindungen (1,5), (1,6), (1,9), (1,19), (1,22), (1,3) oder (1,7) überführt und erst dann gegebenenfalls in einem nachfolgenden Reaktionsschritt gemäß den Verfahren, die in den deutschen Patentanmeldungen P 37 03 959.8, P 37 03 962.8 und P 37 03 963.6 vorgeschlagen wurden, zu den erfindungsgemäßen Produkten der Formel I umgesetzt.

Gemäß Verfahrensvariante c) werden gegebenenfalls zur Herstellung weiterer erfindungsgemäßer Substanzen die in den Verbindungen (1,14), (1,16) (1,11), (1,12), (1,21) (1,23) und (1,18) vorhandenen Carboxylschutzgruppen entweder selektiv nacheinander oder auch gemeinsam entfernt und die Verbindungen in die entsprechenden R²⁻Derivate überführt, wie dies in den deutschen Patentanmeldungen P 37 03 959.8, P 37 03 962.8 und P 37 03 963.6 für die Pyridin-2,4- und -2,5-dicarbonsäurediester/diamide/ester-amide vorgeschlagen wurde. Dies eröffnet die Möglichkeit, sowohl symmetrisch als auch unsymmetrisch substituierte Diester, Diamide oder Ester/Amide herzustellen.

Es ist weiterhin möglich, durch geeignete Auswahl der Schutzgruppen und durch geeignete Auswahl des Verfahrens zur Abspaltung dieser Schutzgruppen sowohl die in dem Substituenten R¹ gegebenenfalls vorhandenen Carboxylgruppen als auch die in 2- und 4-Position des Pyridinrings befindlichen Carboxylgruppen mit unterschiedlich oder - falls zweckmäßig - mit identischen Substituenten zu verestern.

Die Verbindungen der Formel 11 erhält man, sofern sie nicht käuflich sind, z.B. aus 1,2-Dihalogeniden, insbesondere 1,2-Dibromiden nach 2-facher Dehydrohalogenierung oder durch Umsetzung von Ketonen oder Aldehyden mit Acetylen und gegebenenfalls anschließender Reduktion des gebildeten Alkohols. Entsprechende Methoden sind z.B. im Organikum, Organisch chemisches Grundpraktikum, 15. Aufl. VEB Deutscher Verlag der Wissenschaften, Berlin, 1976, Seite 299ff. (aus Dihalogeniden) und 560ff (Äthylierung) beschrieben. Substituierte Alkinderivate gemäß Formel 11 lassen sich beispielsweise aus den entsprechenden Alk-(1)-inolen herstellen, die nach literaturbekannten Verfahren z.B. direkt zu Carbonsäuren oxidiert werden können, welche gegebenenfalls in Ester oder Amide überführt werden können. Andererseits läßt sich das Alk(1)inol auch in ein Halogenderivat, bevorzugt Chlorderivat überführen, wobei das Chloratom seinerseits anschließend beispielsweise durch eine Nitrilgruppe ersetzt werden kann, die wiederum gegebenenfalls zu einem Amin umgesetzt werden kann. Falls gewünscht, kann dieses Amin auch noch zu den entsprechenden Nitroverbindungen aufoxidiert werden. Ebenso läßt sich die Nitrilgruppe zu einer Carbonsäure verseifen, die dann ihrerseits in Ester oder Amide überführt werden kann. In analoger Weise lassen sich auch disubstituierte Alkin-Derivate gemäß Formel 11 herstellen. Eine weitere Methode substituierte Alkinderivate gemäß Formel II herzustellen, ist die nukleophile Substitution von Halogenverbindungen wie z.B. Halogenalkane mit Natriumacetylid. Entsprechende Methoden sind z.B. in "Reaktionen und Synthesen" in Org.Chem. Praktikum, Tietze/Eicher, Thieme-Verlag, Stuttgart/New York 1981, Seite 38 beschrieben.

Die Alkenverbindungen der Formel II' lassen sich - sofern sie nicht käuflich sind - auf einfache Weise nach literaturbekannten Verfahren herstellen.

Die Verbindungen der Formel III, III' und IV lassen sich ebenfalls, sofern sie nicht käuflich sind, auf einfache Weise synthetisieren (z.B. Organikum, Organisch chemisches Grundpraktikum, VEB Deutscher Verlag der Wissenschaften, 15. Aufl. Berlin 1976; eine Übersicht findet sich im Methodenregister, Seite 826: Halogenverbindungen).

Sofern in den Verbindungen gemäß Formel III, III' oder IV freie Carboxylgruppen vorhanden sind, werden diese gegebenenfalls vor der Umsetzung mit den Verbindungen gemäß Formel (1,3) (1,4)/(1,11) oder (1,2)/(1,1/(1,10) mit einer geeigneten Schutzgruppe versehen (s. loc. cit. Kontakte Merck), die nach erfolgter Umsetzung gegebenenfalls wieder hydrolytisch oder hydrogenolytisch abgespaltet werden kann (s. loc.cit. Houben-Weyl, Bd, E5).

Die Verbindungen der Formel V lassen sich nach gängiger Methode durch Umsetzung äquimolarer Mengen eines Alkalimetalls mit einem Alkohol herstellen. Auch hier können gegebenenfalls vorhandene Carboxylgruppen mit einer temporären Schutzgruppe (s. loc. cit. Kontakte Merck) versehen werden.

Gegenbenenfalls kann die Aufarbeitung der Produkte beispielsweise durch Extraktion oder durch Chromatographie z.B. über Kieselgel erfolgen. Das isolierte Produkte kann umkristallisiert und gegebenenfalls mit einer geeigneten Säure zu einem physiologisch verträglichen Salz umgesetzt werden. Als geeignete Säuren kommen beispielsweise in Betracht:

Mineralsäuren, wie Chlorwasserstoff- und Bromwasserstoffsäure, sowie Schwefel-, Phosphor-, Salpeter-oder Perchlorsäure oder organische Säuren wie Ameisen-, Essig-, Propion-, Bernstein-, Glykol-, Milch-, Äpfel-, Wein-, Zitronen-, Malein-, Fumar-, Phenylessig-, Benzoe-, Methansulfon-, Toluolsulfon, Oxal-, 4-Aminobenzoe-, Naphthalin-1 ,5-disulfon- oder Ascorbinsäure.

Die erfindungsgemäßen Verbindungen der Formel 1 bzw. l' besitzen wertvolle pharmakologische Eigenschaften und zeigen insbesondere Wirksamkeit als Hemmer der Prolin-und Lysinhydroxylase, als Fibrosupressivum und Immunsupressivum.

Die Aktivität der Fibrogenase kann durch radioimmunologische Bestimmung des N-terminalen Propeptids des Kollagens-Typ-III oder der N- bzw. C-terminalen Quervernetzungsdomäne des Kollagens Typ-IV (7s-Kollagen, bzw. Typ-IV-Kollagen-NC1) im Serum bestimmt werden.

Zu diesem Zweck wurden die Hydroxyprolin-, Prokollagen-III-Peptid-, 7s-Kollagen- und Typ-IV-Kollagen-NC₁ -Konzentrationen in der Leber von
a) unbehandelten Ratten (Kontrolle)
b) Ratten, denen Tetrachlorkohlenstoff verabreicht wurde (CC1₄-Kontrolle)
c) Ratten, denen zunächst CC1₄ und anschließend eine erfindungsgemäße Verbindung verabreicht wurde gemessen (diese Testmethode wird beschrieben von Rouiller, C., experimental toxic injury of the liver; in The Liver, C. Rouiller, Vol. 2, S. 335-476, New York, Academic Press, 1964).

Die pharmakologische Wirksamkeit der erfindungsgemäßen Substanzen wurde untersucht. Dabei zeigte sich eine deutliche Hemmung der Prolin- und Lysinhydroxylase.

Die Verbindungen der Formel 1 bzw. l' können als Medikamente in Form pharmazeutischer Präparate Verwendung finden, welche sie gegebenenfalls mit verträglichen pharmazeutischen Trägern enthalten. Die Verbindungen können als Heilmittel, z.B. in Form pharmazeutischer Präparate Verwendung finden, welche diese Verbindungen in Mischungen mit einem für die enterale, perkutane oder parenterale Applikation geeigneten pharmazeutischen, organischen oder anorganischen Träger, wie z.B. Wasser, Gummi-arabicum, Gelatine, Milchzucker, Stärke, Magnesiumstearat, Talk, pflanzliche Öle, Polyalkylenglykole, Vaseline usw. enthalten.

Die pharmazeutischen Präparate können in fester Form, z.B. als Tabletten, Dragees, Suppositorien oder Kapseln, in halbfester Form, z.B. als Salben oder in flüssiger Form, z.B. als Lösungen, Suspensionen oder Emulsionen, vorliegen. Gegebenfalls sind sie sterilisiert und/oder enthalten Hilfsstoffe, wie Konservierungs-, Stabilisierungs-, Netz- oder Emulgiermittel, Salze zur Veränderung des osmotischen Drucks oder Puffer. Sie können auch noch andere therapeutisch wirksame Stoffe enthalten.

Im folgenden wird die Erfindung anhand von Beispielen näher erläutert.

### Beispiele

### Beispiel 1

### Herstellung von 5-Brom-2,4-dimethylpyridin

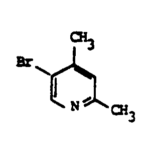

Zu 28,9 ml 2,4-Dimethylpyridin wird unter Eiskühlung und Rühren 150 ml 65 % Oleum so zugetropft, daß die Temperatur 35° C nicht übersteigt. Hat sich die Lösung homogenisiert, wird langsam unter Rühren 6,42 ml Brom zugetropft. Es wird bei 80° C 3 1/2 Stunden gerührt. Nach dem Abkühlen läßt man vorsichtig auf 1 kg Eis tropfen, neutralisiert mit festem Na₂C0₃ und extrahiert 3 mal mit je 300 ml Ether. Die organische Schicht wird abgetrennt und über Magnesiumsulfat getrocknet. Nach Entfernen des Lösungsmittels durch Destillation im Vakuum erhält man 34,6 g blaßgelbes ÖI, welches aus den Isomeren 5-Brom-2,4-dimethylpyridin und 3-Brom-2,4-dimethylpyridin besteht. Die Isomere werden durch Säulenchromatographie an Siliziumdioxidgel getrennt und man erhält 10 g 5-Brom-2,4-dimethylpyridin als farblose Flüssigkeit (13,0 g 3-Brom-2,4-dimethylpyridin).

### Ausbeute: 22 %.

### Beispiel 2

### Herstellung von 5-Brom-2,4-pyridindicarbonsäure

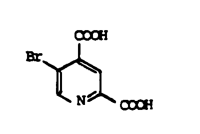

4 g 5-Brom-2,4-dimethylpyridin aus Beispiel 1 werden in 200 ml Wasser und 2,4 g KOH auf 70-80 _{°} C erhitzt. Dann wird portionsweise die Hälfte von 12,74 g Kaliumpermanganat eingetragen. Die Lösung wird zum Sieden erhitzt und der Rest des Kaliumpermanganats zugegeben. Man läßt 20 h bei 70-80° C rühren, saugt dann heiß ab und wäscht den Niederschlag 4 mal mit 50 ml-Portionen heißem Wasser. Die vereinigten Filtrate werden im Vakuum auf 100 ml eingeengt. Man stellt die Lösung mit konzentrierter Salzsäure auf pH 1 ein und läßt die Lösung für 20 Stunden bei 0° C stehen. Der kristalline Feststoff wird abgesaugt und bei 100°C im Vakuum getrocknet. Die Ausbeute beträgt 2,9 g. Fp. 261-263 ° C. Ausbeute: 55 %.

### Beispiel 3

Herstellung von 5-Brom-2,4-pyridindicarbonsäuredimethylester (im folgenden "5-Bromdicarbonsäuredimethylester" genannt)

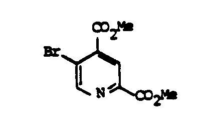

1 g 5-Brom-2,4-pyridindicarbonsäure werden in 20 ml Methanol gelöst und 1 ml konzentrierte Schwefelsäure zugetropft. Die Lösung wird 24 h bei 75 ° C gerührt. Die Lösung wird dann abgekühlt, mit gesättigter Natriumhydrogencarbonatlösung alkalisch gestellt und 3 mal mit Essigester extrahiert. Die vereinigten organischen Phasen werden mit Wasser gewaschen, mit Magnesiumsulfat getrocknet und das Lösungsmittel im Vakuum entfernt. Es verbleibt 1,0 g eines weißen Festkörpers mit einem Fp. von 102-104 ° C. Ausbeute: 90 %

### Beispiel 4

Herstellung von 5-Hydroxy-2,4-pyridindicarbonsäure

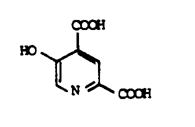

Ein Gemisch aus 500 mg 5-Brom-2,4-pyridindicarbonsäure aus Beispiel 2, 20 ml 10 N wäßriger Natronlauge und 250 mg Kupfervitriol werden im Autoklaven für 4 Stunden auf 165° C erhitzt. Nach dem Erkalten wird vom Kupfersalz abgesaugt, mit konzentrierter Salzsäure auf pH 1 eingestellt und die Lösung eingedampft. Der Festkörper wird in wenig Methanol erhitzt, von den Salzen abfiltriert und die Lösung bei 0° C 20 Stunden stehengelassen. Der ausgefallene Festkörper wird abgetrennt und getrocknet. Ausbeute: 70 mg Fp. 280-285 _{°} C

### Beispiel 5

Herstellung von 5-Amino-pyridin-2,4-dicarbonsäure

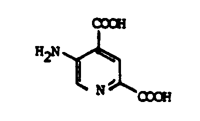

Ein Gemisch aus 500 mg 5-Brom-pyridin-2,4-dicarbonsäure aus Beispiel 2, 100 mg Kupfervitriol und 20 ml Ammoniaklösung (d = 0,91) werden im Autoklaven für 4 Stunden auf 160° C erhitzt. Die Lösung wird zur Trockne eingedampft, der Festkörper mit wenig Methanol erhitzt und die Lösung vom Unlöslichen abgetrennt. Nach 20 Stunden bei 0° C fällt ein weißer Festkörper aus, der abfiltriert und getrocknet wird. Ausbeute: 70 mg Fp. 315°C Zers.

### Beispiel 6

Herstellung von 5-Methoxypyridin-2,4-dicarbonsäuredimethylester

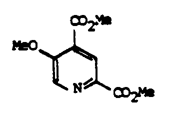

300 mg 5-Brompyridin-2,4-dicarbonsäuredimethylester (aus Beispiel 3) werden in 5 ml abs. Methanol gelöst und mit 120 mg Natriummethylat versetzt. Nach 60 h Rückfluß wird auf Eis und 2 ml 2N HCI gegossen, mit NaHCO₃ alkalisiert und mit CH₂CI₂ 2 mal extrahiert. Nach Trocknen über MgS0₄ wird das Lösungsmittel eingedampft. Es verbleiben 175 mg weißer Festkörper. Fp: 133-135 °C

### Beispiel 7

### Herstellung von 5-(4-Hydroxy-1-butinyl)-pyridin-2,4-dicarbonsäuredimethylester

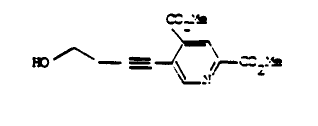

In einen mit Argon gespülten Kolben werden 461 mg 5-Brompyridin-2,4-dicarbonsäuredimethylester (aus Beispiel 3) und 142 mg 4-Hydroxy-1-butin in Methylenchlorid gelöst und 680 µl Triethylamin zugetropft. Es wird 15 Minuten bei Raumtemperatur gerührt, 13 mg ((C₆H₅)₃P)₂PdCl₂ und 2 mg CuJ zugegeben und 2 Stunden unter Rückfluß gekocht. Nach dem Abkühlen wird mit Methylenchlorid verdünnt, mit Wasser und Natriumchlorid-Lösung gewaschen und die vereinigten organischen Phasen über Kaliumcarbonat getrocknet. Nach dem Eindampfen des Lösungsmittels verbleiben 347 mg eines weißen Festkörpers.

### Fp: 87 _{°} C

### Beispiel 8

### Herstellung von 5-(3-Hydroxy-1-pentinyl)-pyridin-2,4-dicarbonsäure-dimethylester

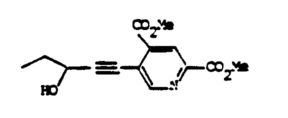

In einem mit Argon gespülten Kolben werden 546 mg 5-Bromdicarbonsäuredimethylester (aus Beispiel 3) und 202 mg 3-Hydroxy-1-pentinyl in Methylenchlorid gelöst und 840 µl Triethylamin zugetropft. Es wird 15 Minuten bei Raumtemperatur gerührt, 28 mg ((C₆H₅)₃P)₂ PdC1₂ und 4 mg CuJ zugegeben und 18 Stunden unter Rückfluß gekocht. Nach dem Abkühlen wird mit Methylenchlorid verdünnt, mit Wasser und Natriumchlorid-Lösung gewaschen und die vereinigten organischen Phasen über Kaliumcarbonat getrocknet. Nach dem Eindampfen verbleiben 404 mg eines gelben Öles, welches bei 0 _{°} C kristallisiert.

### Fp.: 65 ° C.

### Beispiel 9

### Herstellung von 5-(4-Methoxycarbonyl-1-butinyl)-pyridin-2,4-dicarbonsäuredimethylester

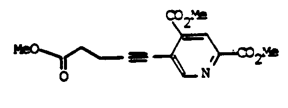

In einem mit Argon gespülten Kolben werden 546 mg 5-Bromdicarbonsäuredimethylester (aus Beispiel 3) und 269 mg 4-Pentinsäuremethylester in Methylenchlorid gelöst und 840 µl Triethylamin zugetropft. Es wird 15 Minuten bei Raumtemperatur gerührt, 28 mg ((C₆H₅)₃P)₂PdCl₂ und 4 mg CuJ zugegeben und 18 Stunden unter Rückfluß gekocht. Nach dem Abkühlen wird mit Methylenchlorid verdünnt, mit Wasser und Natriumchlorid-Lösung gewaschen und die vereinigten organischen Phasen über Kaliumcarbonat getrocknet. Nach dem Eindampfen und Chromatographieren an Kieselgel verbleiben 460 mg eines weißen Festkörpers.

### Fp.: 113° C.

### Beispiel 10

Herstellung von 5-(3-Hydroxy-1-propinyl)-pyridin-2,4-dicarbonsäuredimethylester

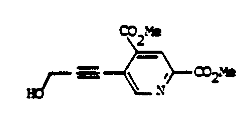

In einem mit Argon gespülten Kolben werden 500 mg 5-Bromdicarbonsäuredimethylester (aus Beispiel 3) und 121 mg Propargylalkohol in Methylenchlorid gelöst und 840 µl Triethylamin zugetropft. Es wird 15 Minuten bei Raumtemperatur gerührt, 25 mg ((C₆H₅)₃P)₂PdCl₂ und 4 mg CuJ zugegeben und 30 Stunden unter Rückfluß gekocht. Nach dem Abkühlen wird mit Methylenchlorid verdünnt, mit Wasser und Natriumchlorid-Lösung gewaschen und die vereinigten organischen Phasen über Kaliumcarbonat getrocknet. Nach dem Eindampfen verbleiben nach Chromatographie an Kieselgel 260 mg eines weißen Festkörpers. Fp.: 104-106°C.

### Beispiel 11

### Herstellung von 5-(5-Cyano-1-pentinyl)-pyridin-2,4-dicarbonsäuredimethylester

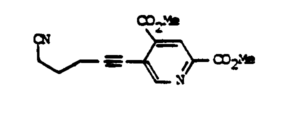

In einem mit Argon gespülten Kolben werden 500 mg 5-Bromdicarbonsäuredimethylester (aus Beispiel 3) und 201 mg Hexinsäurenitril in Methylenchlorid gelöst und 840 µl Triethylamin zugetropft. Es wird 15 Minuten bei Raumtemperatur gerührt, 25 mg ((C₆H₅)₃P)₂PdCl₂ und 4 mg CuJ zugegeben und 40 Stunden unter Rückfluß gekocht. Nach dem Abkühlen wird mit Methylenchlorid verdünnt, mit Wasser und Natriumchlorid-Lösung gewaschen und die vereinigten organischen Phasen über Kaliumcarbonat getrocknet. Nach dem Eindampfen verbleiben 364 mg eines weißen Festkörpers.

### Fp.: 55-57 _{°} C.

### Beispiel 12

### Herstellung von 5-(N-Benzylamino-1-propinyl)-pyridin-2,4-dicarbonsäuredimethylester

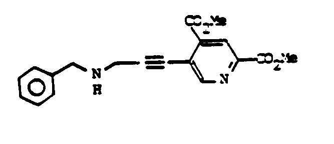

In einem mit Argon gespülten Kolben werden 2g 5-Bromdicarbonsäuredimethylester (aus Beispiel 3) und 1,25 g N-Benzylpropargylamin in Methylenchlorid gelöst und 3,4 ml Triethylamin zugetropft. Es wird 15 Minuten bei Raumtemperatur gerührt, 25 mg ((C₆H₅)₂P)₂PdCl₂und 4 mg CuJ zugegeben und 36 Stunden unter Rückfluß gekocht. Nach dem Abkühlen wird mit Methylenchlorid verdünnt, mit Wasser und Natriumchlorid-Lösung gewaschen und die vereinigten organischen Phasen über Kaliumcarbonat getrocknet. Nach dem Eindampfen verbleiben 1,25 g eines dunklen Öles, welches ohne Reinigung hydriert wird (Beispiel 18).

### Beispiel 13

### Herstellung von 5-(4-Hydroxy-butyl)-pyridin-2,4-dicarbonsäuredimethylester

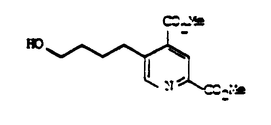

200 mg 5-(4-Hydroxy-1-butinyl)-pyridin-2,4-dicarbonsäuredimethylester (aus Beispiel 7) werden in 25 ml Methanol gelöst und nach Zugabe des Palladium-Katalysators (10 % auf Kohle) hydriert. Die Umsetzung ist nach 4 Stunden beendet (Dünnschichtkontrolle). Der Katalysator wird abfiltriert und die Lösung im Vakuum eingeengt. Das farblose Öl wird an Kieselgel chromatographiert. Ausbeute: 157 mg Öl

### Beispiel 14

### Herstellung von 5-(3-Hydroxy-pentyl)-pyridin-2,4-dicarbonsäuredimethylester

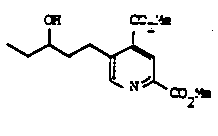

317 mg 5-(3-Hydroxy-1-pentinyl)-pyridin-2,4-dicarbonsäuredimethylester(Beispiel 8) werden in 25 ml Methanol gelöst und nach Zugabe des Palladium-Katalysators (10 % auf Kohle) hydriert. Die Umsetzung ist nach 4 Stunden beendet (Dünnschichtkontrolle). Der Katalysator wird abfiltriert und die Lösung im Vakuum eingeengt. Das farblose Öl wird an Kieselgel chromatographiert. Ausbeute: 200 mg Fp.: 77-78 _{°} C.

### Beispiel 15

### Herstellung von 5-(4-Methoxycarbonyl-butyl)-pyridin-2,4-dicarbonsäuredimethylester

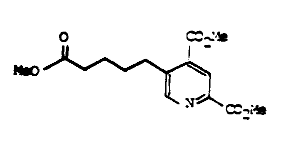

305 mg 5-(4-Methoxycarbonyl-1-butinyl)-pyridin-2,4-dicarbonsäuredimethylester (aus Beispiel 9) werden in 30 ml Methanol gelöst und nach Zugabe des Palladium-Katalysators (10 % auf Kohle) hydriert. Die Umsetzung ist nach 4 Stunden beendet (Dünnschichtkontrolle). Der Katalysator wird abfiltriert und die Lösung im Vakuum eingeengt. Das farblose Öl wird an Kieselgel chromatographiert. Ausbeute: 260 mg Fp.: 39 ° C.

### Beispiel 16

### Herstellung von 5-(3-Hydroxy-propyl-)-pyridin-2,4-dicarbonsäuredimethylester

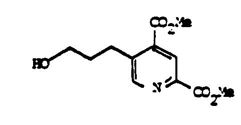

655 mg 5-(3-Hydroxy-1-propinyl)-pyridin-2,4-dicarbonsäuredimethylester(Beispiel 10) werden in 50 ml Methanol gelöst und nach Zugabe des Palladium-Katalysators (10 % auf Kohle) hydriert. Die Umsetzung ist nach 4 Stunden beendet (Dünnschichtkontrolle). Der Katalysator wird abfiltriert und die Lösung im Vakuum eingeengt. Das farblose Öl wird an Kieselgel chromatographiert. Ausbeute: 540 mg Fp.: 92-94 °C.

### Beispiel 17

### Herstellung von 5-(5-Cyano-pentyl)-pyridin-2,4-dicarbonsäuredimethylester

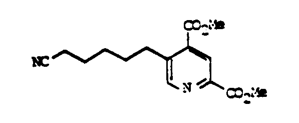

64 mg 5-(5-Cyano-1-pentinyl)-pyridin-2,4-dicarbonsäuredimethylester (aus Beispiel 11) werden in 25 ml Methanol gelöst und nach Zugabe des Palladium-Katalysators (10 % auf Kohle) hydriert. Die Umsetzung ist nach 4 Stunden beendet (Dünnschichtkontrolle). Der Katalysator wird abfiltriert und die Lösung im Vakuum eingeengt. Das farblose Öl wird an Kieselgel chromatographiert. Ausbeute: 47 mg Öl.

### Beispiel 18

### Herstellung von 5-(3-N-Benzyl-aminopropyl)-pyridin-2,4-dicarbonsäuredimethylester

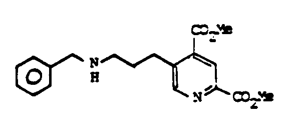

1,25 g 5-(N-Benzylamino-1-propinyl)-pyridin-2,4-dicarbonsäuredimethylester (aus Beispiel 12) werden in 10 ml Methanol gelöst und nach Zugabe des Palladium-Katalysators (10 % auf Kohle) hydriert. Die Umsetzung ist nach 4 Stunden beendet (Dünnschichtkontrolle). Der Katalysator wird abfiltriert und die Lösung im Vakuum eingeengt. Das farblose Öl wird an Kieselgel chromatographiert. Ausbeute: 1,01 g Öl

### Beispiel 19

### Herstellung von 5-Amino-pyridin-2,4-dicarbonsäurediethylester

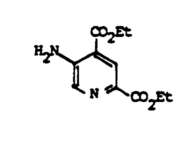

850 mg 5-Amino-pyridin-2,4-dicarbonsäure (aus Beispiel 5) werden in 100 ml absolutem Ethanol gelöst, mit 5 ml konz. Schwefelsäure versetzt und 20 Stunden unter Rückfluß erhitzt. Die Lösung wird eingeengt, mit Essigsäureethylester und ges. Natriumhydrogencarbonat-Lösung versetzt und extrahiert. Die wäßrige alkalische Phase wird noch 3 x mit Essigsäureethylester extrahiert, die vereinigten organischen Phasen über Magnesiumsulfat getrocknet und eingedampft. Es verbleiben 850 mg eines weißen Festkörpers. Fp.: 155-157 _{°} C.

### Beispiel 20

### Herstellung von 5-(3-Chlorpropyl)-pyridin-2,4-dicarbonsäuredimethylester

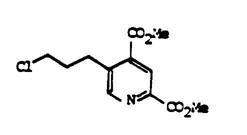

370 mg 5-(3-Hydroxypropyl)-pyridin-2,4-dicarbonsäuredimethylester (aus Beispiel 16) werden in 10 ml Chloroform gelöst, auf 0° C abgekühlt und mit 0,18 ml Thionylchlorid in 2 ml Chloroform langsam versetzt. Es wird eine Stunde bei Raumtemperatur, dann eine Stunde bei 60° C nachgerührt. Nach dem Abkühlen wird eingedampft und mit Chloroform und Wasser aufgenommen; die Phasen werden getrennt, die organische Phase mit Natriumsulfatlösung gewaschen, über Magnesiumsulfat getrocknet und eingedampft. Es verbleiben nach Chromatographie an Kieselgel 286 mg eines gelben Öles.

### Beispiel 21

### Herstellung von 3-Phenyl-propionsäure-N-([2,4-diethoxycarbonyl]-pyridyl)-amid

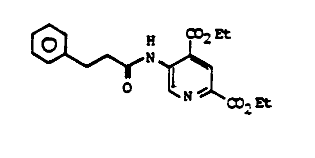

100 mg 5-Amino-pyridin-2,4-dicarbonsäurediethylester (aus Beispiel 19) werden in 10 ml Tetrahydrofuran gelöst und 20,2 mg Natriumhydrid (50 % Suspension in Mineralöl) unter Stickstoffatmosphäre langsam zugegeben. Danach wird 1 Stunde auf 60° C erwärmt, anschließend abgekühlt und 70,9 mg 3-Phenylpropionsäurechlorid in 10 ml Tetrahydrofuran bei 0_{°}C langsam dazugetropft. Die Lösung wird 6 Stunden gekocht, danach 16 Stunden bei Raumtemperatur gerührt. Die Mischung wird anschließend bei 0° C mit Wasser versetzt, mit Ether verdünnt und die organische Phase abgetrennt. Die wäßrige Phase wird nochmals mit Ether extrahiert, die vereingten organischen Phasen über Magnesiumsulfat getrocknet und das Lösungsmittel abgezogen. Nach Chromatographie an Kieselgel verbleiben 106 mg eines farblosen Öles.

### Beispiel 22

### Herstellung von 5-(3-Phenylpropylamino)-pyridin-2,4-dicarbonsäuredimethylester

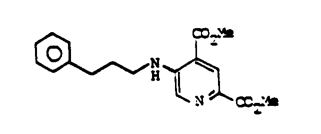

500 mg 5-Brom-pyridin-2,4-dicarbonsäuredimethylester (aus Beispiel 3) werden in 10 ml Toluol gelöst. Dazu tropft man 0,26 ml Phenylpropylamin und rührt 10 Stunden bei 120_{°}C. Nach dem Abkühlen wird das Lösungsmittel eingedampft, der Rückstand in Essigsäureethylester aufgenommen und die organische Phase jeweils 2 x mit Zitronensäure, Natriumhydrogencarbonat-Lösung und Wasser gewaschen. Nach Trocknen über Magnesiumsulfat wird das Lösungsmittel eingedampft. Nach Chromatographie an Kieselgel erhält man 117 mg Produkt mit einem Schmelzpunkt von 102-104° C.

### Beispiel 23

### Herstellung von 5-(2-Methoxycarbonyl-ethenyl)-pyridin-2,4-dicarbonsäuredimethylester

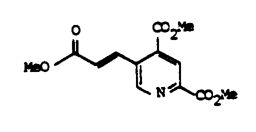

500 mg 5-Brom-pyridin-2,4-dicarbonsäuredimethylester (aus Beispiel 3) werden mit 10 ml Methylacrylat, 0,5 ml Triethylamin, 11 mg Palladiumdiacetat und 22 mg Triphenylphosphin 14 Stunden bei 140 _{°} C im Autoklaven erhitzt. Nach dem Abkühlen wird mit Essigsäureethylester verdünnt, vom Festkörper abfiltriert und das Lösungsmittel zusammen mit dem überschüssigen Methylacrylat eingedampft. Das Produkt wird chromatographiert (Kieselgel) und ergibt 330 mg eines weißen Festkörpers. Fp.: 126-128° C.

### Beispiel 24

### Herstellung von 5-(2-Methoxycarbonyl-ethyl)-pyridin-2,4-dicarbonsäuredimethylester

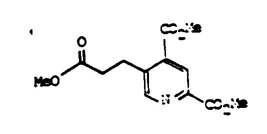

260 mg 5-(2-Methoxycarbonyl-ethenyl)-pyridin-2,4-dicarbonsäuredimethylester (aus Beispiel 23) werden in 25 ml Methanol gelöst und nach Zugabe des Palladium-Katalysators (10 % auf Kohle) hydriert. Die Umsetzung ist nach 4 Stunden beendet. Der Katalysator wird abfiltriert und die Lösung im Vakuum eingeengt. Das gelbe Öl wird chromatographiert (Kieselgel), das Produkt kristallisiert aus. Fp.: 64 C

## Patentansprüche (Patentansprüche für folgende(n) Vertragsstaat(en) : AT, BE, CH, DE, FR, GB, IT, LI, LU, NL, SE)

1. Substituierte Pyridin-2,4-dicarbonsäure-Derivate der Formel I 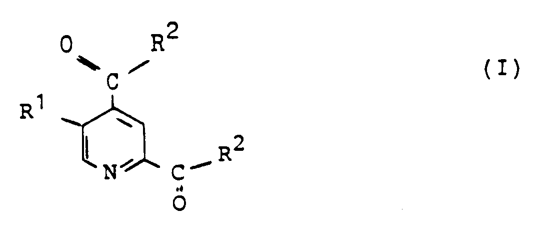 in der bedeuten:
R¹ Halogen, Carboxyl oder C₁-C₄-Alkoxycarbonyl oder
R¹ Alkyl, Alkenyl oder Alkinyl mit bis zu 9 C-Atomen, wobei die genannten Reste gegebenenfalls durch eine Carbonylgruppe unterbrochen sind und wobei die genannten Reste gegebenenfalls mono- oder disubstituiert sind mit
Halogen, Hydroxy, Nitro, Cyano, Amino, C₁-C₄-Alkoxy, Carboxyl, C1-C₄-Alkoxycarbonyl, C1-C₄-Alkylcarbonyloxy, C₁-C₄-Alkyl- oder C₁-C₄-Dialkylamino,
oder gegebenenfalls substituiert sind mit Phenyl, Naphthyl, Thienyl, Furyl, Pyrrolyl oder Pyridyl, wobei diese genannten Aryl- oder Heteroaryl-Reste gegebenenfalls ihrerseits mit Halogen, Carboxyl, Amino, C₁-C₄-Alkyl- oder C₁-C₄-Dialkylamino oder Hydroxy monosubstituiert sind, oder
R¹ Phenyl, Naphthyl, Thienyl, Furyl, Pyrrolyl oder Pyridyl, wobei diese genannten Aryl- oder Heteroaryl-Reste gegebenenfalls ihrerseits mit Carboxyl, Amino, Hydroxy, C₁-C₄-Alkyl- oder C₁-C₄-Dialkylamino, monosubstituiert sind, oder
R¹ ein Substituent der Formel -OR³, oder -N(R³)₂ ist, wobei R³ Wasserstoff ist oder C₁-C₉-Alkyl, C₁-C₉-Alkenyl, C₁-C₉-Alkinyl oder C₁-C₉-Alkylcarbonyl, wobei diese Reste gegebenenfalls mono- oder disubstituiert sind mit
Halogen, Hydroxy, Nitro, Cyano, Amino, C₁-C₄-Alkoxy, Carboxyl, C1-C₄-Alkoxycarbonyl, C₁-C₄-Alkylcarbonyloxy, C₁-C₄-Alkyl- oder C₁-C₄-Dialkylamino,
oder gegebenenfalls substituiert sind mit Phenyl, Naphthyl, Thienyl, Furyl, Pyrrolyl oder Pyridyl, wobei diese genannten Aryl- oder Heteroaryl-Reste gegebenenfalls ihrerseits mit Halogen, Carboxyl, Amino, C₁-C₄-Alkyl- oder C₁-C₄-Dialkylamino oder Hydroxy monosubstituiert sind, und wobei die beiden Substituenten R³ in -N(R³)₂ auch unabhängig voneinander verschieden sein können und
R² ein Substituent der Formel -OR⁴ oder R⁴⁻N-R⁵, wobei R⁴ Wasserstoff oder C₁-C₁₂-Alkyl, welches gegebenenfalls 1- oder 2-fach substituiert ist mit
Halogen, Hydroxy, Cyano, Carboxyl, C₁-C₄-Alkoxy, C₁-C₄-Alkoxycarbonyl, C₁-C₄-Alkylcar- bonyloxy, C₁-C₄-Alkyl- oder C1-C4-Dialkylamino oder gegebenenfalls substituiert ist mit Phenyl, welches seinerseits gegebenenfalls mit Halogen, C₁-C₄-Alkyl oder C₁-C₄-Alkoxy, 1-, 2-, oder 3-fach substituiert ist, wobei bei mehrfach Substitutionen die Substituenten auch voneinander unabhängig verschieden sein können, oder
R⁴ Cyclohexyl, welches gegebenenfalls benzoanelliert ist, oder
R⁴ Phenyl, Naphthyl, Thienyl, Furyl, Pyrrolyl oder Pyridyl, wobei gegebenenfalls die Phenyl-, Naphthyl- und Pyridylreste 1-, 2- oder 3-fach und die Thienyl-Furyl- und Pyrrolylreste 1-fach substituiert sind mit Halogen, C₁-C₄-Alkyl oder C₁-C₄-Alkoxy, wobei bei Mehrfachsubstitutionen die Substituenten auch unabhängig voneinander verschieden sein können und
R⁵ Wasserstoff oder C₁-C₃-Alkyl, wobei gegebenenfalls R⁵ im Falle der C₁-C₃-Alkyle zusammen mit R⁴, welches in diesem Falle C₃-C₅-Alkyl bedeutet, einen heterocyclischen, gesättigten 6-Ring bildet, wobei der heterocyclische Ring auch ein zweites Stickstoffatom beinhalten kann und seinerseits mit Phenyl oder Phenyl-C₁-C₃-alkyl substituiert sein kann,
und wobei die beiden über die Carbonylgruppe in 2- und 4-Stellung an das Pyridingerüst gebundenen Reste R² auch unabhängig voneinander verschieden sein können und wobei alle genannten Alkylreste mit mehr als 2 C-Atomen auch verzweigt sein können,
sowie die physiologisch verträglichen Salze, ausgenommen die Pyridin-2,4,5-tricarbonsäure und deren Trimethylester, die 5-Ethyl-Pyridin-2,4-dicarbonsäure, die Verbindungen, in denen R¹ ein Aminomethylrest ist, 5-Methyl-pyridin-2,4-dicarbonsäuredimethylester, 5-Methoxy-pyridin-2,4-dicarbonsäuredimethy- lester, Pyridin-2,4-dicarbonsäuredimethylester-5-carbonsäureethylester, Pyridin-2,5-dicarbonsäuredime- thylester-4-carbonsäuremethylester.

2. Substituierte Pyridin-2,4-dicarbonsäure-Derivate der Formel 1 nach Anspruch 1, in der bedeuten:
R¹ Halogen, Carboxyl oder
R¹ C₁-C₄-Alkyl, oder C₂-C₄-Alkenyl oder -Alkinyl, wobei die genannten Reste gegebenenfalls durch eine Carbonylgruppe unterbrochen sind und wobei die genannten Reste ihrerseits gegebenenfalls mono-substituiert sind mit Halogen, Hydroxy, Nitro, Cyano, Amino oder Carboxyl oder
R¹ Phenyl, Thienyl, Furyl oder Pyrrolyl, wobei die genannten Aryl- oder Heteroarylreste gegebenenfalls ihrerseits mit Carboxyl monosubstituiert sind oder
R¹ ein Substituent der Formel -OR³ oder -N(R³)₂ ist, wobei R³ Wasserstoff, C1-C3-Alkylcarbonyl oder C₁-C₃-Alkyl ist, wobei diese Reste ihrerseits gegebenenfalls mit Carboxyl substituiert sind oder
R³ Phenyl bedeutet, welches seinerseits gegebenenfalls mit Halogen para-substituiert ist und wobei die beiden Substituenten R³ in -N(R³)₂ auch unabhängig voneinander verschieden sein können und
R² ein Substituent der Formel -OR⁴ oder R⁴⁻N-R⁵, wobei R⁴ Wasserstoff oder C₁-C₁₂-Alkyl, welches gegebenenfalls 1- oder 2-fach substituiert ist mit Halogen, Hydroxy, Cyano, Carboxyl, C1-C4-Alkoxy, C1-C4-Alkoxycarbonyl, C₁-C₄-Alkylcarbonyloxy, C₁-C₄-Alkyl- oder C₁-C₄-Dial- kylamino oder gegebenenfalls substituiert ist mit Phenyl, welches seinerseits gegebenenfalls mit Halogen, C₁-C₄-Alkyl oder C₁-C₄-Alkoxy, 1-, 2-, oder 3-fach substituiert ist, wobei bei mehrfach Substitutionen die Substituenten auch voneinander unabhängig verschieden sein können, oder
R⁴ Cyclohexyl, welches gegebenenfalls benzoanelliert ist, oder
R⁴ Phenyl, Naphthyl, Thienyl, Furyl, Pyrrolyl oder Pyridyl, wobei gegebenenfalls die Phenyl-, Naphthyl- und Pyridylreste 1-, 2- oder 3-fach und die Thienyl-Furyl- und Pyrrolylreste 1-fach substituiert sind mit Halogen, C₁-C₄-Alkyl oder C₁-C₄-Alkoxy, wobei bei Mehrfachsubstitutionen die Substituenten auch unabhängig voneinander verschieden sein können und
R⁵ Wasserstoff oder C₁-C₃-Alkyl, wobei gegebenenfalls R⁵ im Falle der C₁-C₃-Alkyle zusammen mit R⁴, welches in diesem Falle C₃-C₅-Alkyl bedeutet, einen heterocyclischen, gesättigten 6-Ring bildet, wobei der heterocyclische 6-Ring auch ein zweites Stickstoffatom beinhalten kann und seinerseits mit Phenyl oder Phenyl-C₁-C₃-alkyl substituiert sein kann,
und wobei die beiden über die Carbonylgruppe in 2- und 4-Stellung an das Pyridingerüst gebundenen Reste R² auch unabhängig voneinander verschieden sein können und wobei alle genannten Alkylreste mit mehr als 2 C-Atomen auch verzweigt sein können,
sowie die physiologisch verträglichen Salze, ausgenommen die Pyridin-2,4,5-tricarbonsäure und deren Trimethylester, die 5-Ethyl-Pyridin-2,4-dicarbonsäure, die Verbindungen, in denen R¹ ein Aminomethylrest ist, 5-Methyl-pyridin-2,4-dicarbonsäuredimethylester, 5-Methoxy-pyridin-2,4-dicarbonsäuredimethy- lester, Pyridin-2,4-dicarbonsäuredimethylester-5-carbonsäureethylester, Pyridin-2,5-dicarbonsäuredime- thylester-4-carbonsäuremethylester.

3. Substituierte Pyridin-2,4-dicarbonsäure-Derivate der Formel I' 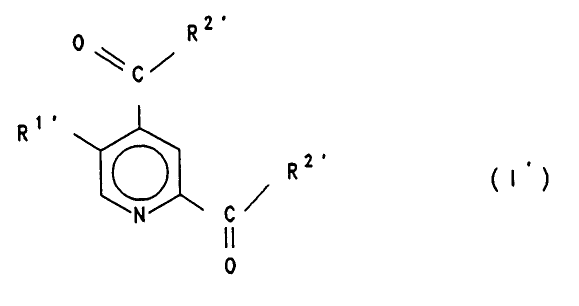 in der die Substituenten R^{1'} und R die gleiche Bedeutung wie R¹ und R² in Formel I gemäß Anspruch 1 haben, jedoch einschließlich Pyridin-2,4,5-tricarbonsäure und deren Trimethylester, 5-Ethyl-Pyridin-2,4-dicarbonsäure, die Verbindungen, in denen R^{1'} ein Aminomethylrest ist, 5-Methyl-pyridin-2,4-dicarbonsäuredimethylester, 5-Methoxy-pyridin-2,4-dicarbonsäuredimethylester, Pyridin-2,4-dicarbon- säuredimethylester-5-carbonsäureethylester, Pyridin-2,5-dicarbonsäuredimethylester-4-carbonsäuremethylester, zur Anwendung als Arzneimittel.

4. Subtituierte Pyridin-2,4-dicarbonsäure-Derivate der Formel I' nach Anspruch 3, in der R^{1'} und R die gleiche Bedeutung haben wie R¹ und R² in Formel I gemäß Anspruch 2, zur Anwendung als Arzneimittel.

5. Verfahren zur Herstellung von Verbindungen der Formel I gemäß Anspruch 1, dadurch gekennzeichnet, daß man entweder Pyridin-2,4-dimethyl-5-halogen zunächst in Pyridin-2,4,5-trimethyl überführt, welches zur Pyridin-2,4,5-tricarbonsäure oxidiert wird, welche in die Trimethoxycarbonyl-Verbindung überführt wird und anschließend zum Pyridin-2,4,5-tricarbonsäuretrikaliumsalz umgesetzt wird, oder daß man Pyridin-2,4-dimethyl-5-halogen zu einer Verbindung der Formel (1,1) oxidiert, 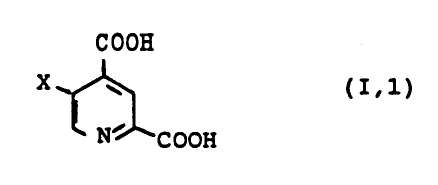 wobei X Halogen bedeutet und anschließend gegebenenfalls
a) die Verbindung der Formel (1,1) in eine Verbindung der Formel (1,2), 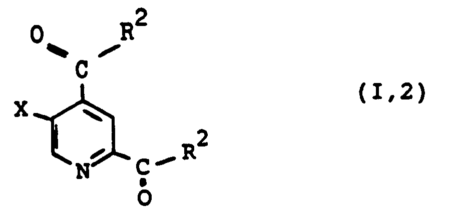 in der X Halogen ist und R² die zu Formel I gemäß Anspruch 1 angegebenen Bedeutungen hat, überführt
und gegebenenfalls anschließend die Verbindung der Formel (1,2) mit einer Verbindung der Formel II oder 11' 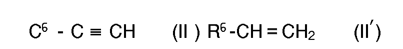 worin R⁶ C₁ -C₇-Alkyl bedeutet, welches gegebenenfalls mono- oder disubstituiert ist mit
Halogen, Hydroxy, Nitro, Cyano, Amino, C₁-C₄-Alkoxy, Carboxyl, C1-C₄-Alkoxycarbonyl, C1-C₄-Alkyl- oder C₁-C₄-Dialkylamino, oder gegebenenfalls substituiert ist mit
Phenyl, Naphthyl, Thienyl, Furyl, Pyrrolyl oder Pyridyl, wobei diese genannten Aryl- oder Heteroaryl-Reste gegebenenfalls ihrerseits mit
Halogen, Carboxyl, Amino, C₁-C₄-Alkyl- oder C₁-C₄-Dialkylamino oder Hydroxy monosubstituiert sind,
und in der gegebenenfalls vorhandene freie Carboxylgruppen gegebenenfalls geschützt sind, umsetzt und gegebenenfalls die verbliebene C-C-Dreifach- oder C-C-Doppelbindung in dem nunmehr an das Pyridingerüst gebundenen Fragment, das sich von der Verbindung der Formel II oder II' ableitet, hydriert oder gegebenenfalls die Verbindung der Formel (1,2) umsetzt mit einer Verbindung der Formel H₂ N-R³, wobei R³ die in Anspruch 1 zu Formel I angegebenen Bedeutungen hat und worin gegebenenfalls vorhandene freie Carboxylgruppen gegebenenfalls geschützt sind
oder gegebenenfalls die Verbindung der Formel (1,2) in an sich bekannter Weise in eine Verbindung der Formel (1,3) 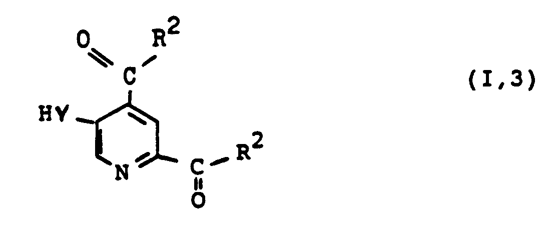 worin Y für O oder NH steht und R² die in Anspruch 1 zu Formel I angegebenen Bedeutungen hat, überführt
und dann gegebenenfalls mit einer Verbindung der Formel III oder III' 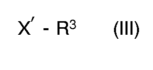 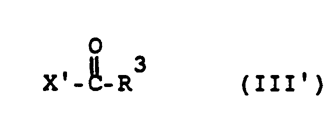 worin X' Chlor, Brom oder Jod bedeutet und R³ die in Anspruch 1 zu Formel I angegebenen Bedeutungen hat und worin gegebenenfalls vorhandene freie Carboxylgruppen gegebenenfalls geschützt sind, umsetzt
oder gegebenenfalls die Verbindung der Formel (1,2) mit einer Verbindung der Formel IV, 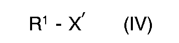 worin X' Chlor, Brom oder Jod und R¹ die in Anspruch 1 zu Formel I angegebenen Bedeutungen hat, wobei die Bedeutungen -OR³, und -N(R³)₂, in denen R³ die in Anspruch 1 zu Formel I angegebenen Bedeutungen hat, ausgenommen sind und worin gegebenenfalls vorhandene freie Carboxylgruppen gegebenenfalls geschützt sind, umsetzt
oder gegebenenfalls die Verbindung der Formel (1,2) mit einer Verbindung der Formel V 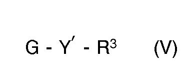 worin
Y' für O oder NR³ steht,
G für ein Alkalimetall
R³ die in Anspruch 1 zu Formel I angegebenen Bedeutungen hat und worin gegebenenfalls vorhandene freie Carboxylgruppen gegebenenfalls geschützt sind, umsetzt
oder daß man gegebenenfalls
b) die Verbindung der Formel (1,1) mit einer Verbindung der Formel II oder 11' in der gegebenenfalls vorhandene freie Carboxylgruppen gegebenenfalls geschützt sind, umsetzt und gegebenenfalls anschließend die vorhandenen Carbonsäuren in 2- und 4-Stellung des Pyridingerüstes verestert oder in die Diamide oder Ester/Amide überführt und gegebenenfalls die verbliebene C-C-Dreifachbindung oder C-C-Doppelbindung in dem nunmehr an das Pyridingerüst gebundenen Fragment, das sich von der Verbindung der Formel II oder 11' ableitet, hydriert oder gegebenenfalls die Verbindung der Formel (1,1) umsetzt mit einer Verbindung der Formel H₂N-R³, wobei R³ die in Anspruch 1 zu Formel I angegebenen Bedeutungen hat und worin gegebenenfalls vorhandene freie Carboxylgruppen gegebenenfalls geschützt sind oder
gegebenenfalls die Verbindung der Formel (1,1) in eine Verbindung der Formel (1,4), 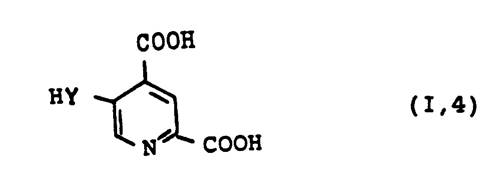 worin Y für O oder NH steht,
überführt und gegebenenfalls anschließend die vorhandenen Carbonsäuren in 2- und 4-Stellung des Pyridingerüstes verestert oder in die Diamide oder Ester/Amide überführt
und gegebenenfalls anschließend mit einer Verbindung der Formel III oder III', in der gegebenenfalls vorhandene freie Carboxylgruppen gegebenenfalls geschützt sind, umsetzt oder
gegebenenfalls die Verbindung der Formel (1,1) mit einer Verbindung der Formel IV, in der gegebenenfalls vorhandene freie Carboxylgruppen gegebenenfalls geschützt sind und wobei die Verbindungen der Formel R³0-J und (R³)₂ N-J, in denen R³ die in Anspruch 1 zu Formel I angegebenen Bedeutungen hat, ausgeschlossen sind, umsetzt oder
gegebenenfalls die Verbindung der Formel (1,1) mit einer Verbindung der Formel V, in der gegebenenfalls vorhandene freie Carboxylgruppen gegebenenfalls geschützt sind,
umsetzt und gegebenenfalls anschließend die in den nach b) erhaltenen Produkten gegebenenfalls vorhandenen Carbonsäuren in 2- und 4-Stellung des Pyridingerüstes verestert oder in die Diamide oder Ester/Amide überführt
oder daß man gegebenenfalls
c) zunächst die in der Verbindung der Formel (1,1) vorhandenen Carboxylgruppen in 2- und 4-Stellung des Pyridingerüstes mit einer Schutzgruppe schützt, wobei man eine Verbindung der Formel (1,10) erhält, 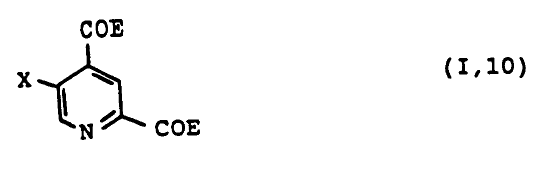 worin E eine Schutzgruppe bedeutet und gegebenenfalls anschließend
die Verbindung der Formel (1,10) mit einer Verbindung der Formel II oder II' in der gegebenenfalls vorhandene freie Carboxylgruppen gegebenenfalls geschützt sind, umsetzt und gegebenenfalls anschließend die Schutzgruppen E der Carboxylgruppen in 2- und 4-Stellung des Pyridingerüsts entweder selektiv oder gemeinsam abspaltet und gegebenenfalls die erhaltenen freien Carbonsäuren verestert oder in die Diamide oder Ester/Amide überführt und gegebenenfalls die verbliebene C-C-Dreifachbindung oder C-C-Doppelbindung in dem nunmehr an das Pyridingerüst gebundenen Fragment, das sich von der Verbindung der Formel II oder II' ableitet, hydriert
oder gegebenenfalls die Verbindung der Formel (1,10) umsetzt mit einer Verbindung der Formel H₂ N-R³, wobei R³ die in Anspruch 1 zu Formel I angegebenen Bedeutungen hat und worin gegebenenfalls vorhandene freie Carboxylgruppen gegebenenfalls geschützt sind oder
gegebenenfalls die Verbindung der Formel (1,10) in eine Verbindung der Formel (1,11), 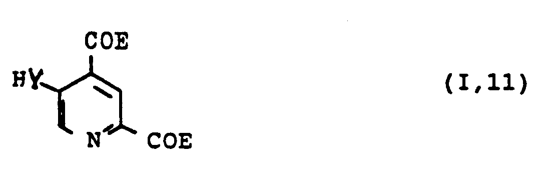 worin Y für O oder NH steht,
überführt und gegebenenfalls anschließend die Schutzgruppen E der Carboxylgruppen in 2- und 4-Stellung des Pyridingerüsts entweder selektiv oder gemeinsam abspaltet, und gegebenenfalls die erhaltenen freien Carbonsäuren verestert oder in die Diamide oder Ester/Amide überführt
und gegebenenfalls anschließend mit einer Verbindung der Formel III oder III' in der gegebenenfalls vorhandene freie Carboxylgruppen gegebenenfalls geschützt sind, umsetzt oder
gegebenenfalls die Verbindung der Formel (1,10) mit einer Verbindung der Formel IV, in der gegebenenfalls vorhandene freie Carboxylgruppen gegebenenfalls geschützt sind und wobei die Verbindungen der Formel R³0-J und (R³)₂ N-J, in denen R³ die in Anspruch 1 zu Formel I angegebenen Bedeutungen hat, ausgeschlossen sind, umsetzt oder
gegebenenfalls die Verbindung der Formel (1,10) mit einer Verbindung der Formel V, in der gegebenenfalls vorhandene freie Carboxylgruppen gegebenenfalls geschützt sind, umsetzt und gegebenenfalls anschließend in den nach c) erhaltenen Produkten die gegebenenfalls vorhandenen Schutzgruppen E der Carboxylgruppen in 2- und 4-Stellung des Pyridingerüsts entweder selektiv oder gemeinsam abspaltet und
gegebenenfalls die erhaltenen freien Carbonsäuren verestert oder in die Diamide oder Ester/Amide überführt
und anschließend gegebenenfalls die in den Produkten vorhandenen Schutzgruppen hydrolytisch oder hydrogenolytisch abspaltet und gegebenenfalls die nach a), b) oder c) erhaltenen Verbindungen in ihre physiologisch verträglichen Salze überführt.

6. Verbindungen nach einem der Ansprüche 1 bis 4 zur Inhibierung der Prolin- und Lysinhydroxylase.

7. Verbindungen nach Anspruch 1 bis 4 zur Anwendung als Fibrosupressiva und Immunsupressiva.

8. Arzneimittel, enthaltend eine Verbindung der Formel 1 oder i' mit verträglichen pharmazeutischen Trägern.

9. Verwendung von Verbindungen der Formel 1 oder I' zur Beeinflussung des Stoffwechsels von Kollagen und kollagenähnlichen Stoffen bzw. der Biosynthese von C1 q.

10. Verwendung von Verbindungen der Formel 1 oder I' zur Behandlung von Störungen des Stoffwechsels von Kollagen und kollagenähnlichen Stoffen bzw. der Biosynthese von C1 q.

11. Verfahren zur Herstellung von Arzneimitteln zur Beeinflussung des Stoffwechsels von Kollagen und kollagenähnlichen Stoffen bzw. der Biosynthese von C1q,dadurch gekennzeichnet, daß man in das Arzneimittel eine Verbindung der Formel 1 oder I' einverleibt.

## Patentansprüche (Patentansprüche für folgende(n) Vertragsstaat(en) : ES, GR)

1. Verfahren zur Herstellung von substituierten Pyridin-2,4-dicarbonsäure-Derivaten der Formel I 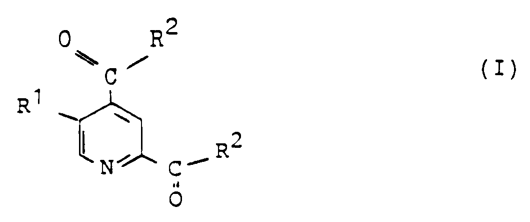 in der bedeuten:
R¹ Halogen, Carboxyl oder C₁-C₄-Alkoxycarbonyl oder
R¹ Alkyl, Alkenyl oder Alkinyl mit bis zu 9 C-Atomen, wobei die genannten Reste gegebenenfalls durch eine Carbonylgruppe unterbrochen sind und wobei die genannten Reste gegebenenfalls mono- oder disubstituiert sind mit
Halogen, Hydroxy, Nitro, Cyano, Amino, C₁-C₄-Alkoxy, Carboxyl, C1-C₄-Alkoxycarbonyl, C1-C₄-Alkylcarbonyloxy, C₁-C₄-Alkyl- oder C₁-C₄-Dialkylamino, oder gegebenenfalls substituiert sind mit
Phenyl, Naphthyl, Thienyl, Furyl, Pyrrolyl oder Pyridyl, wobei diese genannten Aryl- oder Heteroaryl-Reste gegebenenfalls ihrerseits mit
Halogen, Carboxyl, Amino, C₁-C₄-Alkyl- oder C₁-C₄-Dialkylamino oder Hydroxy monosubstituiert sind, oder
R¹ Phenyl, Naphthyl, Thienyl, Furyl, Pyrrolyl oder Pyridyl, wobei diese genannten Aryl- oder Heteroaryl-Reste gegebenenfalls ihrerseits mit
Carboxyl, Amino, Hydroxy, C₁-C₄-Alkyl- oder C₁-C₄-Dialkylamino, monosubstituiert sind, oder
R¹ ein Substituent der Formel -OR³, oder -N(R³)₂ ist, wobei R³ Wasserstoff ist oder C₁-C₉-Alkyl, C₁-C₉-Alkenyl, C₁-C₉-Alkinyl oder C₁-C₉-Alkylcarbonyl, wobei diese Reste gegebenenfalls mono- oder disubstituiert sind mit
Halogen, Hydroxy, Nitro, Cyano, Amino, C₁-C₄-Alkoxy, Carboxyl, C1-C₄-Alkoxycarbonyl, C₁-C₄-Alkylcarbonyloxy, C₁-C₄-Alkyl- oder C₁-C₄-Dialkylamino, oder gegebenenfalls substituiert sind mit
Phenyl, Naphthyl, Thienyl, Furyl, Pyrrolyl oder Pyridyl, wobei diese genannten Aryl- oder Heteroaryl-Reste gegebenenfalls ihrerseits mit
Halogen, Carboxyl, Amino, C₁-C₄-Alkyl- oder C₁-C₄-Dialkylamino oder Hydroxy monosubstituiert sind, und wobei die beiden Substituenten R³ in -N(R³)₂ auch unabhängig voneinander verschieden sein können und
R² ein Substituent der Formel -OR⁴ oder R⁴⁻N-R⁵, wobei
R⁴ Wasserstoff oder C₁-C₁₂-Alkyl, welches gegebenenfalls 1- oder 2-fach substituiert ist mit Halogen, Hydroxy, Cyano, Carboxyl, C₁-C₄-Alkoxy, C₁-C₄-Alkoxycarbonyl, C₁-C₄-Alkylcar- bonyloxy, C₁-C₄-Alkyl- oder C₁-C₄-Dialkylamino oder gegebenenfalls substituiert ist mit Phenyl, welches seinerseits gegebenenfalls mit Halogen, C₁-C₄-Alkyl oder C₁-C₄-Alkoxy, 1-, 2-, oder 3-fach substituiert ist, wobei bei mehrfach Substitutionen die Substituenten auch voneinander unabhängig verschieden sein können, oder
R⁴ Cyclohexyl, welches gegebenenfalls benzoanelliert ist, oder
R⁴ Phenyl, Naphthyl, Thienyl, Furyl, Pyrrolyl oder Pyridyl, wobei gegebenenfalls die Phenyl-, Naphthyl- und Pyridylreste 1-, 2- oder 3-fach und die Thienyl-Furyl- und Pyrrolylreste 1-fach substituiert sind mit Halogen, C₁-C₄-Alkyl oder C₁-C₄-Alkoxy, wobei bei Mehrfachsubstitutionen die Substitutionen auch unabhängig voneinander verschieden sein können und
R⁵ Wasserstoff oder C₁-C₃-Alkyl, wobei gegebenenfalls R⁵ im Falle der C₁-C₃-Alkyle zusammen mit R⁴, welches in diesem Falle C₃-C₅-Alkyl bedeutet, einen heterocyclischen, gesättigten 6-Ring bildet, wobei der heterocyclische 6-Ring auch ein zweites Stickstoffatom beinhalten kann und seinerseits mit Phenyl oder Phenyl-C₁-C₃-alkyl substituiert sein kann,
und wobei die beiden über die Carbonylgruppe in 2- und 4-Stellung an das Pyridingerüst gebundenen Reste R² auch unabhängig voneinander verschieden sein können und wobei alle genannten Alkylreste mit mehr als 2 C-Atomen auch verzweigt sein können,
sowie die physiologisch verträglichen Salze, ausgenommen die Pyridin-2,4,5-tricarbonsäure und deren Trimethylester, die 5-Ethyl-Pyridin-2,4-dicarbonsäure, die Verbindungen, in denen R¹ ein Aminomethylrest ist, 5-Methyl-pyridin-2,4-dicarbonsäuredimethylester, 5-Methoxy-pyridin-2,4-dicarbonsäuredimethy- lester, Pyridin-2,4-dicarbonsäuredimethylester-5-carbonsäureethylester, Pyridin-2,5-dicarbonsäuredime- thylester-4-carbonsäuremethylester, dadurch gekennzeichnet, daß man entweder Pyridin-2,4-dimethyl-5-halogen zunächst in Pyridin-2,4-5-trimethyl überführt, welches zur Pyridin-2,4,5-tricarbonsäure oxidiert wird, welche in die Trimethoxycarbonyl-Verbindung überführt wird und anschließend zum Pyridin-2,4,5-tricarbonsäuretrikaliumsalz umgesetzt wird, oder daß man Pyridin-2,4-dimethyl-5-halogen zu einer Verbindung der Formel (I, 1) oxidiert 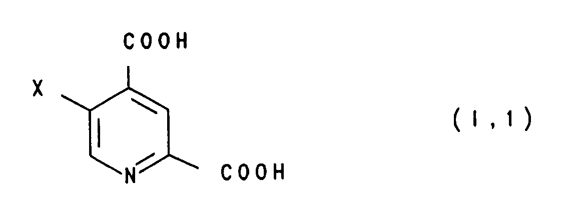 wobei X Halogen bedeutet und anschließend gegebenenfalls
a) die Verbindung der Formel (1,1) in eine Verbindung der Formel (1,2), 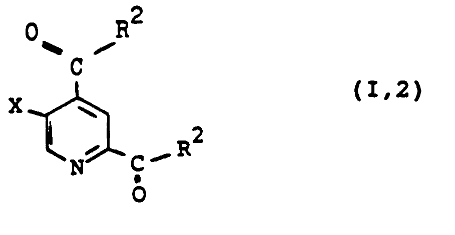 in der X Halogen ist und R² die zu Formel I gemäß Anspruch 1 angegebenen Bedeutungen hat, überführt
und gegebenenfalls anschließend die Verbindung der Formel (1,2) mit einer Verbindung der Formel II oder 11' 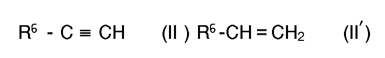 worin R⁶ C₁ -C₇-Alkyl bedeutet, welches gegebenenfalls mono- oder disubstituiert ist mit
Halogen, Hydroxy, Nitro, Cyano, Amino, C₁-C₄-Alkoxy, Carboxyl, C1-C₄-Alkoxycarbonyl, C1-C₄-Alkyl- oder C₁-C₄-Dialkylamino, oder gegebenenfalls substituiert ist mit
Phenyl, Naphthyl, Thienyl, Furyl, Pyrrolyl oder Pyridyl, wobei diese genannten Aryl- oder Heteroaryl-Reste gegebenenfalls ihrerseits mit
Halogen, Carboxyl, Amino, C1-C4-Alkyl oder C1-C4-Dialkylamino oder Hydroxy monosubstituiert sind,
und in der gegebenenfalls vorhandene freie Carboxylgruppen gegebenenfalls geschützt sind, umsetzt und gegebenenfalls die verbliebene C-C-Dreifach- oder C-C-Doppelbindung in dem nunmehr an das Pyridingerüst gebundenen Fragment, das sich von der Verbindung der Formel II oder II' ableitet, hydriert oder gegebenenfalls die Verbindung der Formel (1,2) umsetzt mit einer Verbindung der Formel H₂ N-R³, wobei R³ die in Anspruch 1 zu Formel I angegebenen Bedeutungen hat und worin gegebenenfalls vorhandene freie Carboxylgruppen gegebenenfalls geschützt sind oder gegebenenfalls die Verbindung der Formel (1,2) in an sich bekannter Weise in eine Verbindung der Formel (1,3) 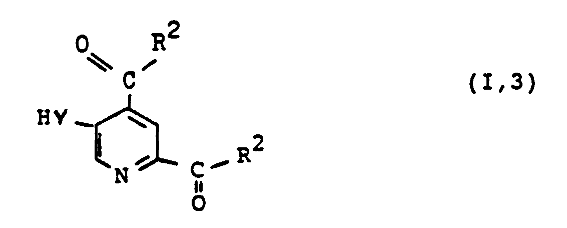 worin Y für O oder NH steht und R² die in Anspruch 1 zu Formel I angegebenen Bedeutungen hat, überführt
und dann gegebenenfalls mit einer Verbindung der Formel III oder III' 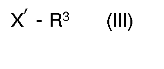 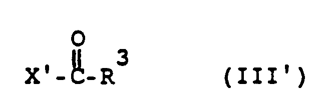 worin X' Chlor, Brom oder Jod bedeutet und R³ die in Anspruch 1 zu Formel I angegebenen Bedeutungen hat und worin gegebenenfalls vorhandene freie Carboxylgruppen gegebenenfalls geschützt sind, umsetzt
oder gegebenenfalls die Verbindung der Formel (1,2) mit einer Verbindung der Formel IV, 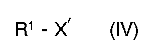 worin X' Chlor, Brom oder Jod und R¹ die in Anspruch 1 zu Formel I angegebenen Bedeutungen hat, wobei die Bedeutungen -OR³, und -N(R³)₂, in denen R³ die in Anspruch 1 zu Formel I angegebenen Bedeutungen hat, ausgenommen sind und worin gegebenenfalls vorhandene freie Carboxylgruppen gegebenenfalls geschützt sind, umsetzt
oder gegebenenfalls die Verbindung der Formel (1,2) mit einer Verbindung der Formel V 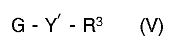 worin
Y' für O oder NR³ steht,
G für ein Alkalimetall
R³ die in Anspruch 1 zu Formel I angegebenen Bedeutungen hat
und worin gegebenenfalls vorhandene freie Carboxylgruppen gegebenenfalls geschützt sind, umsetzt
oder daß man gegebenenfalls
b) die Verbindung der Formel (1,1) mit einer Verbindung der Formel II oder 11' in der gegebenenfalls vorhandene freie Carboxylgruppen gegebenenfalls geschützt sind, umsetzt und gegebenenfalls anschließend die vorhandenen Carbonsäuren in 2- und 4-Stellung des Pyridingerüstes verestert oder in die Diamide oder Ester/Amide überführt und gegebenenfalls die verbliebene C-C-Dreifachbindung oder C-C-Doppelbindung in dem nunmehr an das Pyridingerüst gebundenen Fragment, das sich von der Verbindung der Formel II oder 11' ableitet, hydriert oder gegebenenfalls die Verbindung der Formel (1,1) umsetzt mit einer Verbindung der Formel H₂N-R³, wobei R³ die in Anspruch 1 zu Formel I angegebenen Bedeutungen hat und worin gegebenenfalls vorhandene freie Carboxylgruppe gegebenenfalls geschützt sind oder
gegebenenfalls die Verbindung der Formel (1,1) in eine Verbindung der Formel (1,4), 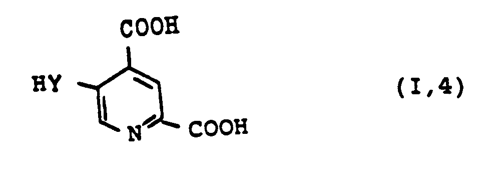 worin Y für O oder NH steht,
überführt und gegebenenfalls anschließend die vorhandenen Carbonsäuren in 2- und 4-Stellung des Pyridingerüstes verestert oder in die Diamide oder Ester/Amide überführt
und gegebenenfalls anschließend mit einer Verbindung der Formel III oder III', in der gegebenenfalls vorhandene freie Carboxylgruppen gegebenenfalls geschützt sind, umsetzt oder
gegebenenfalls die Verbindung der Formel (1,1) mit einer Verbindung der Formel IV, in der gegebenenfalls vorhandene freie Carboxylgruppen gegebenenfalls geschützt sind und wobei die Verbindungen der Formel R³0-J und (R³)₂ N-J,
in denen R³ die in Anspruch 1 zu Formel I angegebenen Bedeutungen hat, ausgeschlossen sind, umsetzt oder
gegebenenfalls die Verbindung der Formel (1,1) mit einer Verbindung der Formel V, in der gegebenenfalls vorhandene freie Carboxylgruppen gegebenenfalls geschützt sind,
umsetzt und gegebenenfalls anschließend die in den nach b) erhaltenen Produkten gegebenenfalls vorhandenen Carbonsäuren in 2- und 4-Stellung des Pyridingerüstes verestert oder in die Diamide oder Ester/Amide überführt oder daß man gegebenenfalls
c) zunächst die in der Verbindung der Formel (1,1) vorhandenen Carboxylgruppen in 2- und 4-Stellung des Pyridingerüstes mit einer Schutzgruppe schützt, wobei man eine Verbindung der Formel (1,10) erhält, 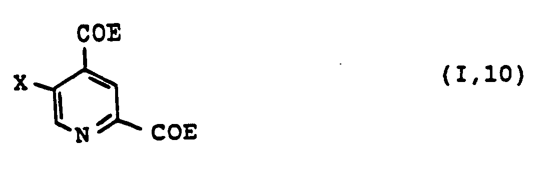 worin E eine Schutzgruppe bedeutet und gegebenenfalls anschließend
die Verbindung der Formel (1,10) mit einer Verbindung der Formel II oder 11' in der gegebenenfalls vorhandene freie Carboxylgruppen gegebenenfalls geschützt sind, umsetzt und gegebenenfalls anschließend die Schutzgruppen E der Carboxylgruppen in 2- und 4-Stellung des Pyridingerüsts entweder selektiv oder gemeinsam abspaltet und gegebenenfalls die erhaltenen freien Carbonsäuren verestert oder in die Diamide oder Ester/Amide überführt und gegebenenfalls die verbliebene C-C-Dreifachbindung oder C-C-Doppelbindung in dem nunmehr an das Pyridingerüst gebundenen Fragment, das sich von der Verbindung der Formel II oder 11' ableitet, hydriert
oder gegebenenfalls die Verbindung der Formel (1,10) umsetzt mit einer Verbindung der Formel H₂ N-R³, wobei R³ die in Anspruch 1 zu Formel I angegebenen Bedeutungen hat und worin gegebenenfalls vorhandene freie Carboxylgruppen gegebenenfalls geschützt sind oder
gegebenenfalls die Verbindung der Formel (1,10) in eine Verbindung der Formel (1,11), 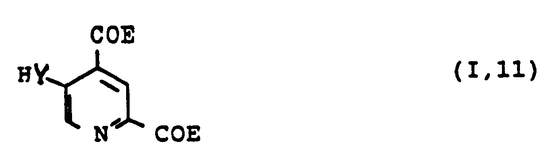 worin Y für O oder NH steht,
überführt und gegebenenfalls anschließend die Schutzgruppen E der Carboxylgruppen in 2- und 4-Stellung des Pyridingerüsts entweder selektiv oder gemeinsam abspaltet, und gegebenenfalls die erhaltenen freien Carbonsäuren verestert oder in die Diamide oder Ester/Amide überführt
und gegebenenfalls anschließend mit einer Verbindung der Formel III oder III' in der gegebenenfalls vorhandene freie Carboxylgruppen gegebenenfalls geschützt sind, umsetzt oder
gegebenenfalls die Verbindung der Formel (1,10) mit einer Verbindung der Formel IV, in der gegebenenfalls vorhandene freie Carboxylgruppen gegebenenfalls geschützt sind und wobei die Verbindungen der Formel R³0-J und (R³)₂ N-J, in denen R³ die in Anspruch 1 zu Formel I angegebenen Bedeutungen hat, ausgeschlossen sind, umsetzt oder
gegebenenfalls die Verbindung der Formel (1,10) mit einer Verbindung der Formel V, in der gegebenenfalls vorhandene freie Carboxylgruppen gegebenenfalls geschützt sind, umsetzt und gegebenenfalls anschließend in den nach c) erhaltenen Produkten die gegebenenfalls vorhandenen Schutzgruppen E der Carboxylgruppen in 2- und 4-Stellung des Pyridingerüsts entweder selektiv oder gemeinsam abspaltet und
gegebenenfalls die erhaltenen freien Carbonsäuren verestert oder in die Diamide oder Ester/Amide überführt
und anschließend gegebenenfalls die in den Produkten vorhandenen Schutzgruppen hydrolytisch oder hydrogenolytisch abspaltet und gegebenenfalls die nach a), b) oder c) erhaltenen Verbindungen in ihre physiologisch verträglichen Salze überführt.
2. Verfahren nach Anspruch 1, dadurch gekennzeichnet, daß substituierte Pyridin-2,4-dicarbonsäure-Derivate der Formel I hergestellt werden, in der bedeuten:
R¹ Halogen, Carboxyl oder
R¹ C1-C4-Alkyl, oder C₂-C₄-Alkenyl oder -Alkinyl, wobei die genannten Reste gegebenenfalls durch eine Carbonylgruppe unterbrochen sind und wobei die genannten Reste ihrerseits gegebenenfalls mono-substituiert sind mit Halogen, Hydroxy, Nitro, Cyano, Amino oder Carboxyl oder
R¹ Phenyl, Thienyl, Furyl oder Pyrrolyl, wobei die genannten Aryl- oder Heteroarylreste gegebenenfalls ihrerseits mit Carboxyl monosubstituiert sind oder
R¹ ein Substituent der Formel -OR³ oder -N(R³)₂ ist, wobei R³ Wasserstoff, C1-C3-Alkylcarbonyl oder C₁-C₃-Alkyl ist, wobei diese Reste ihrerseits gegebenenfalls mit Carboxyl substituiert sind oder
R³ Phenyl bedeutet, welches seinerseits gegebenenfalls mit Halogen para-substituiert ist und wobei die beiden Substituenten R³ in -N(R³)₂ auch unabhängig voneinander verschieden sein können und
R² ein Substituent der Formel -OR⁴ oder R⁴⁻N-R⁵, wobei R⁴ Wasserstoff oder C₁-C₁₂-Alkyl, welches gegebenenfalls 1- oder 2-fach substituiert ist mit
Halogen, Hydroxy, Cyano, Carboxyl, C₁-C₄-Alkoxy, C₁-C₄-Alkoxycarbonyl, C₁-C₄-Alkylcar- bonyloxy, C1-C4-Alkyl- oder C1-C4-Dialkylamino oder gegebenenfalls substituiert ist mit Phenyl, welches seinerseits gegebenenfalls mit Halogen, C₁-C₄-Alkyl oder C₁-C₄-Alkoxy, 1-, 2-, oder 3-fach substituiert ist, wobei bei mehrfach Substitutionen die Substituenten auch voneinander unabhängig verschieden sein können, oder
R⁴ Cyclohexyl, welches gegebenenfalls benzoanelliert ist, oder
R⁴ Phenyl, Naphthyl, Thienyl, Furyl, Pyrrolyl oder Pyridyl, wobei gegebenenfalls die Phenyl-, Naphthyl- und Pyridylreste 1-, 2- oder 3-fach und die Thienyl-Furyl- und Pyrrolylreste 1-fach substituiert sind mit Halogen, C₁-C₄-Alkyl oder C₁-C₄-Alkoxy, wobei bei Mehrfachsubstitutionen die Substituenten auch unabhängig voneinander verschieden sein können und
R⁵ Wasserstoff oder C₁-C₃-Alkyl, wobei gegebenenfalls R⁵ im Falle der C₁-C₃-Alkyle zusammen mit R⁴, welches in diesem Falle C₃-C₅-Alkyl bedeutet, einen heterocyclischen, gesättigten 6-Ring bildet, wobei der heterocyclische 6-Ring auch ein zweites Stickstoffatom beinhalten kann und seinerseits mit Phenyl oder Phenyl-C₁-C₃-alkyl substituiert sein kann,
und wobei die beiden über die Carbonylgruppe in 2- und 4-Stellung an das Pyridingerüst gebundenen Reste R² auch unabhängig voneinander verschieden sein können und wobei alle genannten Alkylreste mit mehr als 2 C-Atomen auch verzweigt sein können,
sowie die physiologisch verträglichen Salze, ausgenommen die Pyridin-2,4,5-tricarbonsäure und deren Trimethylester, die 5-Ethyl-Pyridin-2,4-dicarbonsäure, die Verbindungen, in denen R¹ ein Aminomethylrest ist, 5-Methyl-pyridin-2,4-dicarbonsäuredimethylester, 5-Methoxy-pyridin-2,4-dicarbonsäuredimethy- lester, Pyridin-2,4-dicarbonsäuredimethylester-5-carbonsäureethylester, Pyridin-2,5-dicarbonsäuredime- thylester-4-carbonsäuremethylester.
3. Verfahren nach Anspruch 1, dadurch gekennzeichnet, daß substituierte Pyridin-2,4-dicarbonsäure-Derivate der Formel I' hergestellt werden, 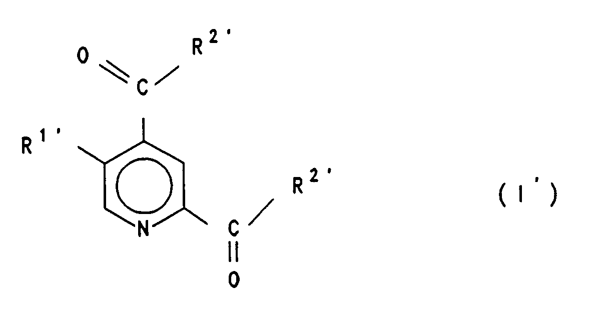 in der die Substituenten R^{1'} und R^{2'} die gleiche Bedeutung wie R¹ und R² in Formel I gemäß Anspruch 1 haben, jedoch einschließlich Pyridin-2,4,5-tricarbonsäure und deren Trimethylester, 5-Ethyl-Pyridin-2,4-dicarbonsäure, die Verbindungen, in denen R^{1'} ein Aminomethylrest ist, 5-Methyl-pyridin-2,4-dicarbonsäuredimethylester, 5-Methoxy-pyridin-2,4-dicarbonsäuredimethylester, Pyridin-2,4-dicarbon- säuredimethylester-5-carbonsäureethylester, Pyridin-2,5-dicarbonsäuredimethylester-4-carbonsäuremethylester, zur Anwendung als Arzneimittel.
4. Verfahren nach Anspruch 3, dadurch gekennzeichnet, daß Pyridin-2,4-dicarbonsäure-Derivate der Formel I' hergestellt werden, in der R^{1'} und R²' die gleiche Bedeutung haben wie R¹ und R² in Formel gemäß Anspruch 2, zur Anwendung als Arzneimittel.
5. Verwendung der Verbindungen, hergestellt nach einem oder mehreren der Ansprüche 1 bis 4, zur Inhibierung der Prolin- und Lysinhydroxylase.
6. Verwendung der Verbindungen, hergestellt nach einem oder mehreren der Ansprüche 1 bis 4, als Fibrosupressiva und Immunsupressiva.
7. Arzneimittel, enthaltend eine Verbindung der Formel I oder I' mit verträglichen pharmazeutischen Trägern.
8. Verwendung von Verbindungen der Formel I oder I' zur Beeinflussung des Stoffwechsels von Kollagen und kollagenähnlichen Stoffen bzw. der Biosynthese von C1 q.
9. Verwendung von Verbindungen der Formel I oder I' zur Behandlung von Störungen des Stoffwechsels von Kollagen und kollagenähnlichen Stoffen bzw. der Biosynthese von C1 q.
10. Verfahren zur Herstellung von Arzneimitteln zur Beeinflussung des Stoffwechsels von Kollagen und kollagenähnlichen Stoffen bzw. der Biosynthese von C1q,dadurch gekennzeichnet, daß man in das Arzneimittel eine Verbindung der Formel I oder I' einverleibt.

## Claims (Claims for the following Contracting State(s) : AT, BE, CH, DE, FR, GB, IT, LI, LU, NL, SE)

1. A substituted pyridine-2,4-dicarboxylic acid derivative of the formula I 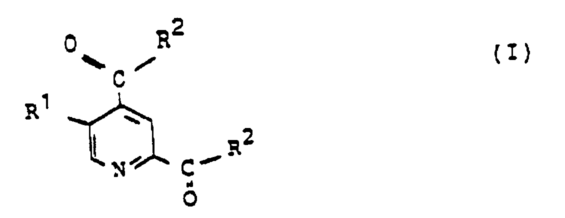 in which
R¹ denotes halogen, carboxyl or C₁-C₄-alkoxycarbonyl or
R¹ denotes alkyl, alkenyl or alkynyl with up to 9 carbon atoms, the radicals mentioned being optionally interrupted by a carbonyl group and the radicals mentioned being optionally mono- or disubstituted by
halogen, hydroxyl, nitro, cyano, amino, C₁-C₄-alkoxy, carboxyl, C₁-C₄-alkoxycarbonyl, C₁-C₄- alkylcarbonyloxy or C₁-C₄-alkyl- or C₁-C₄-dialkylamino, or optionally substituted by
phenyl, naphthyl, thienyl, furyl, pyrrolyl or pyridyl, these aryl or heteroaryl radicals mentioned being in turn optionally monosubstituted by halogen, carboxyl, amino, C₁-C₄-alkyl-or C₁-C₄- dialkylamino or hydroxyl, or
R¹ denotes phenyl, naphthyl, thienyl, furyl, pyrrolyl or pyridyl, these aryl or heteroaryl radicals mentioned being in turn optionally monosubstituted by carboxyl, amino, hydroxyl or C₁-C4-alkyl- or C₁-C₄-dialkylamino, or
R¹ is a substituent of the formula -OR³, or -N(R³)₂, in which R³ is hydrogen or C₁-C₉-alkyl, C₁-C^{g-}alkenyl, C₁-C₉-alkynyl or C₁-C₉-alkylcarbonyl, these radicals being optionally monoor disubstituted by
halogen, hydroxyl, nitro, cyano, amino, C₁ -C₄-alkoxy, carboxyl, C₁-C₄-alkoxycarbonyl, C₁-C₄- alkylcarbonyloxy or C₁-C₄-alkyl- or C₁-C₄-dialkylamino, or optionally substituted by
phenyl, naphthyl, thienyl, furyl, pyrrolyl or pyridyl, these aryl or heteroaryl radicals mentioned being in turn optionally monosubstituted by halogen, carboxyl, amino, Ci-C4-alkyl-or C₁-C₄- dialkylamino or hydroxyl,
and it also being possible for the two substituents R³ in -N(R³)₂ to differ independently of one another, and
R² denotes a substituent of the formula -OR⁴ or R⁴⁻N-R⁵, in which
R⁴ denotes hydrogen or C₁-C₁₂-alkyl, which is optionally mono- or disubstituted by halogen, hydroxyl, cyano, carboxyl, C1-C4-alkoxy, C₁-C₄-alkoxycarbonyl, C₁-C₄-alkylcarbonyloxy, or C₁-C₄-alkyl- or C₁-C₄-dialkylamino, or is optionally substituted by phenyl, which is in turn optionally mono-, di- or trisubstituted by halogen, C₁-C₄-alkyl or C₁-C₄-alkoxy, it also being possible for the substituents to differ independently of one another in the case of polysubstitution, or
R⁴ denotes cyclohexyl, which is optionally benzofused, or
R⁴ denotes phenyl, naphthyl, thienyl, furyl, pyrrolyl or pyridyl, the phenyl, naphthyl and pyridyl radicals being optionally mono-, di- or trisubstituted and the thienyl, furyl and pyrrolyl radicals being optionally monosubstituted by halogen, C1-C4-alkyl or C1-C4-alkoxy, it also being possible for the substituents to differ independently of one another in the case of polysubstitution, and
R⁵ denotes hydrogen or C₁-C₃-alkyl, R⁵ in the case of C₁-C₃-alkyl radicals together with R⁴, which in this case denotes C₃-C₅-alkyl, optionally forming a heterocyclic saturated 6- membered ring, it also being possible for the heterocyclic ring to contain a second nitrogen atom and in turn to be substituted by phenyl or phenyl-C₁-C₃-alkyl,
and it also being possible for the two radicals R² bonded to the pyridine skeleton via the carbonyl group in the 2- and 4-position to differ independently of one another, and it also being possible for all the alkyl radicals mentioned with more than 2 carbon atoms to be branched, or a physiologically tolerated salt, excluding pyridine-2,4,5-tricarboxylic acid and its trimethyl ester, 5-ethylpyridine-2,4-dicarboxylic acid, the compounds in which R¹ is an aminomethyl radical, dimethyl 5-methylpyridine-2,4-dicarboxylate, dimethyl 5-methoxypyridine-2,4-dicarboxylate, dimethyl 5-ethoxycarbonylpyridine-2,4-dicarboxylate and dimethyl 4-ethoxycarbonylpyridine-2,5-dicarboxylate.

2. A substituted pyridine-2,4-dicarboxylic acid derivative of the formula I as claimed in claim 1, in which:
R¹ denotes halogen or carboxyl, or
R¹ denotes C₁-C₄-alkyl or C₂-C₄-alkenyl or -alkynyl, the radicals mentioned being optionally interrupted by a carbonyl group and the radicals mentioned being in turn optionally monosubstituted by halogen, hydroxyl, nitro, cyano, amino or carboxyl, or
R¹ denotes phenyl, thienyl, furyl or pyrrolyl, the aryl or heteroaryl radicals mentioned being in turn optionally monosubstituted by carboxyl, or
R¹ is a substituent of the formula -OR³ or -N(R³)₂, in which R³ is hydrogen, C₁-C₃-alkylcarbonyl or C₁-C₃-alkyl, these radicals being in turn optionally substituted by carboxyl, or
R³ denotes phenyl, which is in turn optionally para-substituted by halogen, and it also being possible for the two substituents R³ in -N(R³)₂ to differ independently of one another, and
R² denotes a substituent of the formula -OR⁴ or R⁴⁻N-R^{s}, in which R⁴ denotes hydrogen or C₁-C₁₂-alkyl, which is optionally mono- or disubstituted by halogen, hydroxyl, cyano, carboxyl, C1-C4-alkoxy, C₁-C₄-alkoxycarbonyl, C₁-C₄-alkylcarbonyloxy or C₁-C₄-alkyl- or C₁-C₄-dial- kylamino, or is optionally substituted by phenyl, which is in turn optionally mono-, di- or trisubstituted by halogen, C₁-C₄-alkyl or C₁-C₄-alkoxy, it also being possible for the substituents to differ independently of one another in the case of polysubstitution, or
R⁴ denotes cyclohexyl, which is optionally benzofused, or
R⁴ denotes phenyl, naphthyl, thienyl, furyl, pyrrolyl or pyridyl, the phenyl, naphthyl and pyridyl radicals being optionally mono-, di- or trisubstituted and the thienyl, furyl and pyrrolyl radicals being optionally monosubstituted by halogen, C1-C4-alkyl or C₁-C₄-alkoxy, it also being possible for the substituents to differ independently of one another in the case of polysubstitution, and
R⁵ denotes hydrogen or C₁-C₃-alkyl, R⁵ in the case of the C₁-C₃-alkyl radicals together with R⁴, which in this case denotes C₃-C₅-alkyl, optionally forming a heterocyclic saturated 6- membered ring, it also being possible for the heterocyclic 6-membered ring to contain a second nitrogen atom and to be in turn substituted by phenyl or phenyl-C₁-C₃-alkyl,
and it also being possible for the two radicals R² bonded to the pyridine skeleton via the carbonyl group in the 2- and 4-position to differ independently of one another, and it also being possible for all the alkyl radicals mentioned with more than 2 carbon atoms to be branched, or a physiologically tolerated salt, excluding pyridine-2,4,5-tricarboxylic acid and its trimethyl ester, 5-ethylpyridine-2,4-dicarboxylic acid, the compounds in which R¹ is an aminomethyl radical, dimethyl 5-methylpyridine-2,4-dicarboxylate, dimethyl 5-methoxypyridine-2,4-dicarboxylate, dimethyl 5-ethoxycarbonylpyridine-2,4-dicarboxylate and dimethyl 4-ethoxycarbonylpyridine-2,5-dicarboxylate.

3. A substituted pyridine-2,4-dicarboxylic acid derivative of the formula I' 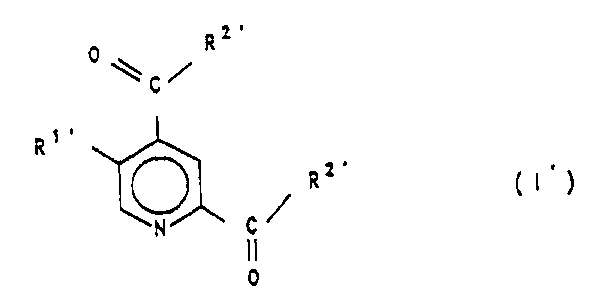 in which the substituents R^{1'} and R have the same meaning as R¹ and R² in formula I as claimed in claim 1, but including pyridine-2,4,5-tricarboxylic acid and its trimethyl ester, 5-ethyl-pyridine-2,4-dicarboxylic acid, the compounds in which R^{1'} is an aminomethyl radical, dimethyl 5-methylpyridine-2,4-dicarboxylate, dimethyl 5-methoxypyridine-2,4-dicarboxylate, dimethyl 5-ethoxycarbonylpyridine-2,4-dicarbloxylate and dimethyl 4-ethoxycarbonylpyridine-2,5-dicarboxylate, for use as a medicament.

4. A substituted pyridine-2,4-dicarboxylic acid derivative of the formula I' as claimed in claim 3, in which R^{1'} and R have the same meaning as R¹ and R² in formula I as claimed in claim 2, for use as a medicament.

5. A process for the preparation of a compound of the formula I as claimed in claim 1, which comprises either converting a 2,4-dimethyl-5-halopyridine first into 2,4,5-trimethylpyridine and oxidizing this to pyridine-2,4,5-tricarboxylic acid, which is converted into the trimethoxycarbonyl compound and then reacted to give tripotassium pyridine-2,4,5-tricarboxylate, or oxidizing a 2,4-dimethyl-5-halopyridine to give a compound of the formula (1,1) 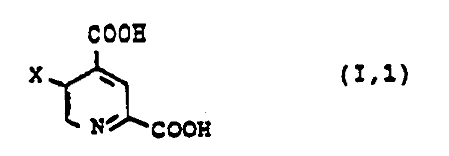 in which X denotes halogen and subsequently, if appropriate,
a) converting the compound of the formula (1,1) into a compound of the formula (1,2) 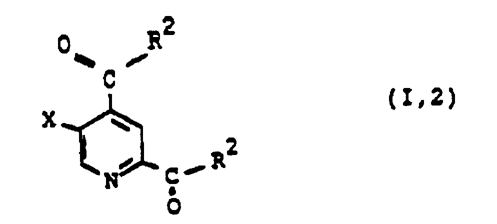 in which X is halogen and R² has the meanings given in the case of formula I as claimed in claim 1, and, if appropriate, subsequently reacting the compound of the formula (1,2) with a compound of the formula II or II' 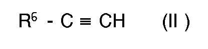 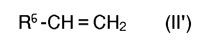 in which R⁶ denotes C₁ -C₇-alkyl, which is optionally mono- or disubstituted by
halogen, hydroxyl, nitro, cyano, amino, C₁-C₄-alkoxy, carboxyl, C₁-C₄-alkoxycarbonyl or C₁-C₄-alkyl-or C₁-C₄-dialkylamino, or is optionally substituted by
phenyl, naphthyl, thienyl, furyl, pyrrolyl or pyridyl, these aryl or heteroaryl radicals mentioned being in turn optionally monosubstituted by halogen, carboxyl, amino, C₁-C₄-alkyl- or C1-C4-dialkylamino or hydroxyl,
and in which any free carboxyl groups present are optionally protected,
and, if appropriate, hydrogenating the remaining C-C triple or C-C double bond in the fragment which is now bonded to the pyridine skeleton and is derived from the compound of the formula II or 11', or if appropriate reacting the compound of the formula (1,2) with a compound of the formula H₂-N-R³, in which R³ has the meanings given in the case of formula I in claim 1 and in which any free carboxyl groups present are optionally protected,
or, if appropriate, converting the compound of the formula (1,2) into a compound of the formula (1,3) 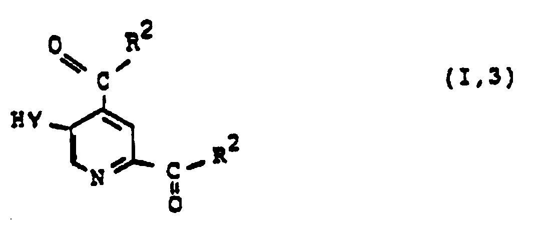 in which Y stands for O or NH and R² has the meanings given in the case of formula I in claim 1, in a manner which is known per se,
and then, if appropriate, reacting the product with a compound of the formula III or III' 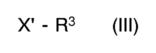 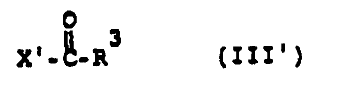 in which X' denotes chlorine, bromine or iodine and R³ has the meanings given in the case of formula I in claim 1, and in which any free carboxyl groups present are optionally protected, or, if appropriate, reacting the compound of the formula (1,2) with a compound of the formula IV 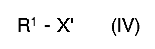 in which X' is chlorine, bromine or iodine and R¹ has the meanings given in the case of formula I in claim 1, excluding the meanings -OR³ and -N(R³)₂, in which R³ has the meanings given in the case of formula I in claim 1, and in which any free carboxyl groups present are optionally protected, or, if appropriate, reacting the compound of the formula (1,2) with a compound of the formula V 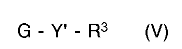 in which
Y' stands for O or NR³,
G stands for an alkali metal and
R³ has the meanings given in the case of formula I in claim 1,
and in which any free carboxyl groups present are optionally protected, or, if appropriate,
b) reacting the compound of the formula (1,1) with a compound of the formula II or 11', in which any free carboxyl groups present are optionally protected, and if appropriate then esterifying the carboxylic acids present in the 2- and 4-position of the pyridine skeleton or converting them into the diamides or ester/amides, and if appropriate hydrogenating the remaining C-C triple bond or C-C double bond in the fragment which is now bonded to the pyridine skeleton and is derived from the compound of the formula II or 11', or if appropriate reacting the compound of the formula (1,1) with a compound of the formula H₂ N-R³, in which R³ has the meanings given in the case of formula I in claim 1 and in which any free carboxyl groups present are optionally protected, or
if appropriate converting the compound of the formula (1,1) into a compound of the formula (1,4) 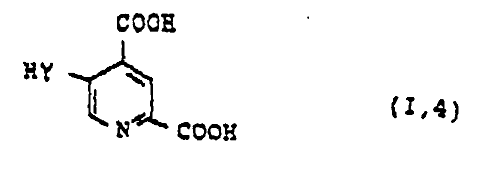 in which Y stands for O or NH,
and if appropriate then esterifying the carboxylic acids present in the 2- and 4-position of the pyridine skeleton, or converting them into the diamides or ester/amides,
and, if appropriate, subsequently reacting the product with a compound of the formula III or III', in which any free carboxyl groups present are optionally protected, or
if appropriate reacting the compound of the formula (1,1) with a compound of the formula IV, in which any free carboxyl groups present are optionally protected, the compounds of the formula R³0-1 and (R³)₂ N-I, in which R³ has the meanings given in the case of formula I in claim 1, being excluded, or
if appropriate reacting the compound of the formula (1,1) with a compound of the formula V, in which any free carboxyl groups present are optionally protected, and if appropriate then esterifying the carboxylic acids optionally present in the 2- and 4-position of the pyridine skeleton in the products obtained according to b), or converting them into the diamides or ester/amides, or, if appropriate,
c) first protecting the carboxyl groups present in the 2- and 4-position of the pyridine skeleton in the compound of the formula (1,1) with a protective group, to give a compound of the formula (1,10) 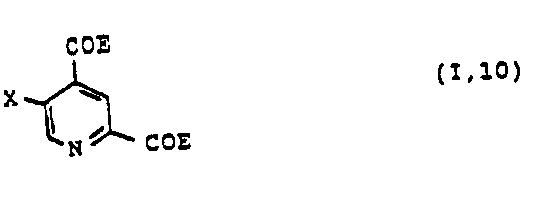 in which E denotes a protective group, and, if appropriate, subsequently
reacting the compound of the formula (1,10) with a compound of the formula II or 11', in which any free carboxyl groups present are optionally protected, and if appropriate then splitting off the protective groups E of the carboxyl groups in the 2- and 4-position of the pyridine skeleton either selectively or together, and if appropriate esterifying the resulting free carboxylic acids or converting them into the diamides or ester/amides, and if appropriate hydrogenating the remaining C-C triple bond or C-C double bond in the fragment which is now bonded to the pyridine skeleton and is derived from the compound of the formula II or 11',
or if appropriate reacting the compound of the formula (1,10) with a compound of the formula H₂N-R³, in which R³ has the meanings given in the case of formula I in claim 1 and in which any free carboxyl groups present are optionally protected, or
if appropriate converting the compound of the formula (1,10) into a compound of the formula (1,11) 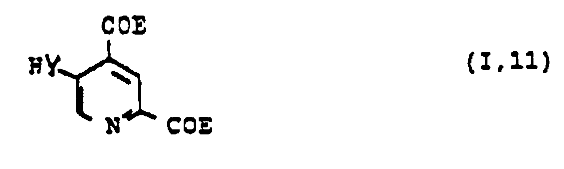 in which Y stands for O or NH,
and if appropriate then splitting off the protective groups E of the carboxyl groups in the 2- and 4- position of the pyridine skeleton either selectively or together, and if appropriate esterifying the resulting free carboxylic acid or converting it into the diamide or ester/amide,
and if appropriate subsequently reacting the product with a compound of the formula III or III', in which any free carboxyl groups present are optionally protected, or
if appropriate reacting the compound of the formula (1,10) with a compound of the formula IV, in which any free carboxyl groups present are optionally protected, the compounds of the formula R³0-I and (R³)₂ N-I in which R³ has the meanings given in the case of formula I in claim 1 being excluded, or
if appropriate reacting the compound of the formula (1,10) with a compound of the formula V, in which any free carboxyl groups present are optionally protected, and, if appropriate, subsequently splitting off any protective groups E of the carboxyl groups in the 2- and 4-position of the pyridine skeleton, either selectively or together, in a product obtained according to c), and
if appropriate esterifying the resulting free carboxylic acid or converting it into the diamide or ester/amide,
and if appropriate subsequently splitting off the protective groups present in the product hydrolytically or hydrogenolytically, and if appropriate converting the compound obtained according to a), b) or c) into one of its physiologically tolerated salts.

6. A compound as claimed in any one of claims 1 to 4 for inhibiting proline hydroxylase and lysine hydroxylase.

7. A compound as claimed in claim 1 to 4 for use as a fibrosuppressant and immunosuppressant.

8. A medicament containing a compound of the formula I or I' with tolerated pharmaceutical excipients.

9. The use of a compound of the formula I or I' for influencing the metabolism of collagen and collagen- like substances and/or the biosynthesis of C1 q.

10. The use of a compound of the formula I or I' for the treatment of disturbances in the metabolism of collagen and collagen-like substances and/or the biosynthesis of C1 q.

11. A process for the preparation of medicaments for influencing the metabolism of collagen and collagen- like substances and/or the biosynthesis of C1 q, which comprises incorporating a compound of the formula I or I' into the medicament.

## Claims (Claims for the following Contracting State(s) : ES, GR)

1. A process for the preparation of a substituted pyridine-2,4-dicarboxylic acid derivative of the formula I 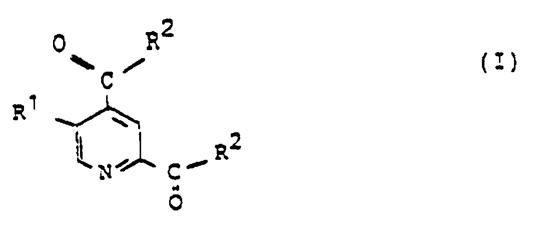 in which
R¹ denotes halogen, carboxyl or C₁-C₄-alkoxycarbonyl or
R¹ denotes alkyl, alkenyl or alkynyl with up to 9 carbon atoms, the radicals mentioned being optionally interrupted by a carbonyl group and the radicals mentioned being optionally monoor disubstituted by
halogen, hydroxyl, nitro, cyano, amino, C₁-C₄-alkoxy, carboxyl, C₁-C₄-alkoxycarbonyl, C₁-C₄- alkylcarbonyloxy or C₁-C₄-alkyl- or C₁-C₄-dialkylamino, or optionally substituted by
phenyl, naphthyl, thienyl, furyl, pyrrolyl or pyridyl, these aryl or heteroaryl radicals mentioned being in turn optionally monosubstituted by halogen, carboxyl, amino, C₁-C₄-alkyl-or C₁-C₄- dialkylamino or hydroxyl, or
R¹ denotes phenyl, naphthyl, thienyl, furyl, pyrrolyl or pyridyl, these aryl or heteroaryl radicals mentioned being in turn optionally monosubstituted by carboxyl, amino, hydroxyl or C₁-C4-alkyl- or C₁-C₄-dialkylamino, or
R¹ is a substituent of the formula -OR³, or -N(R³)₂, in which R³ is hydrogen or C₁-C₉-alkyl, C₁-C^{g-}alkenyl, C₁-C₉-alkynyl or C₁-C₉-alkylcarbonyl, these radicals being optionally mono-or disubstituted by
halogen, hydroxyl, nitro, cyano, amino, C₁-C₄-alkoxy, carboxyl, C₁-C₄-alkoxycarbonyl, C₁-C₄- alkylcarbonyloxy or C₁-C₄-alkyl- or C₁-C₄-dialkylamino, or optionally substituted by
phenyl, naphthyl, thienyl, furyl, pyrrolyl or pyridyl, these aryl or heteroaryl radicals mentioned being in turn optionally monosubstituted by halogen, carboxyl, amino, Ci-C4-alkyl-or C₁-C4-dialkylamino or hydroxyl,
and it also being possible for the two substituents R³ in -N(R³)₂ to differ independently of one another, and
R² denotes a substituent of the formula -OR⁴ or R⁴⁻N-R⁵, in which
R⁴ denotes hydrogen or C₁-C₁₂-alkyl, which is optionally mono- or disubstituted by halogen, hydroxyl, cyano, carboxyl, C₁-C₄-alkoxy, C₁-C₄-alkoxycarbonyl, C₁-C₄-alkylcarbonyloxy, or C₁-C₄-alkyl- or C₁-C₄-dialkylamino, or is optionally substituted by phenyl, which is in turn optionally mono-, di- or trisubstituted by halogen, C₁-C₄-alkyl or C₁-C₄-alkoxy, it also being possible for the substituents to differ independently of one another in the case of polysubstitution, or
R⁴ denotes cyclohexyl, which is optionally benzofused, or
R⁴ denotes phenyl, naphthyl, thienyl, furyl, pyrrolyl or pyridyl, the phenyl, naphthyl and pyridyl radicals being optionally mono-, di- or trisubstituted and the thienyl, furyl and pyrrolyl radicals being optionally monosubstituted by halogen, C1-C4-alkyl or C1-C4-alkoxy, it also being possible for the substituents to differ independently of one another in the case of polysubstitution, and
R⁵ denotes hydrogen or C₁-C₃-alkyl, R⁵ in the case of C₁-C₃-alkyl radicals together with R⁴, which in this case denotes C₃-C₅-alkyl, optionally forming a heterocyclic saturated 6- membered ring, it also being possible for the heterocyclic 6-membered ring to contain a second nitrogen atom and in turn to be substituted by phenyl or phenyl-C₁-C₃-alkyl,
and it also being possible for the two radicals R² bonded to the pyridine skeleton via the carbonyl group in the 2- and 4-position to differ independently of one another, and it also being possible for all the alkyl radicals mentioned with more than 2 carbon atoms to be branched, or a physiologically tolerated salt, excluding pyridine-2,4,5-tricarboxylic acid and its trimethyl ester, 5-ethylpyridine-2,4-dicarboxylic acid, the compounds in which R¹ is an aminomethyl radical, dimethyl 5-methylpyridine-2,4-dicarboxylate, dimethyl 5-methoxypyridine-2,4-dicarboxylate, dimethyl 5-ethoxycarbonylpyridine-2,4-dicarboxylate and dimethyl 4-ethoxycarbonylpyridine-2,5-dicarboxylate, which comprises either converting a 2,4-dimethyl-5-halopyridine first into 2,4,5-trimethylpyridine and oxidizing this to pyridine-2,4,5-tricarboxylic acid, which is converted into the trimethoxycarbonyl compound and then reacted to give tripotassium pyridine-2,4,5-tricarboxylate, or oxidizing a 2,4-dimethyl-5-halopyridine to give a compound of the formula (1,1) 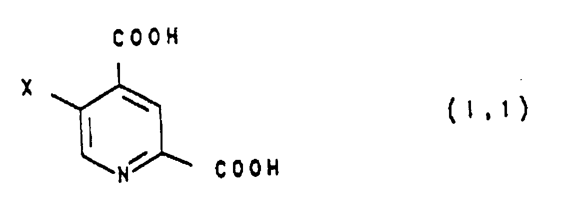 in which X denotes halogen, and subsequently, if appropriate,
a) converting the compound of the formula (1,1) into a compound of the formula (1,2) 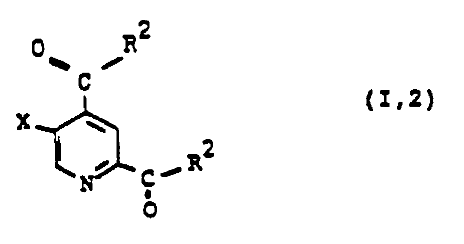 in which X is halogen and R² has the meanings given in the case of formula I as claimed in claim 1, and, if appropriate, subsequently reacting the compound of the formula (1,2) with a compound of the formula II or II' 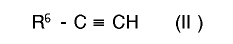 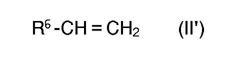 in which R⁶ denotes C₁ -C₇-alkyl, which is optionally mono- or disubstituted by
halogen, hydroxyl, nitro, cyano, amino, C₁-C₄-alkoxy, carboxyl, C₁-C₄-alkoxycarbonyl or C₁-C₄-alkyl-or C₁-C₄-dialkylamino, or is optionally substituted by
phenyl, naphthyl, thienyl, furyl, pyrrolyl or pyridyl, these aryl or heteroaryl radicals mentioned being in turn optionally monosubstituted by halogen, carboxyl, amino, C₁-C₄-alkyl- or C1-C4-dialkylamino or hydroxyl,
and in which any free carboxyl groups present are optionally protected,
and, if appropriate, hydrogenating the remaining C-C triple or C-C double bond in the fragment which is now bonded to the pyridine skeleton and is derived from the compound of the formula II or 11', or if appropriate reacting the compound of the formula (1,2) with a compound of the formula H₂-N-R³, in which R³ has the meanings given in the case of formula I in claim 1 and in which any free carboxyl groups present are optionally protected,
or, if appropriate, converting the compound of the formula (1,2) into a compound of the formula (1,3) 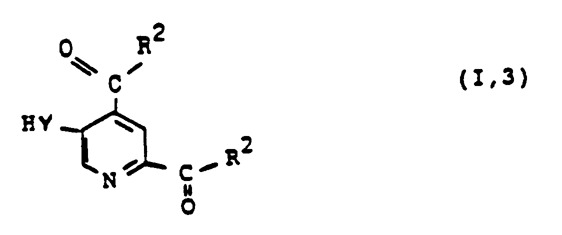 in which Y stands for O or NH and R² has the meanings given in the case of formula I in claim 1, in a manner which is known per se,
and then, if appropriate, reacting the product with a compound of the formula III or III' 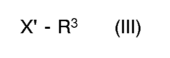 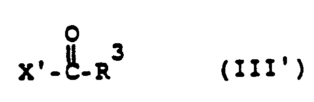 in which X' denotes chlorine, bromine or iodine and R³ has the meanings given in the case of formula I in claim 1, and in which any free carboxyl groups present are optionally protected, or, if appropriate, reacting the compound of the formula (1,2) with a compound of the formula IV 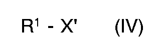 in which X' is chlorine, bromine or iodine and R¹ has the meanings given in the case formula I in claim 1, excluding the meanings of -OR³ and -N(R³)₂, in which R³ has the meanings given in the case of formula I in claim 1, and in which any free carboxyl groups present are optionally protected, or, if appropriate, reacting the compound of the formula (1,2) with a compound of the formula V 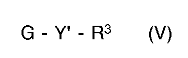 in which
Y' stands for O or NR³,
G stands for an alkali metal and
R³ has the meanings given in the case of formula I in claim 1, and in which any free carboxyl groups present are optionally protected, or, if appropriate,
b) reacting the compound of the formula (1,1) with a compound of the formula II or 11', in which any free carboxyl groups present are optionally protected, and if appropriate then esterifying the carboxylic acids present in the 2- and 4-position of the pyridine skeleton or converting them into the diamides or ester/amides, and if appropriate hydrogenating the remaining C-C triple bond or C-C double bond in the fragment which is now bonded to the pyridine skeleton and is derived from the compound of the formula II or 11', or if appropriate reacting the compound of the formula (1,1) with a compound of the formula H₂ N-R³, in which R³ has the meanings given in the case of formula I in claim 1 and in which any free carboxyl groups present are optionally protected, or
if appropriate converting the compound of the formula (1,1) into a compound of the formula (1,4) 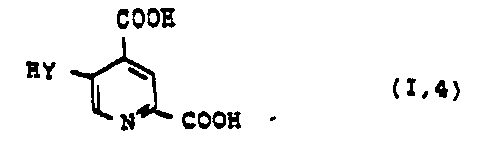 in which Y stands for O or NH,
and if appropriate then esterifying the carboxylic acids present in the 2- and 4-position of the pyridine skeleton, or converting them into the diamides or ester/amides,
and, if appropriate, subsequently reacting the product with a compound of the formula III or III', in which any free carboxyl groups present are optionally protected, or
if appropriate reacting the compound of the formula (1,1) with a compound of the formula IV, in which any free carboxyl groups present are optionally protected, the compounds of the formula R³0-1 and (R³)₂ N-I, in which R³ has the meanings given in the case of formula I in claim 1, being excluded, or
if appropriate reacting the compound of the formula (1,1) with a compound of the formula V, in which any free carboxyl groups present are optionally protected, and if appropriate then esterifying the carboxylic acids optionally present in the 2- and 4-position of the pyridine skeleton in the products obtained according to b), or converting them into the diamides or ester/amides, or, if appropriate,
c) first protecting the carboxyl groups present in the 2- and 4-position of the pyridine skeleton in the compound of the formula (1,1) with a protective group, to give a compound of the formula (1,10) 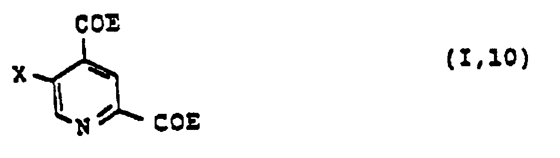 in which E denotes a protective group, and, if appropriate, subsequently
reacting the compound of the formula (1,10) with a compound of the formula II or 11', in which any free carboxyl groups present are optionally protected, and if appropriate then splitting off the protective groups E of the carboxyl groups in the 2- and 4-position of the pyridine skeleton either selectively or together, and if appropriate esterifying the resulting free carboxylic acids or converting them into the diamides or ester/amides, and if appropriate hydrogenating the remaining C-C triple bond or C-C double bond in the fragment which is now bonded to the pyridine skeleton and is derived from the compound of the formula II or 11',
or if appropriate reacting the compound of the formula (1,10) with a compound of the formula H₂N-R³, in which R³ has the meanings given in the case of formula I in claim 1 and in which any free carboxyl groups present are optionally protected, or
if appropriate converting the compound of the formula (1,10) into a compound of the formula (1,11) 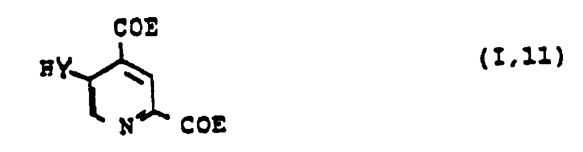 in which Y stands for O or NH,
and if appropriate then splitting off the protective groups E of the carboxyl groups in the 2- and 4- position of the pyridine skeleton either selectively or together, and if appropriate esterifying the resulting free carboxylic acid or converting it into the diamide or ester/amide,
and if appropriate subsequently reacting the product with a compound of the formula III or III', in which any free carboxyl groups present are optionally protected, or
if appropriate reacting the compound of the formula (1,10) with a compound of the formula IV, in which any free carboxyl groups present are optionally protected, the compounds of the formula R³0-I and (R³)₂ N-I in which R³ has the meanings given in the case of formula I in claim 1 being excluded, or
if appropriate reacting the compound of the formula (1,10) with a compound of the formula V, in which any free carboxyl groups present are optionally protected, and, if appropriate, subsequently splitting off any protective groups E of the carboxyl groups in the 2- and 4-position of the pyridine skeleton, either selectively or together, in a product obtained according to c), and
if appropriate esterifying the resulting free carboxylic acid or converting it into the diamide or ester/amide,
and if appropriate subsequently splitting off the protective groups present in the product hydrolytically or hydrogenolytically, and if appropriate converting the compound obtained according to a), b) or c) into one of its physiologically tolerated salts.

2. The process as claimed in claim 1, which comprises preparing a substituted pyridine-2,4-dicarboxylic acid derivative of the formula I, in which:
R¹ denotes halogen or carboxyl, or
R¹ denotes C₁-C₄-alkyl or C₂-C₄-alkenyl or -alkynyl, the radicals mentioned being optionally interrupted by a carbonyl group and the radicals mentioned being in turn optionally monosubstituted by halogen, hydroxyl, nitro, cyano, amino or carboxyl, or
R¹ denotes phenyl, thienyl, furyl or pyrrolyl, the aryl or heteroaryl radicals mentioned being in turn optionally monosubstituted by carboxyl, or
R¹ is a substituent of the formula -OR³ or -N(R³)₂, in which R³ is hydrogen, C₁-C₃-alkylcarbonyl or C₁-C₃-alkyl, these radicals being in turn optionally substituted by carboxyl, or
R³ denotes phenyl, which is in turn optionally para-substituted by halogen, and it also being possible for the two substituents R³ in -N(R³)₂ to differ independently of one another, and
R² denotes a substituent of the formula -OR⁴ or R4-N-R5, in which R⁴ denotes hydrogen or C₁-C₁₂-alkyl, which is optionally mono- or disubstituted by halogen, hydroxyl, cyano, carboxyl, C1-C4-alkoxy, C₁-C₄-alkoxycarbonyl, C₁-C₄-alkylcarbonyloxy or C₁-C₄-alkyl- or C₁-C₄-dial- kylamino, or is optionally substituted by phenyl, which is in turn optionally mono-, di- or trisubstituted by halogen, C₁-C₄-alkyl or C1-C4-alkoxy, it also being possible for the substituents to differ independently of one another in the case of polysubstitution, or
R⁴ denotes cyclohexyl, which is optionally benzofused, or
R⁴ denotes phenyl, naphthyl, thienyl, furyl, pyrrolyl or pyridyl, the phenyl, naphthyl and pyridyl radicals being optionally mono-, di- or trisubstituted and the thienyl, furyl and pyrrolyl radicals being optionally monosubstituted by halogen, C1-C4-alkyl or C₁-C₄-alkoxy, it also being possible for the substituents to differ independently of one another in the case of polysubstitution, and
R⁵ denotes hydrogen or C₁-C₃-alkyl, R⁵ in the case of the C₁-C₃-alkyl radicals together with R⁴, which in this case denotes C₃-C₅-alkyl, optionally forming a heterocyclic saturated 6- membered ring, it also being possible for the heterocyclic 6-membered ring to contain a second nitrogen atom and to be in turn substituted by phenyl or phenyl-C₁-C₃-alkyl,
and it also being possible for the two radicals R² bonded to the pyridine skeleton via the carbonyl group in the 2- and 4-position to differ independently of one another, and it also being possible for all the alkyl radicals mentioned with more than 2 carbon atoms to be branched, or a physiologically tolerated salt, excluding pyridine-2,4,5-tricarboxylic acid and its trimethyl ester, 5-ethyl-pyridine-2,4-dicarboxylic acid, the compounds in which R¹ is an aminomethyl radical, dimethyl 5-methylpyridine-2,4-dicarboxylate, dimethyl 5-methoxypyridine-2,4-dicarboxylate, dimethyl 5-ethoxycarbonylpyridine-2,4-dicarboxylate and dimethyl 4-ethoxycarbonylpyridine-2,5-dicarboxylate.

3. The process as claimed in claim 1, which comprises preparing a substituted pyridine-2,4-dicarboxylic acid derivative of the formula I' 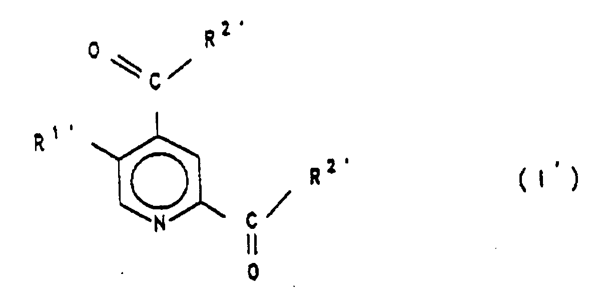 in which the substituents R^{1'} and R^{2'} have the same meaning as R¹ and R² in formula I as claimed in claim 1, but including pyridine-2,4,5-tricarboxylic acid and its trimethyl ester, 5-ethyl-pyridine-2,4-dicarboxylic acid, the compounds in which R^{1'} is an aminomethyl radical, dimethyl 5-methylpyridine-2,4-dicarboxylate, dimethyl 5-methoxypyridine-2,4-dicarboxylate, dimethyl 5-ethoxycarbonylpyridine-2,4-dicarboxylate and dimethyl 4-ethoxycarbonylpyridine-2,5-dicarboxylate, for use as a medicament.

4. The process as claimed in claim 3, which comprises preparing a substituted pyridine-2,4-dicarboxylic acid derivative of the formula I', in which R^{1'} and R²' have the same meaning as R¹ and R² in formula I as claimed in claim 2, for use as a medicament.

5. The use of a compound prepared as claimed in one or more of claims 1 to 4 for inhibiting proline hydroxylase and lysine hydroxylase.

6. The use of a compound prepared as claimed in one or more of claims 1 to 4 as a fibrosuppressant and immunosuppressant.

7. A medicament containing a compound of the formula I or I' with tolerated pharmaceutical excipients.

8. The use of a compound of the formula I or I' for influencing the metabolism of collagen and collagenlike substances and/or the biosynthesis of C1 q.

9. The use of a compound of the formula I or I' for the treatment of disturbances in the metabolism of collagen and collagen-like substances and/or the biosynthesis of C1 q.

10. A process for the preparation of medicaments for influencing the metabolism of collagen and collagen- like substances and/or the biosynthesis of C1 q, which comprises incorporating a compound of the formula I or I' into the medicament.

## Revendications (Revendications pour l'(les) Etat(s) contractant(s) suivant(s) : suivants : AT, BE, CH, DE, FR, GB, IT, LI, LU, NL, SE)

1. Dérivés de substitution de l'acide pyridine-2,4-dicarboxylique, de formule I, 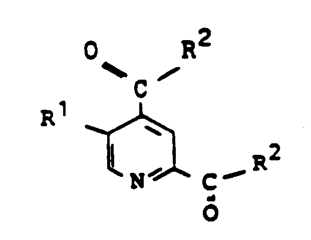 dans laquelle les symboles suivants représentent respectivement :
R¹ un atome d'halogène, un groupe carboxyle ou alcoxY(C₁₋₄)carbonyle, ou bien
R¹ un groupe alkyle, alcényle ou alcynyle, pouvant comporter jusqu'à 9 atomes de carbone et pouvant être éventuellement interrompu par un groupe carbonyle et éventuellement mono- ou disubstitué par des groupes halogéno, hydroxy, nitro, cyano, amino, alcoxy en C₁₋₄, carboxyle, alcoxy(C₁₋₄)-carbonyle, alkyl(C₁₋₄)carbonyloxy, alkyl(C₁₋₄)amino ou dialkyl(C₁₋₄)amino, ou pouvant être éventuellement substitué par des groupes phényle, naphtyle, thiényle, furyle, pyrrolyle ou pyridyle, ces groupes aryle ou hétéroaryle étant quant à eux éventuellement monosubstitués par un groupe halogéno, carboxyle, amino, alkyl(C₁₋₄)amino ou dialkyl(C₁₋₄)amino, ou hydroxy, ou bien
R¹ un groupe phényle, naphtyle, thiényle, furyle, pyrrolyle ou pyridyle, ces groupes aryle ou hétéroaryle étant quant à eux éventuellement monosubstitués par un groupe carboxyle, amino, alkyl-(C₁₋₄)amino ou dialkyl(C₁₋₄)amino, ou hydroxy, ou bien
R¹ un substituant de formule -OR³ ou -N(R³)₂, où R³ est un atome d'hydrogène ou un groupe alkyle en Ci-a, alcényle en Ci-a, alcynyle en C₁₋₉ ou alkyl(C₁ -9)carbonyle, ces groupes pouvant être mono- ou disubstitués par des groupes halogéno, hydroxy, nitro, cyano, amino, alcoxy en C₁₋₄, carboxyle, alcoxy(C₁₋₄)carbonyle, alkyl(C₁₋₄)carbonyloxy, alkyl(C₁₋₄)amino ou dialkyl(C₁₋₄)amino, ou pouvant être substitués par des groupes phényle, naphtyle, thiényle, furyle, pyrrolyle ou pyridyle, ces groupes aryle ou hétéroaryle étant quant à eux éventuellement monosubstitués par un groupe halogéno, carboxyle, amino, alkyl(C1-4)amino ou dialkyl(C₁₋₄)amino, ou hydroxy, et où les deux substituants R³ dans -N(R³)₂ peuvent aussi être différents indépendamment l'un de l'autre, et
R² un substituant de formule -OR⁴ ou R⁴⁻N-R⁵, où R⁴ est hydrogène ou alkyle en C₁₋₁₂, éventuellement substitué 1 ou 2 fois par des groupes halogéno, hydroxy, cyano, carboxyle, alcoxy en C₁₋₄, alcoxY(C₁₋₄)carbonyle, alkyl(C₁₋₄)carbonyloxy, alkyl(C₁₋₄)amino ou dialkyl(C₁₋₄)amino, ou pouvant être substitués par un groupe phényle éventuellement lui-même substitué 1, 2 ou 3 fois par un atome d'halogène, un groupe alkyle en C₁₋₄ ou alcoxy en C₁₋₄, les substituants pouvant, dans le cas de substitutions multiples, être différents indépendamment les uns des autres, ou bien R⁴ représente un groupe cyclohexyle, qui est éventuellement condensé à un noyau benzène, ou R⁴ représente un groupe phényle, naphtyle, thiényle, furyle, pyrrolyle ou pyridyle, les groupes phényle, naphtyle et pyridyle pouvant être substitués 1, 2 ou 3 fois, et les groupes thiényle, furyle, pyrrolyle pouvant être substitués 1 fois, par halogène, alkyle en C₁₋₄ ou alcoxy en C₁₋₄, les substituants pouvant ici aussi, dans le cas de substitutions multiples, être différents indépendamment les uns des autres, et R⁵ représente l'atome d'hydrogène ou un groupe alkyle en C₁₋₃, R⁵ pouvant, dans le cas d'un groupe alkyle en C₁₋₃, former avec R⁴, qui, en ce cas, désigne un groupe alkyle en C₃₋₅, un cycle hétérocyclique saturé à 6 chaînons, le cycle hétérocyclique saturé à 6 chaînons pouvant renfermer en outre un deuxième atome d'azote et être substitué à son tour par des groupes phényle ou phénylalkyle(C₁₋₃), et
où les deux groupes R², liés au squelette pyridine en positions 2 et 4 par les groupes carbonyle, peuvent aussi être différents indépendamment l'un de l'autre, et où tous les groupes alkyle nommés, comportant plus de 2 atomes de carbone, peuvent également être ramifiés,
ainsi que leurs sels physiologiquement acceptables, à l'exception de l'acide pyridine-2,4,5-tricarboxyli- que et ses esters triméthylés, de l'acide 5-éthylpyridine-2,4-dicarboxylique, des composés dans lesquels R¹ est un groupe aminométhyle, du 5-méthylpyridine-2,4-dicarboxylate de diméthyle, du 5-méthoxypyridine-2,4-dicarboxylate de diméthyle, du 5-éthoxycarbonylpyridine-2,4-dicarboxylate de diméthyle, du 4-méthoxycarbonylpyridine-2,5-dicarboxylate de diméthyle.

2. Dérivés de substitution de l'acide pyridine-2,4-dicarboxylique de formule I, selon la revendication 1, dans laquelle les symboles suivants représentent respectivement:
R¹ un atome d'halogène ou un groupe carboxyle, ou bien
R¹ un groupe alkyle en C₁₋₄, alcényle en C2 -4 ou alcynyle en C₂-₄, pouvant être éventuellement interrompu par un groupe carbonyle, et les groupes mentionnés étant éventuellement, quant à eux, monosubstitués par un groupe halogéno, hydroxy, nitro, cyano, amino ou carboxyle, ou bien
R¹ un groupe phényle, thiényle, furyle ou pyrrolyle, ces groupes aryle ou hétéroaryle étant quant à eux éventuellement monosubstitués par un groupe carboxyle, ou bien
R¹ un substituant de formule -OR³ ou -N(R³)₂, où R³ est un atome d'hydrogène ou un groupe alkyle en C₁₋₃ ou alkyl(C_{1 -3})carbonyle, ces groupes pouvant être à leur tour substitués par des groupes carboxyle, ou bien R³ représente un groupe phényle, pouvant être substitué quant à lui par un atome d'halogène, en position para, et les deux substituants R³ dans -N(R³)₂ peuvent aussi être différents indépendamment l'un de l'autre, et
R² un substituant de formule -OR⁴ ou R⁴⁻N-R⁵, où R⁴ est hydrogène ou alkyle en C₁₋₁₂, éventuellement substitué 1 ou 2 fois par des groupes halogéno, hydroxy, cyano, carboxyle, alcoxy en C₁₋₄, alcoxy(C₁₋₄)carbonyle, alkyl(C₁₋₄)carbonyloxy, alkyl(C₁₋₄)amino ou dialkyl(C₁₋₄)amino, ou pouvant être substitués par des groupes phényle éventuellement eux-mêmes substitués 1, 2 ou 3 fois par un atome d'halogène, un groupe alkyle en C₁₋₄ ou alcoxy en C₁₋₄, les substituants pouvant, dans le cas de substitutions multiples, être différents indépendamment les uns des autres, ou bien R⁴ représente un groupe cyclohexyle, qui est éventuellement condensé à un noyau benzène, ou R⁴ représente un groupe phényle, naphtyle, thiényle, furyle, pyrrolyle ou pyridyle, les groupes phényle, naphtyle et pyridyle pouvant être substitues 1, 2 ou 3 fois, et les groupes thiényle, furyle, pyrrolyle pouvant être substitués 1 fois, par halogène, alkyle en C₁₋₄ ou alcoxy en C₁₋₄, les substituants pouvant ici aussi, dans le cas de substitutions multiples, être différents indépendamment les uns des autres, et R⁵ représente l'atome d'hydrogène ou un groupe alkyle en C₁₋₃, R⁵ pouvant, dans le cas d'un groupe alkyle en C₁₋₃, former avec R⁴, qui, en ce cas, désigne un groupe alkyle en C₃ -₅, un cycle hétérocyclique saturé à 6 chaînons, le cycle hétérocyclique saturé à 6 chaînons pouvant renfermer en outre un deuxième atome d'azote et être substitué à son tour par des groupes phényle ou phénylalkyle(C₁₋₃), et
où les deux groupes R², liés au squelette pyridine en positions 2 et 4 par les groupes carbonyle, peuvent aussi être différents indépendamment l'un de l'autre, et où tous les groupes alkyle nommés, comportant plus de 2 atomes de carbone, peuvent également être ramifiés,
ainsi que leurs sels physiologiquement acceptables, à l'exception de l'acide pyridine-2,4,5-tricarboxyli- que et ses esters triméthylés, de l'acide 5-éthylpyridine-2,4-dicarboxylique, des composés dans lesquels R¹ est un groupe aminométhyle, du 5-méthylpyridine-2,4-dicarboxylate de diméthyle, du 5-méthoxypyridine-2,4-dicarboxylate de diméthyle, du 5-éthoxycarbonylpyridine-2,4-dicarboxylate de diméthyle, du 4-méthoxycarbonylpyridine-2,5-dicarboxylate de diméthyle.

3. Dérivés de substitution d'acide pyridine-2,4-dicarboxylique de formule l', 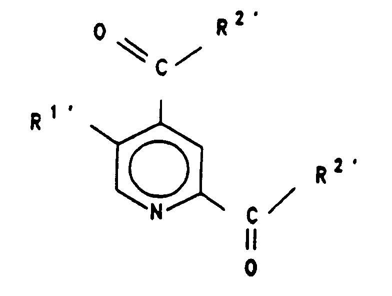 dans laquelle les substituants R^{1'} et R^{2'} ont les mêmes définitions que R¹ et R² de la formule 1 selon la revendication 1, mais sans exclusion de l'acide pyridine-2,4,5-tricarboxylique et de ses esters triméthylés, de l'acide 5-éthylpyridine-2,4-dicarboxylique, des composés dans lesquels R^{1'} est un groupe aminométhyle, du 5-méthylpyridine-2,4-dicarboxylate de diméthyle, du 5-méthoxypyridine-2,4-dicar- boxylate de diméthyle, du 5-éthoxycarbonylpyridine-2,4-dicarboxylate de diméthyle, du 4-méthoxycar- bonylpyridine-2,5-dicarboxylate de diméthyle, pour leur utilisation comme médicaments.

4. Dérivés de substitution d'acide pyridine-2,4-dicarboxylique de formule l' selon la revendication 3, dans laquelle R^{1'} et R^{2'} ont les mêmes définitions que R¹ et R² de la formule 1 selon la revendication 2, pour leur utilisation comme médicaments.

5. Procédé de préparation des composés de formule I selon la revendication 1, caractérisé en ce que soit on transforme d'abord la 5-halogéno-2,4-diméthylpyridine en 2,4,5-triméthylpyridine, que l'on oxyde ensuite en acide pyridine-2,4,5-tricarboxylique, qui est transformé en composé triméthoxycarbonylé et ensuite en pyridine-2,4,5-tricarboxylate tripotassique, soit on oxyde la 5-halogéno-2,4-diméthylpyridine en un composé de formule suivante (1,1), 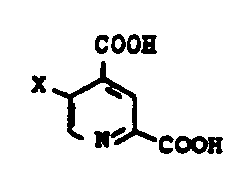 dans laquelle X représente un atome d'halogène, puis on transforme éventuellement
a) le composé de formule (1,1) en un composé de formule (1,2) suivante, 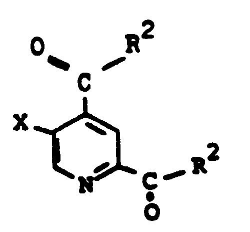 dans laquelle X est halogène et R² a les définitions indiquées pour la formule I selon la revendication 1, et on fait ensuite réagir éventuellement le composé de formule (1,2) avec un composé de formule Il ou Il' 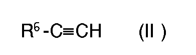 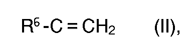 où R⁶ représente un groupe alkyle en C₁₋₇, éventuellement mono-ou disubstitué par un atome d'halogène ou un groupe hydroxy, nitro, cyano, amino, alcoxy en C₁₋₄, carboxyle,
alcoxy(C₁₋₄)carbonyle, alkyl(C₁₋₄)amino ou dialkyl(C₁₋₄)amino, ou éventuellement substitué par un groupe phényle, naphtyle, thiényle, furyle, pyrrolyle ou pyridyle, ces groupes aryle ou hétéroaryle étant quant à eux éventuellement monosubstitués par un groupe halogéno, carboxyle, amino, alkyl-(C₁₋₄)amino ou dialkyl(C₁₋₄)amino, ou hydroxy,
les groupes carboxyle libres éventuellement présents étant éventuellement protégés,
et on hydrogène éventuellement la double ou triple liaison carbone-carbone restant dans le fragment désormais lié au squelette pyridine, qui dérive du composé de formule Il ou II', ou bien on fait réagir éventuellement le composé de formule (1,2) avec un composé de formule H₂N-R³, dans laquelle R³ a les définitions indiquées dans la revendication 1 pour la formule I et dans laquelle les groupes carboxyle libres éventuellement présents sont éventuellement protégés, ou on transforme éventuellement le composé de formule (1,2) de manière connue en soi en un composé de formule (1,3) suivante, 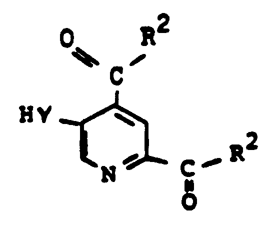 dans laquelle Y représente O ou NH et R² a les définitions indiquées dans la revendication 1 pour la formule I, et on le fait ensuite réagir éventuellement avec un composé de formule III ou III' 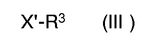 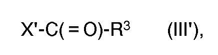 où X' représente chlore, brome ou iode et R³ a les définitions indiquées dans la revendication 1 pour la formule I, et où les groupes carboxyle libres éventuellement présents sont éventuellement protégés, ou
on fait réagir éventuellement le composé de formule (1,2) avec un composé de formule IV, 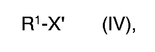 où X' est chlore, brome ou iode et R¹ a les définitions indiquées dans la revendication 1 pour la formule I, les définitions -OR³ et -N(R³)₂, où R³ a les mêmes significations que pour la formule I de la revendication 1, étant exclues, et où les groupes carboxyle libres éventuellement présents sont éventuellement protégés, ou bien
on fait éventuellement réagir le composé de formule (1,2) avec un composé de formule V 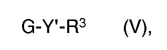 dans laquelle
Y' représente O ou NR³,
G représente un métal alcalin,
R³ a les définitions indiquées dans la revendication 1 pour la formule I, et dans laquelle les groupes carboxyle libres éventuellement présents sont éventuellement protégés, ou bien on fait éventuellement réagir
b) le composé de formule (1,1) avec un composé de formule II ou II' où les groupes carboxyle libres éventuellement présents sont éventuellement protégés,
puis on estérifie éventuellement les acides carboxyliques présents en positions 2 et 4 du squelette pyridine ou on les transforme en diamides ou esteramides et on hydrogène éventuellement la double ou triple liaison carbone-carbone restant dans le fragment désormais lié au squelette pyridine, qui dérive du composé de formule II ou II', ou bien
on fait réagir éventuellement le composé de formule (1,1) avec un composé de formule H2N-R3, dans laquelle R³ a les définitions indiquées dans la revendication 1 pour la formule I et dans laquelle les groupes carboxyle libres éventuellement présents sont éventuellement protégés, ou bien
on transforme éventuellement le composé de formule (1,1) en un composé de formule (1,4) suivante, 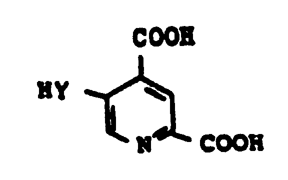 dans laquelle Y représente O ou NH, puis on estérifie éventuellement les acides carboxyliques présents en positions 2 et 4 du squelette pyridine ou on les transforme en diamides ou esteramides, et
on fait ensuite éventuellement réagir le produit avec un composé de formule III ou III' où les groupes carboxyle libres éventuellement présents sont éventuellement protégés, ou bien on fait réagir éventuellement le composé de formule (1,1) avec un composé de formule IV dans laquelle les groupes carboxyle libres éventuellement présents sont éventuellement protégés, les composés de formules R³0-1 et (R³)₂N-I, où R³ a les définitions indiquées dans la revendication 1 pour la formule I, étant exclus, ou bien
on fait réagir éventuellement le composé de formule (1,1) avec un composé de formule V dans laquelle les groupes carboxyle libres éventuellement présents sont éventuellement protégés, puis on estérifie éventuellement les acides carboxyliques éventuellement présents en positions 2 et 4 du squelette pyridine dans les produits obtenus selon b) ou on les transforme en diamides ou esteramides,
ou bien, éventuellement,
c) on protège tout d'abord, à l'aide d'un groupe protecteur, les groupes acide carboxyliques présents en positions 2 et 4 du squelette pyridine dans le composé de formule (1,1), ce qui permet d'obtenir un composé de formule (1,10) suivante, 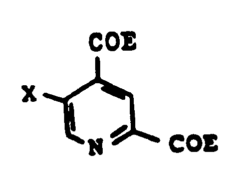 dans laquelle E représente un groupe protecteur,
et, éventuellement, on fait ensuite réagir le composé de formule (1,10) avec un composé de formule Il ou II' dans lequel des groupes carboxyle libres sont éventuellement protégés, puis on chasse éventuellement les groupes protecteurs E des groupes carboxyle en positions 2 et 4 du squelette pyridine, soit sélectivement, soit ensemble, et on estérifie éventuellement les groupes carboxyle libres résultants ou on les transforme en diamides ou esteramides, et on hydrogène éventuellement la double ou triple liaison carbone-carbone restant dans le fragment désormais lié au squelette pyridine, qui dérive du composé de formule Il ou II', ou bien
on fait réagir éventuellement le composé de formule (1,10) avec un composé de formule H₂N-R³, dans laquelle R³ a les définitions indiquées dans la revendication 1 pour la formule I et dans laquelle les groupes carboxyle libres éventuellement présents sont éventuellement protégés, ou bien
on transforme éventuellement le composé de formule (1,10) en un composé de formule (1,11) suivante, 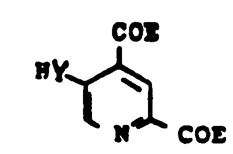 dans laquelle Y représente O ou NH, et on chasse ensuite éventuellement les groupes protecteurs E des groupes carboxyle en positions 2 et 4 du squelette pyridine, soit sélectivement, soit ensemble, et on estérifie éventuellement les groupes carboxyle libres résultants ou on les transforme en diamides ou esteramides, et
on fait ensuite éventuellement réagir le produit avec un composé de formule III ou III' où les groupes carboxyle libres éventuellement présents sont éventuellement protégés, ou bien on fait réagir éventuellement le composé de formule (1,10) avec un composé de formule IV dans laquelle les groupes carboxyle libres éventuellement présents sont éventuellement protégés, les composés de formules R³0-1 et (R³)₂N-I, où R³ a les définitions indiquées dans la revendication 1 pour la formule I, étant exclus, ou bien
on fait réagir éventuellement le composé de formule (1,10) avec un composé de formule V dans laquelle les groupes carboxyle libres éventuellement présents sont éventuellement protégés, puis on chasse éventuellement, sélectivement ou ensemble, les groupes protecteurs E des acides carboxyliques éventuellement présents en positions 2 et 4 du squelette pyridine dans les produits obtenus selon c),
puis on estérifie éventuellement les acides carboxyliques libres obtenus ou on les transforme en diamides ou esteramides, puis on chasse éventuellement, par hydrolyse ou par hydrogénolyse, les groupes protecteurs présents dans les produits et on transforme éventuellement les composés obtenus selon a), b) ou c) en leurs sels physiologiquement acceptables.

6. Composés selon une quelconque des revendications 1 à 4 comme inhibiteurs de la proline- ou lysine hydroxylase.

7. Composés selon une quelconque des revendications 1 à 4 comme fibrosuppresseurs et immunosuppresseurs.

8. Médicaments contenant un composé de formule I ou l' ainsi que des véhicules pharmaceutiquement acceptables.

9. Utilisation de composés de formule I ou l' pour influencer le métabolisme du collagène et des substances analogues au collagène ou la biosynthèse de C1 q.

10. Utilisation de composés de formule I ou l' pour traiter les troubles du métabolisme du collagène et des substances analogues au collagène ou de la biosynthèse de C1 q.

11. Procédé de fabrication de médicaments pour influencer le métabolisme du collagène et des substances analogues au collagène ou la biosynthèse de C1 q, caractérisé en ce qu'on incorpore aux médicaments un composé de formule I ou l'.

## Revendications (Revendications pour l'(les) Etat(s) contractant(s) suivant(s) : suivants : ES, GR)

1. Procédé de préparation de dérivés de substitution de I,acide pyridine-2,4-dicarboxylique, de formule I, 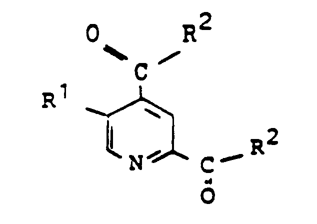 dans laquelle les symboles suivants représentent respectivement:
R¹ un atome d'halogène, un groupe carboxyle ou alcoxy(C₁₋₄)carbonyle, ou bien
R¹ un groupe alkyle, alcényle ou alcynyle, pouvant comporter jusqu'à 9 atomes de carbone et pouvant être éventuellement interrompu par un groupe carbonyle et éventuellement mono- ou disubstitué par des groupes halogéno, hydroxy, nitro, cyano, amino, alcoxy en C₁₋₄, carboxyle, alcoxy(C₁₋₄)-carbonyle, alkyl(C₁₋₄)carbonyloxy, alkyl(C₁₋₄)amino ou dialkyl(C₁₋₄)amino, ou pouvant être éventuellement substitué par des groupes phényle, naphtyle, thiényle, furyle, pyrrolyle ou pyridyle, ces groupes aryle ou hétéroaryle étant quant à eux éventuellement monosubstitués par un groupe halogéno, carboxyle, amino, alkyl(C₁₋₄)amino ou dialkyl(C₁₋₄)amino, ou hydroxy, ou bien
R¹ un groupe phényle, naphtyle, thiényle, furyle, pyrrolyle ou pyridyle, ces groupes aryle ou hétéroaryle étant quant à eux éventuellement monosubstitués par un groupe carboxyle, amino, alkyl-(C₁₋₄)amino ou dialkyl(C₁₋₄)amino, ou hydroxy, ou bien
R¹ un substituant de formule -OR³ ou -N(R³)₂, où R³ est un atome d'hydrogène ou un groupe alkyle en Ci-a, alcényle en Ci-a, alcynyle en C₁₋₉ ou alkyl(C₁ -9)carbonyle, ces groupes pouvant être mono- ou disubstitués par des groupes halogéno, hydroxy, nitro, cyano, amino, alcoxy en C₁₋₄, carboxyle, alcoxy(C₁₋₄)carbonyle, alkyl(C₁₋₄)carbonyloxy, alkyl(C₁₋₄)amino ou dialkyl(C₁₋₄)amino, ou pouvant être substitués par des groupes phényle, naphtyle, thiényle, furyle, pyrrolyle ou pyridyle, ces groupes aryle eu hétéroaryle étant quant à eux éventuellement monosubstitués par un groupe halogéno, carboxyle, amino, alkyl(C1-4)amino ou dialkyl(C₁₋₄)amino, ou hydroxy, et où les deux substituants R³ dans -N(R³)₂ peuvent aussi être différents indépendamment l'un de l'autre, et
R² un substituant de formule -OR⁴ ou R⁴⁻N-R⁵, où R⁴ est hydrogène ou alkyle en C₁₋₁₂, éventuellement substitué 1 ou 2 fois par des groupes halogéno, hydroxy, cyano, carboxyle, alcoxy en C₁₋₄, alcoxy(C₁₋₄)carbonyle, alkyl(C₁₋₄)carbonyloxy, alkyl(C₁₋₄)amino ou dialkyl(C₁₋₄)amino, ou pouvant être substitués par un groupe phényle éventuellement lui-même substitué 1, 2 ou 3 fois par un atome d'halogène, un groupe alkyle en C, -₄ ou alcoxy en C₁₋₄, les substituants pouvant, dans le cas de substitutions multiples, être différents indépendamment les uns des autres, ou bien R⁴ représente un groupe cyclohexyle, qui est éventuellement condensé à un noyau benzène, ou R⁴ représente un groupe phényle, naphtyle, thiényle, furyle, pyrrolyle ou pyridyle, les groupes phényle, naphtyle et pyridyle pouvant être substitués 1, 2 ou 3 fois, et les groupes thiényle, furyle, pyrrolyle pouvant être substitués 1 fois, par halogène, alkyle en C₁₋₄ ou alcoxy en C₁₋₄, les substituants pouvant ici aussi, dans le cas de substitutions multiples, être différents indépendamment les uns des autres, et R⁵ représente l'atome d'hydrogène ou un groupe alkyle en C₁₋₃, R⁵ pouvant, dans le cas d'un groupe alkyle en C₁₋₃, former avec R⁴, qui, en ce cas, désigne un groupe alkyle en C₃₋₅, un cycle hétérocyclique saturé à 6 chaînons, le cycle hétérocyclique saturé à 6 chaînons pouvant renfermer en outre un deuxième atome d'azote et être substitué à son tour par des groupes phényle ou phénylalkyle(C₁₋₃), et
où les deux groupes R², liés au squelette pyridine en positions 2 et 4 par les groupes carbonyle, peuvent aussi être différents indépendamment l'un de l'autre, et où tous les groupes alkyle nommés, comportant plus de 2 atomes de carbone, peuvent également être ramifiés,
ainsi que leurs sels physiologiquement acceptables, à l'exception de l'acide pyridine-2,4,5-tricarboxyli- que et ses esters triméthylés, de l'acide 5-éthylpyridine-2,4-dicarboxylique, des composés dans lesquels R¹ est un groupe aminométhyle, du 5-méthylpyridine-2,4-dicarboxylate de diméthyle, du 5-méthoxypyridine-2,4-dicarboxylate de diméthyle, du 5-éthoxycarbonylpyridine-2,4-dicarboxylate de diméthyle, du 4-méthoxycarbonylpyridine-2,5-dicarboxylate de diméthyle, caractérisé en ce que soit on transforme d'abord la 5-halogéno-2,4-diméthylpyridine en 2,4,5-triméthylpyridine, que l'on oxyde ensuite en acide pyridine-2,4,5-tricarboxylique, qui est transformé en composé triméthoxycarbonylé et ensuite en pyridine-2,4,5-tricarboxylate tripotassique, soit on oxyde la 5-halogéno-2,4-diméthylpyridine en un composé de formule suivante (1,1), 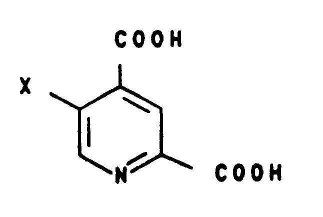
dans laquelle X représente un atome d'halogène, puis on transforme éventuellement
a) le composé de formule (1,1) en un composé de formule (1,2) suivante, 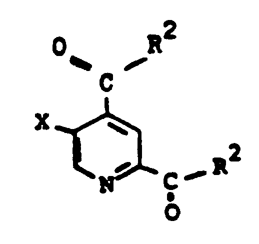 dans laquelle X est halogène et R² a les définitions indiquées pour la formule I selon la revendication 1,
et on fait ensuite réagir éventuellement le composé de formule (1,2) avec un composé de formule II ou Il' 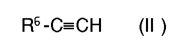 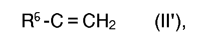 où R⁶ représente un groupe alkyle en C₁₋₇, éventuellement mono-ou disubstitué par un atome d'halogène ou un groupe hydroxy, nitro, cyano, amino, alcoxy en C₁₋₄, carboxyle,
alcoxy(C₁₋₄)carbonyle, alkyl(C₁₋₄)amino ou dialkyl(C₁₋₄)amino, ou éventuellement substitué par un groupe phényle, naphtyle, thiényle, furyle, pyrrolyle ou pyridyle, ces groupes aryle ou hétéroaryle étant quant à eux éventuellement monosubstitués par un groupe halogéno, carboxyle, amino, alkyl-(C₁₋₄)amino ou dialkyl(C₁₋₄)amino, ou hydroxy,
les groupes carboxyle libres éventuellement présents étant éventuellement protégés, et
on hydrogène éventuellement la double ou triple liaison carbone-carbone restant dans le fragment désormais lié au squelette pyridine, qui dérive du composé de formule II ou II', ou bien on fait réagir éventuellement le composé de formule (1,2) avec un composé de formule H₂ N-R³, dans laquelle R³ a les définitions indiquées dans la revendication 1 pour la formule I et dans laquelle les groupes carboxyle libres éventuellement présents sont éventuellement protégés, ou bien
on transforme éventuellement le composé de formule (1,2) de manière connue en soi en un composé de formule (1,3) suivante, 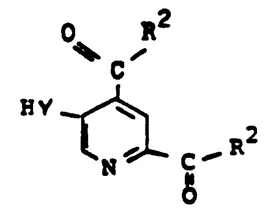 dans laquelle Y représente O ou NH et R² a les définitions indiquées dans la revendication 1 pour la formule I,
et on le fait ensuite réagir éventuellement avec un composé de formule III ou III' 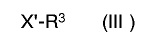 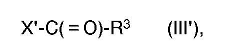 où X' représente chlore, brome ou iode et R³ a les définitions indiquées dans la revendication 1 pour la formule I, et où les groupes carboxyle libres éventuellement présents sont
éventuellement protégés, ou bien on fait réagir éventuellement le composé de formule (1,2) avec un composé de formule IV, 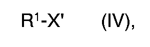 où X' est chlore, brome ou iode et R¹ a les définitions indiquées dans la revendication 1 pour la formule I, les définitions -OR³ et -N(R³)₂, où R³ a les mêmes significations que pour la formule I de la revendication 1, étant exclues, et où les groupes carboxyle libres éventuellement présents sont éventuellement protégés, ou bien on fait éventuellement réagir le composé de formule (1,2) avec un composé de formule V 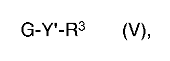 dans laquelle
Y' représente O ou NR³,
G représente un métal alcalin,
R³ a les définitions indiquées dans la revendication 1 pour la formule I, et dans laquelle les groupes carboxyle libres éventuellement présents sont éventuellement protégés, ou bien
on fait éventuellement réagir
b) le composé de formule (1,1) avec un composé de formule II ou II' où les groupes carboxyle libres éventuellement présents sont éventuellement protégés,
puis on estérifie éventuellement les acides carboxyliques présents en positions 2 et 4 du squelette pyridine ou on les transforme en diamides ou esteramides et on hydrogène éventuellement la double ou triple liaison carbone-carbone restant dans le fragment désormais lié au squelette pyridine, qui dérive du composé de formule II ou II', ou bien
on fait réagir éventuellement le composé de formule (1,1 ) avec un composé de formule H2N-R3, dans laquelle R³ a les définitions indiquées dans la revendication 1 pour la formule I et dans laquelle les groupes carboxyle libres éventuellement présents sont éventuellement protégés, ou bien
on transforme éventuellement le composé de formule (1,1) en un composé de formule (1,4) suivante, 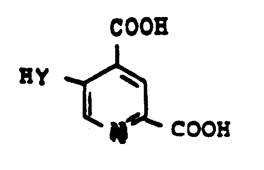 dans laquelle Y représente O ou NH, puis on estérifie éventuellement les acides carboxyliques présents en positions 2 et 4 du squelette pyridine ou on les transforme en diamides ou esteramides, et
on fait ensuite éventuellement réagir le produit avec un composé de formule III ou III' où les groupes carboxyle libres éventuellement présents sont éventuellement protégés, ou bien on fait réagir éventuellement le composé de formule (1,1) avec un composé de formule IV dans laquelle les groupes carboxyle libres éventuellement présents sont éventuellement protégés, les composés de formules R³0-1 et (R³)₂ N-I, où R³ a les définitions indiquées dans la revendication 1 pour la formule I, étant exclus, ou bien
on fait réagir éventuellement le composé de formule (1,1) avec un composé de formule V dans laquelle les groupes carboxyle libres éventuellement présents sont éventuellement protégés, puis on estérifie éventuellement les acides carboxyliques éventuellement présents en positions 2 et 4 du squelette pyridine dans les produits obtenus selon b) ou on les transforme en diamides ou esteramides, ou bien, éventuellement,
c) on protège tout d'abord, à l'aide d'un groupe protecteur, les groupes acide carboxyliques présents en positions 2 et 4 du squelette pyridine dans le composé de formule (1,1), ce qui permet d'obtenir un composé de formule (1,10) suivante, 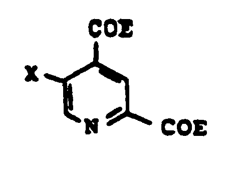 dans laquelle E représente un groupe protecteur,
et, éventuellement, on fait ensuite réagir le composé de formule (1,10) avec un composé de formule Il ou II' dans lequel des groupes carboxyle libres sont éventuellement protégés, puis on chasse éventuellement les groupes protecteurs E des groupes carboxyle en positions 2 et 4 du squelette pyridine, soit sélectivement, soit ensemble, et on estérifie éventuellement les groupes carboxyle libres résultants ou on les transforme en diamides ou esteramides, et on hydrogène éventuellement la double ou triple liaison carbone-carbone restant dans le fragment désormais lié au squelette pyridine, qui dérive du composé de formule II ou II', ou bien
on fait réagir éventuellement le composé de formule (1,10) avec un composé de formule H₂N-R³, dans laquelle R³ a les définitions indiquées dans la revendication 1 pour la formule I et dans laquelle les groupes carboxyle libres éventuellement présents sont éventuellement protégés, ou on transforme éventuellement le composé de formule (1,10) en un composé de formule (1,11) suivante, 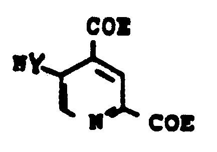 dans laquelle Y représente O ou NH, et on chasse ensuite éventuellement les groupes protecteurs E des groupes carboxyle en positions 2 et 4 du squelette pyridine, soit sélectivement, soit ensemble, et on estérifie éventuellement les groupes carboxyle libres résultants ou on les transforme en diamides ou esteramides, et
on fait ensuite éventuellement réagir le produit avec un composé de formule III ou III' où les groupes carboxyle libres éventuellement présents sont éventuellement protégés,
ou bien on fait réagir éventuellement le composé de formule (1,10) avec un composé de formule IV dans laquelle les groupes carboxyle libres éventuellement présents sont éventuellement protégés, les composés de formules R³0-1 et (R³)₂N-I, où R³ a les définitions indiquées dans la revendication 1 pour la formule I, étant exclus, ou bien
on fait réagir éventuellement le composé de formule (1,10) avec un composé de formule V dans laquelle les groupes carboxyle libres éventuellement présents sent éventuellement protégés, puis on chasse éventuellement, sélectivement ou ensemble, les groupes protecteurs E des acides carboxyliques éventuellement présents en positions 2 et 4 du squelette pyridine dans les produits obtenus selon c),
puis on estérifie éventuellement les acides carboxyliques libres obtenus ou on les transforme en diamides ou esteramides, puis on chasse éventuellement, par hydrolyse ou par hydrogénolyse, les groupes protecteurs présents dans les produits et on transforme éventuellement les composés obtenus selon a), b) ou c) en leurs sels physiologiquement acceptables.

2. Procédé selon la revendication 1, caractérisé en ce qu'on prépare des dérivés de substitution de l'acide pyridine-2,4-dicarboxylique de formule I, dans laquelle les symboles suivants représentent respectivement:
R¹ un atome d'halogène ou un groupe carboxyle, ou bien
R¹ un groupe alkyle en C₁₋₄, alcényle en C₂₋₄ ou alcynyle en C₂-₄, pouvant être éventuellement interrompu par un groupe carbonyle, et les groupes mentionnés étant éventuellement, quant à eux, monosubstitués par un groupe halogéno, hydroxy, nitro, cyano, amino ou carboxyle, ou bien
R¹ un groupe phényle, thiényle, furyle ou pyrrolyle, ces groupes aryle ou hétéroaryle étant quant à eue éventuellement monosubstitués par un groupe carboxyle, ou bien
R¹ un substituant de formule -OR³ ou -N(R³)₂, où R³ est un atome d'hydrogène ou un groupe alkyle en C₁₋₃ ou alkyl(C_{1 -3})carbonyle, ces groupes pouvant être à leur tour substitués par des groupes carboxyle, ou bien R³ représente un groupe phényle, pouvant être substitué quant à lui par un atome d'halogène, en position para, et les deux substituants R³ dans -N(R³)₂ peuvent aussi être différents indépendamment l'un de l'autre, et
R² un substituant de formule -OR⁴ ou R⁴⁻N-R⁵, où R⁴ est hydrogène ou alkyle en C₁₋₁₂, éventuellement substitué 1 ou 2 fois par des groupes halogéno, hydroxy, cyano, carboxyle, alcoxy en C₁₋₄, alcoxy(C₁₋₄)carbonyle, alkyl(C₁₋₄)carbonyloxy, alkyl(Ci -₄)amino ou dialkyl(C₁₋₄)amino, ou pouvant être substitués par des groupes phényle éventuellement eux-mêmes substitués 1, 2 ou 3 fois par un atome d'halogène, un groupe alkyle en C, -₄ ou alcoxy en C₁₋₄, les substituants pouvant, dans le cas de substitutions multiples, être différents indépendamment les uns des autres, ou bien R⁴ représente un groupe cyclohexyle, qui est éventuellement condensé à un noyau benzène, ou R⁴ représente un groupe phényle, naphtyle, thiényle, furyle, pyrrolyle ou pyridyle, les groupes phényle, naphtyle et pyridyle pouvant être substitués 1, 2 ou 3 fois, et les groupes thiényle, furyle, pyrrolyle pouvant être substitués 1 fois, par halogène, alkyle en C, -₄ ou alcoxy en C₁₋₄, les substituants pouvant ici aussi, dans le cas de substitutions multiples, être différents indépendamment les uns des autres, et R⁵ représente l'atome d'hydrogène ou un groupe alkyle en C₁₋₃, R⁵ pouvant, dans le cas d'un groupe alkyle en C₁₋₃, former avec R⁴, qui, en ce cas, désigne un groupe alkyle en C₃ -₅, un cycle hétérocyclique saturé à 6 chaînons, le cycle hétérocyclique saturé à 6 chaînons pouvant renfermer en outre un deuxième atome d'azote et être substitué à son tour par des groupes phényle ou phénylalkyle(C₁₋₃), et
où les deux groupes R², liés au squelette pyridine en positions 2 et 4 par les groupes carbonyle, peuvent aussi être différents indépendamment l'un de l'autre, et où tous les groupes alkyle nommés, comportant plus de 2 atomes de carbone, peuvent également être ramifiés,
ainsi que leurs sels physiologiquement acceptables, à l'exception de l'acide pyridine-2,4,5-tricarboxyli- que et ses esters triméthylés, de l'acide 5-éthylpyridine-2,4-dicarboxylique, des composés dans lesquels R¹ est un groupe aminométhyle, du 5-méthylpyridine-2,4-dicarboxylate de diméthyle, du 5-méthoxypyridine-2,4-dicarboxylate de diméthyle, du 5-éthoxycarbonylpyridine-2,4-dicarboxylate de diméthyle, du 4-méthoxycarbonylpyridine-2,5-dicarboxylate de diméthyle.

3. Procédé selon la revendication 1, caractérisé en ce qu'on prépare des dérivés de substitution d'acide pyridine-2,4-dicarboxylique de formule l', 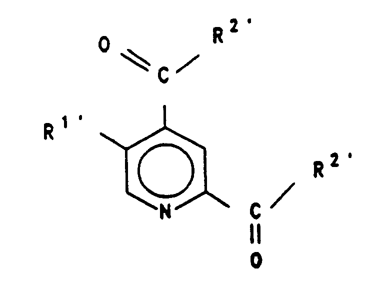 dans laquelle les substituants R^{1'} et R²' ont les mêmes définitions que R¹ et R² de la formule 1 selon la revendication 1, mais sans exclusion de l'acide pyridine-2,4,5-tricarboxylique et de ses esters triméthylés, de l'acide 5-éthylpyridine-2,4-dicarboxylique, des composés dans lesquels R^{1'} est un groupe aminométhyle, du 5-méthylpyridine-2,4-dicarboxylate de diméthyle, du 5-méthoxypyridine-2,4-dicar- boxylate de diméthyle, du 5-éthoxycarbonylpyridine-2,4-dicarboxylate de diméthyle, du 4-méthoxycar- bonylpyridine-2,5-dicarboxylate de diméthyle, pour leur utilisation comme médicaments.

4. Procédé selon la revendication 3, caractérisé en ce qu'on prépare des dérivés d'acide pyridine-2,4-dicarboxylique de formule l', dans laquelle R^{1'} et R^{2'} ont les mêmes définitions que R¹ et R² de la formule 1 selon la revendication 2, pour leur utilisation comme médicaments.

5. Utilisation des composés préparés selon une ou plusieurs des revendications 1 à 4, comme inhibiteurs de la proline- ou lysine hydroxylase.

6. Utilisation des composés préparés selon une ou plusieurs des revendications 1 à 4, comme fibrosuppresseurs et immunosuppresseurs.

7. Médicaments contenant un composé de formule I ou l' ainsi que des véhicules pharmaceutiquement acceptables.

8. Utilisation de composés de formule I ou l' pour influencer le métabolisme du collagène et des substances analogues au collagène ou la biosynthèse de C1 q.

9. Utilisation de composés de formule I ou l' pour traiter les troubles du métabolisme du collagène et des substances analogues au collagène ou de la biosynthèse de C1_{q}.

10. Procédé de fabrication de médicaments pour influencer le métabolisme du collagène et des substances analogues au collagène ou la biosynthèse de C1_{q}, caractérisé en ce qu'on incorpore aux médicaments un composé de formule I ou l'.
